# EUROPEAN PATENT APPLICATION

(11) **EP 2 436 675 A1**
(43) Date of publication of application: **04.04.2012**
(21) Application number: 10780296.9
(22) Date of filing: 28.05.2010
(51) Int. Cl.: C07D 239/34, A61K 31/505, A61K 31/506, A61P 3/04, A61P 3/06, A61P 3/10, A61P 9/00, A61P 9/10, A61P 9/12, A61P 13/12, A61P 25/00, A61P 27/02, A61P 43/00, C07D 239/36, C07D 401/04, C07D 401/06, C07D 401/14, C07D 413/14

(54) **NOVEL -PHENOXYBENZENEACETIC ACID DERIVATIVE AND PHARMACEUTICAL PREPARATION COMPRISING SAME**

(30) Priority: 29.05.2009 JP 2009131443
(71) Applicant: Kowa Company, Ltd., Nagoya-shi, Aichi-ken 460-8625 (JP)
(72) Inventor: MIURA, Toru, Higashimurayama-shi Tokyo 189-0022 (JP); ONOGI, Kazuhiro, Higashimurayama-shi Tokyo 189-0022 (JP); TAGASHIRA, Junya, Higashimurayama-shi Tokyo 189-0022 (JP); WATANABE, Gen, Higashimurayama-shi Tokyo 189-0022 (JP); SEKIMOTO, Ryohei, Higashimurayama-shi Tokyo 189-0022 (JP); ISHIDA, Rie, Higashimurayama-shi Tokyo 189-0022 (JP); AOKI, Hitomi, Higashimurayama-shi Tokyo 189-0022 (JP); OHGIYA, Tadaaki, Higashimurayama-shi Tokyo 189-0022 (JP)
(74) Representative: Adam, Holger
(86) International application number: PCT/JP2010/003582
(87) International publication number: WO 2010/137336

(57) **Abstract**

An α-phenoxybenzeneacetic acid derivative represented by general formula (I), which has both an angiotensin II receptor antagonistic activity and a PPARγ activation activity and is useful as a prophylactic and/or therapeutic agent for hypertension, metabolic syndrome or the like, a salt of the derivative, or a solvate of the derivative or the salt; and a pharmaceutical composition containing the derivative, the salt or the solvate. [In the formula, Q represents a group represented by formula (II) or (III) [wherein R¹ and R^{1'} independently represent a C₁₋₆ alkyl group; R² and. R^{2'} independently represent a substituted or unsubstituted C₁₋₆ alkyl group; R³ represents a hydrogen atom, a substituted or unsubstituted C₁₋₆ alkyl group, or a group represented by formula (IV) [wherein X and Y independently represent CH or N; Z represents a C₁₋₆ alkylene chain, or the like; R¹¹ represents a hydrogen atom, a C₆₋₁₀ aryl group, or the like; and m represents 0 or 1] ; and R⁴ represents a substituted C₁₋₆ alkyl group]; R⁵ and R⁶ independently represent a C₁₋₆ alkyl group: R⁷ represents a carboxyl group, or the like; R⁸, R⁹ and R¹⁰ independently represent a hydrogen atom, a halogen atom, or the like; and n represents an integer of 1 to 3.]

## Description

### TECHNICAL FIELD

The present invention relates to a novel α-phenoxybenzeneacetic acid having angiotensin II antagonistic activity and PPARγ activation activity, and a pharmaceutical agent containing the same.

### BACKGROUND ART

In recent years, disorders like diabetes, hypertension, dyslipidemia and obesity which can be a risk factor for arteriosclerotic disorders have been rapidly increasing due to changes in life style with improvements in living standard, i.e., high calorie and high cholesterol type diet, obesity, lack of exercise, aging, and the like. It is known that, although being a risk factor independent of each other, overlap of the disorders can cause an occurrence of arteriosclerotic disorders at higher frequency or aggravation of the disorders. As such, with the understanding of a condition having a plurality of risk factors for arteriosclerotic disorders as metabolic syndrome, efforts have been made to elucidate the cause of the syndrome and to develop a therapeutic method therefore

Angiotensin II (herein below, it may be also abbreviated as AII) is a peptide that is found to be an intrinsic pressor substance produced by renin-angiotensin system (i.e., RA system). It is believed that pharmacological inhibition of angiotensin II activity can lead to treatment or prevention of circulatory disorders like hypertension. Accordingly, an inhibitor for angiotensin converting enzyme (ACE) which inhibits the enzyme promoting the conversion of angiotensin I (AI) to angiotensin II (AII) has been clinically used as an inhibitory agent for RA system. Furthermore, an orally administrable AII receptor blocker (Angiotensin Receptor Blocker: ARB) has been developed, and losartan, candesartan, telmisartan, valsartan, olmesartan, and irbesartan are already clinically used as a hypotensive agent. It is reported by many clinical or basic studies that, as having not only a hypotensive activity but also other various activities including an anti-inflammatory activity, an endothelial function improving activity, a cardiovascular remodeling inhibiting activity, an oxidation stress inhibiting activity, a proliferation factor inhibiting activity, and insulin resistance improving activity, ARB is useful for cardiovascular disorders, renal diseases, and arteriosclerosis, etc. (Non-Patent Document 1 and 2). Most recently, it is also reported that ARB particularly has a kidney protecting activity which does not depend on a. hypotensive activity (Non-Patent Document 3).

Meanwhile, three isoforms, i.e., α, γ, and δ, are identified so far as peroxisome proliferaor-activated receptors (PPARs) which belong to a nuclear receptor superfamily. Among them, PPARγ is an isoform that is most abundantly expressed in an adipose tissue and it plays an important role in differentiation of adipocytes or metabolism of glycolipids. Currently, thiazolidinedione derivatives (i.e., TZD) like pioglitazone or rosiglitazone are clinically used as a therapeutic agent for diabetes having PPARγ activation activity, and they are known to have an activity of improving insulin resistance, glucose tolerance, and lipid metabolism, etc. Further, it is recently reported that, based on activation of PPARγ, TZD exhibits various activities including a hypotensive activity, an anti-inflammatory activity, an endothelial function improving activity, a proliferation factor inhibiting activity, and an activity of interfering RA system, etc. It is also reported that, according to such multiple activities, TZD shows a kidney protecting activity particularly in diabetic nephropathy without depending on blood sugar control (Non-Patent Document 4, 5, 6, 7, and 8). Meanwhile, there is also a concern regarding adverse effects of TZD caused by PPARγ activation like body fluid accumulation, body weight gain, peripheral edema, and pulmonary edema (Non-Patent Document 9 and 10).

It has been recently reported that telmisartan has a PPARγ activation activity (Non-Patent Document 11) . It has been also reported that the irbesartan has the same activity (Non-Patent Document 12) . These compounds have both a RA system inhibiting activity and a PPARγ activation activity, and thus are expected to be used as an integrated agent for prevention and/or treatment of circulatory disorders (e.g., hypertension, heart disease, angina pectoris, cerebrovascular disorders, cerebral circulatory disorders, ischemic peripheral circulatory disorders, and renal diseases, etc.) or diabetes-related disorders (e.g., type 2 diabetes, diabetic complications, insulin resistant syndrome, metabolic syndrome, and hyperinsulinemia, etc.) without increasing a risk of body fluid accumulation, body weight gain, peripheral edema, pulmonary edema, or congestive heart failure that are concerned over the use of TZD (Patent Document 1). Among them, for diabetic nephropathy, a synergistic prophylactic and/or therapeutic effect is expected from multiple kidney protecting activity based on activities of RA system inhibition and PPARγ activation.

As compounds having such activity, pyrimidine and triazine derivatives (Patent Document 1), imidazopyridine derivatives (Patent Document 2), indole derivatives (Patent Document 3), imidazole derivatives (Patent Document 4), and condensed ring derivatives (Patent Document 5) have been reported.

On the other hand, antihypertensive agents having α-phenoxybenzeneacetic acid or a structure equivalent thereto have been reported. They are based on angiotensin II-inhibitory activity or endothelin A inhibitory activity, and has a common structure of a heterocyclic alkyl group substituted at 4-position of the phenoxy group They are further divided with difference of the hetero ring, and condensed heterocyclic-substituted compounds (Patent Documents 6, 7, 8, 9, and 10, and Non-Patent Documents 13, 14, and 15), imidazole-substituted compounds (Patent Documents 11 and 12), triazine-substituted compounds (Patent Document 13) and pyrimidine-substituted compounds (Patent Document 14) are reported in documents, Particularly, the above Patent Document 14 has reported compounds represented by a general formula (A) described below:

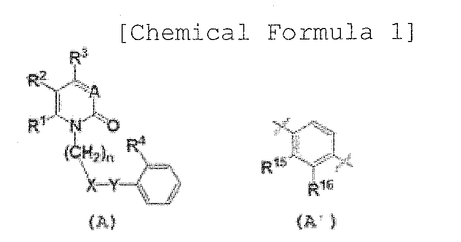

[wherein A represents N or CR⁵, R¹ to R³ represent a C₁₋₄ alkyl group or the like, n represents an integer of 1 to 6, X represents the above formula (A') [wherein R¹⁵ and R¹⁶ represent hydrogen and the like] or the like, Y represents -O-CHR²⁰- or the like, and one of R⁴ and R²⁰ represents a carboxyl group or tetrazolyl group, and the other of R⁴ and R²⁰ represents hydrogen].
However, to these compounds, a heterocyclic moiety of a heterocyclic alkyl group, which binds to the 4-position of a phenoxy group, binds via a heteroatom, and thus these compounds are different from compounds to which a heterocyclic moiety binds by a carbon atom.

In addition, a monocyclic or condensed heterocyclic-substituted compound has been reported (Patent Document 15). In this document, reported is a compound represented by a formula (B) described below:

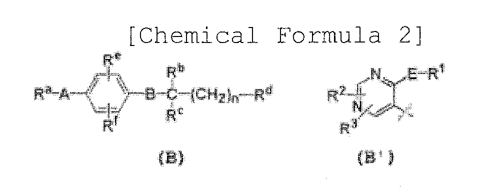

[wherein n represents 0 or 1, A represents an alkylene group, B represents an oxygen atom or the like, examples of R^{a} include the above formula (B') in addition to hetero rings such as benzimidazole and imidazo[4,5-b] pyridine [wherein E represents an oxygen atom or the like, R¹ represents a C₁₋₉ alkyl group that may be substituted with a cycloalkyl group, a fluorine atom, a chlorine atom, a bromine atom, a hydroxy group, an amino group, an alkylamino group, a dialkylamino group, or a α, α-difluoroethane group, or the like, R² represents a C₁₋₅ alkyl group or the like, and R³ represents a C₁₋₆ alkyl group that may be substituted with a hydroxy group or an alkoxy group or the like], or the like, R^{b} represents a tetrazolyl group, a carboxyl group, or a group that is converted to a carboxyl group in a living body, or the like, R^{c} represents a hydrogen atom or the like, and R^{d} represents a phenyl group, a biphenyl group, a naphthyl group, or the like that may be substituted with a fluorine atom, a chlorine atom, or a bromine atom or a methyl group or methoxy group]. However, the heterocyclic groups described as a specific compound in this document are only an imidazol-1-yl group, a benzoimidazol-1-yl group, an imidazo[4,5-b]pyridin-3-yl group, a quinolin-4-oxy group (Example 13), and a quinazolin-4-on-3-yl group (Example 35), and all the heterocyclic groups except for the quinolin-4-oxy group bind to a nitrogen atom of a hetero ring, but the quinolin-4-oxy group binds via an oxygen atom. Patent Document 15 describes the value of IC₅₀ of the compounds A to I with respect to AII receptor, and describes a compound of a quinolin-4-oxy group as the compound D, and a compound of a quinazolin-4-on-3-yl group (although Patent Document 15 describes the compound E as quinoline, this appears a clerical error of quinazoline.) as the compound E. The value of IC₅₀ by the test method described in Patent Document 15 is 2 µM.
As described above, Patent Document 15 does not specifically describe a heterocyclic group binding to a carbon atom of the hetero ring, and neither describe nor suggest a compound having a heterocyclic alkyl group at the 2-position of the pyrimidine in the above formula (B') (R² and R³ in the formula B'), a compound having a substituent on the nitrogen atom at the 3-position and a compound having an alkyl group in which an aryl group or an alkoxy group is substituted on the oxygen atom at the 4-position (R¹ in the formula B'). Thus, the compounds of Patent Document 15 are also different from the compound of the present invention in this point. Furthermore, Patent Document 15 neither describes nor suggests PPARγ activation activity, or treatment for diabetes mellitus, obesity or metabolic syndrome as pharmacological activity for any of compounds having α-phenoxybenzeneacetic acid or compounds having equivalent structure thereto.

### PRIOR ART DOCUMENTS

### PATENT DOCUMENT

Patent Document 1: WO 2008/062905
Patent Document 2: WO 2008/084303
Patent Document 3: WO 2008/096820
Patent Document 4: WO 2008/096829
Patent Document 5: WO 2008/143262
Patent Document 6: WO 1995/003044
Patent Document 7: WO 1994/021259
Patent Document 8: USP 5,334,598
Patent Document 9: Japanese Patent Application National Publication (Laid-Open) No. 5-503530
Patent Document 10: Japanese Patent Application National Publication (Laid-Open) No. 5-504969
Patent Document 11: USP 5,191,086
Patent Document 12: Japanese Patent Application National Publication (Laid-Open) No. 5-504359
Patent Document 13: Japanese Patent Application National Publication (Laid-Open) No. 5-503533
Patent Document 14: Japanese Patent Application National Publication (Laid-Open) No. 6-505715
Patent Document 15: Japanese Patent Application Laid-Open No. 5-247074

### NON-PATENT DOCUMENT

Non-Patent Document 1: AMER. J. Hypertension, 18, 720 (2005)
Non-Patent Document 2: Current Hypertension Report, 10, 261 (2008)
Non-Patent Document 3: Diabetes Care, 30, 1581 (2007)
Non-Patent Document 4: Kidney Int., 70, 1223 (2006)
Non-Patent Document 5: Circulation, 108, 2941 (2003)
Non-Patent Document 6: Best Pract . Res . Clin. Endocrinol. Metab., 21(4), 687 (2007)
Non-Patent Document 7: Diab. Vasc. Dis. Res., 1(2), 76 (2004)
Non-Patent Document 8: Diab. Vasc. Dis. Res., 2(2), 61 (2005)
Non-Patent Document 9: J. Clin. Invest., 116(3), 581 (2006)
Non-Patent Document 10: FASEB J., 20(8), 1203 (2006)
Non-Patent Document 11: Hypertension, 43, 993 (2004)
Non-Patent Document 12: Circulation, 109, 2054 (2004)
Non-Patent Document 13: Journal of Medicinal Chemistry (2002), 45(18), 3829-3835
Non-Patent Document 14: FEBS Letters (1998), 423(1), 15-18
Non-Patent Document 15: Journal of Medicinal Chemistry (1993), 36(23), 3738-42

### SUMMARY OF THE INVENTION

### PROBLEMS TO BE SOLVED BY THE INVENTION

The object of the present invention is to provide a novel compound that is useful as a pharmaceutical agent for prevention and/or treatment of a cardiovascular disease such as hypertension, and a metabolic disease such as diabetes mellitus and the like, and a pharmaceutical composition using the same.

### MEANS FOR SOLVING THE PROBLEMS

The present inventors continued researches earnestly to achieve the above-mentioned object, and as a result, found that there is a compound having particularly excellent activity among compounds of an α-phenoxybenzeneacetic acid derivative wherein an alkylene group binding to the 4-position of the phenoxy group of the α-phenoxybenzeneacetic acid derivative, binds to a carbon atom of a heterocyclic group, and particularly found that an α-phenoxybenzeneacetic acid derivative represented by a general formula (I) described below is a compound that has excellent angiotensin II antagonistic activity and PPARγ activation activity in combination, whereby to complete the present invention.

Specifically, the present invention relates to the inventions described below.
[1] An α-phenoxybenzeneacetic acid derivative represented by the following general formula (I):

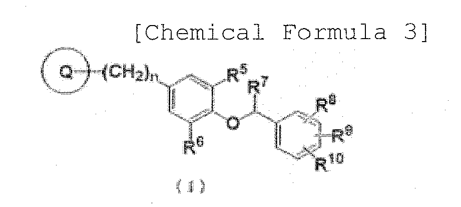

[wherein the ring Q represents the following formula (II) or (III):

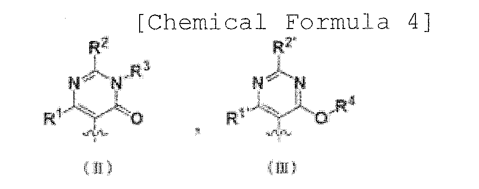

[wherein, in the formula (II) or (III), R¹ and R^{1'} represent a C₁₋₆ alkyl group,
R² and R^{2'} represent a C₁₋₆ alkyl group or a C₁₋₆ alkyl-pyridinyl-C₁₋₆ alkyl group,
R³ represents a hydrogen atom, a C₁₋₆ alkyl group, a C₁₋₆ alkoxy-C₁₋₆ alkyl group, a C₃₋₈ cycloalkyl-C₁₋₆ alkyl group, a C₆₋₁₀ aryl-C₁₋₆ alkyl group or the following formula (IV):

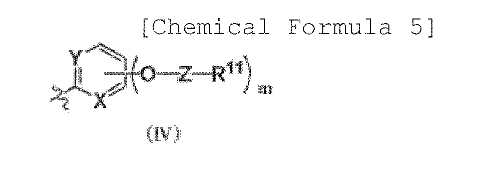

[wherein, in the formula (IV), X and Y represent CH or N, Z represents a single bond or a C₁₋₆ alkylene chain, R¹¹ represents a hydrogen atom, a C₆₋₁₀ aryl group, a carbamoyl group, a C₁₋₆ alkylthio group, a C₁₋₆ alkylsulfinyl group, or a C₁₋₆ alkylsulfonyl group, and m represents 0 or 1], and
R⁴ represents a C₁₋₆ alkoxy-C₁₋₆ alkyl group or a C₆₋₁₀ aryl-C₁₋₆ alkyl group]
R⁵ and R⁶ represent, independently from each other, a C₁₋₆ alkyl group,
R⁷ represents a carboxyl group, a C₂₋₇ alkoxycarbonyl group, a carbamoyl group, a C₁₋₆ alkylsulfonyl-carbamoyl group, a tetrazolyl group or an oxooxadiazolyl group,
R⁸, R⁹, and R¹⁰ represent, independently from each other, a hydrogen atom, a halogen atom or a. C₁₋₆ alkoxy group, and n represents an integer of 1 to 3],
or a salt thereof or a solvate thereof.

[2] The α-phenoxybenzeneacetic acid derivative,or a salt thereof or a solvate thereof according to [1] wherein the compound represented by the general formula (I) is a compound selected from a group consisting of
2-[4-[{4-butyl-2-methyl-6-oxo-1-(pyridin-2-yl)-1,6-di hydropyrimidin-5-yl}methyl]-2,6-dipropylphenoxy]-2-phenylac etic acid,
2-[4-[{2-methyl-6-oxo-4-propyl-1-(pyridin-2-yl)-1,6-d ihydropyrimidin-5-yl}methyl]-2,6-dipropylphenoxy]-2-phenyla cetic acid,
2-[4-[{4-ethyl-2-methyl-6-oxo-1-(pyridin-2-yl)-1,6-di hydropyrimidin-5-yl}methyl]-2,6-dipropylphenoxy]-2-phenylac etic acid,
6-butyl-2-methyl-5-[4-{phenyl(1H-tetrazol-5-yl)methox y}-3,5-dipropylbenzyl]-3-(pyridin-2-yl)pyrimidin-4(3H)-one,
3-[[4-[{4-butyl-2-methyl-6-oxo-1-(pyridin-2-yl)-1,6-d ihydropyrimidin-5-yl}methyl]-2,6-dipropylphenoxy](phenyl)me thyl]-1,2,4-oxadiazol-5(4H)-one,
2-[4-[{4-butyl-2-methyl-6-oxo-1-(pyridin-2-yl)-1,6-di hydropyrimidin-5-yl}methyl]-2,6-dipropylphenoxy]-N-(methyls ulfonyl)-2-phenyl acetoamide,
2-[4-[{4-butyl-2-methyl-6-oxo-1-(pyridin-2-yl)-1,6-di hydropyrimidin-5-yl}methyl]-2,6-dipropylphenoxy]-2-(2-chlor ophenyl)acetic acid,
2-[4-[{4-butyl-2-methyl-6-oxo-1-(pyridin-2-yl)-1,6-di hydropyrimidin-5-yl}methyl]-2,6-dipropylphenoxy]-2-(3-chlor ophenyl)acetic acid,
2-[4-[{4-butyl-2-methyl-6-oxo-1-(pyridin-2-yl)-1,6-di hydropyrimidin-5-yl}methyl]-2,6-dipropylphenoxy]-2-(4-chlor ophenyl)acetic acid,
2-[4-[{4-butyl-2-methyl-6-oxo-1-(pyridin-2-yl)-1,6-di hydropyrimidin-5-yl}methyl]-2,6-dipropylphenoxy]-2-(2-metho xyphenyl)acetic acid,
6-butyl-5-[4-{(2-chlorophenyl)(1H-tetrazol-5-yl)metho xy}-3,5-dipropylbenzyl]-2-methyl-3-(pyridin-2-yl)pyrimidin-4(3H)-one,
2-[4-[[1-{4-(benzyloxy)phenyl}-4-butyl-2-methyl-6-oxo -1,6-dihydropyrimidin-5-yl]methyl]-2,6-dipropylphenoxy]-2-p henylacetic acid,
2-[4-[{4-butyl-1-(4-ethoxyphenyl)-2-methyl-6-oxo-1,6-dihydropyrimidin-5-yl}methyl]-2,6-dipropylphenoxy]-2-phenyl acetic acid,
2-[4-[{4-butyl-1-(4-isopropoxyphenyl)-2-methyl-6-oxo-1,6-dihydropyrimidin-5-yl}methyl]-2,6-dipropylphenoxy]-2-ph enylacetic acid,
2-[4-[{4-butyl-1-(5-methoxypyrimidin-2-yl)-2-methyl-6 -oxo-1,6-dihydropyri-midin-5-yl}methyl]-2,6-dipropylphenoxy] -2-phenylacetic acid,
(-)-2-[4-[{4-butyl-1-(5-methoxypyrimidin-2-yl)-2-meth yl-6-oxo-1,6-dihydropyrimidin-5-yl}methyl]-2,6-dipropylphen oxy]-2-phenylacetic acid,
(+)-2-[4-[{4-butyl-1-(5-methoxypyrimidin-2-yl)-2-meth yl-6-oxo-1,6-dihydropyrimidin-5-yl}methyl]-2,6-dipropylphen oxy]-2-phenylacetic acid,
2-[4-[{4-butyl-1-(5-methoxypyrimidin-2-yl)-2-metliyl-6 -oxo-1,6-dihydropyrimidin-5-yl}methyl]-2,6-dipropylphenoxy] -2-(4-chlorophenyl)acetic acid,
2-[4-[[1-{5-(benzyloxy)pyrimidin-2-yl}-4-butyl-2-meth yl-6-oxo-1,6-dihydropyrimidin-5-yl]methyl]-2,6-dipropylphen oxy]-2-phenylacetic acid,
2-[4-[{4-butyl-1-(5-ethoxypyrimidin-2-yl)-2-methyl-6-oxo-1,6-dihydropyrimidin-5-yl}methyl]-2,6-dipropylphenoxy]-2-phenylacetic acid,
(-)-2-[4-[{4-butyl-1-(5-ethoxypyrimidin-2-yl)-2-methy 1-6-oxo-1,6-dihydropyrimidin-5-yl}methyl]-2,6-dipropylpheno xy]-2-phenylacetic acid,
(+)-2-[4-[{4-butyl-1-(5-ethoxypyrimidin-2-yl)-2-methy 1-6-oxo-1,6-dihydropyrimidin-5-yl}methyl]-2,6-dipropylpheno xy]-2-phenylacetic acid,
2-[4-[[1-{5-(4-amino-4-oxobutoxy)pyrimidin-2-yl}-4-bu tyl-2-methyl-6-oxo-1,6-dihydropyrimidin-5-yl]methyl]-2,6-di propylphenoxy]-2-phenylacetic acid,
2-[4-[[4-butyl-2-methyl-1-[5-{2-(methylthio)ethoxy}py rimidin-2-yl]-6-oxo-1,6-dihydropyrimidin-5-yl]methyl]-2,6-d ipropylphenoxy]-2-phenylacetic acid,
2-[4-[[4-butyl-2-methyl-1-[5-{2-(methylsulfinyl)ethox y}pyrimidin-2-yl]-6-oxo-1,6-dihydropyrimidin-5-yl]methyl]-2 ,6-dipropylphenoxy]-2-phenylacetic acid,
2-[4-[[4-butyl-2-methyl-1-[5-{2-(methylsulfonyl)ethox y}pyrimidin-2-yl]-6-oxo-1,6-dihydropyrimidin-5-yl]methyl]-2 ,6-dipropylphenoxy]-2-phenylacetic acid,
2-[4-[{4-butyl-1-(5-hydroxypyrimidin-2-yl)-2-methyl-6 -oxo-1,6-dihydropyrimidin-5-yl}methyl]-2,6-dipropylphenoxy] -2-phenylacetic acid,
2-[4-{(1-benzyl-4-butyl-2-methyl-6-oxo-1,6-dihydropyr imidin-5-yl)methyl}-2,6-dipropylphenoxy]-2-phenylacetic acid,
2-[4-[{4-(benzyloxy)-6-butyl-2-methylpyrimidin-5-yl}m ethyl]-2,6-dipropylphenoxy]-2-phenylacetic acid,
2-[4-[{4-butyl-1-(cyclohexylmethyl)-2-methyl-6-oxo-1, 6-dihydropyrimidin-5-yl}methyl]-2,6-dipropylphenoxy]-2-phen ylacetic acid,
2-[4-[[4-butyl-2-{2-(5-ethylpyridin-2-yl)ethyl}-6-oxo -1,6-dihydropyrimidin-5-yl]methyl]-2,6-dipropylphenoxy]-2-p henylacetic acid,
2-[4-[{4-butyl-6-(2-methoxyethoxy)-2-methylpyrimidin-5-yl}methyl]-2,6-dipropylphenoxy]-2-phenylacetic acid, and.
2-[4-[{4-butyl-1-(2-methoxyethyl)-2-methyl-6-oxo-1,6-dihydropyrimidin-5-yl}methyl]-2,6-dipropylphenoxy]-2-phenyl acetic acid.
The alkyl group such as butyl and propyl in the nomenclature of the above-mentioned compounds represents a straight (normal) chain unless particularly designated.

[3] A pharmaceutical composition comprising the α-phenoxybenzeneacetic acid derivative, or a salt thereof or a solvate thereof according to [1] or [2], and a pharmaceutically accepted carrier.

[4] A pharmaceutical composition having angiotensin II receptor-antagonistic activity and PPARγ activation activity in combination, which contains the α-phenoxybenzeneacetic acid derivative, or a salt thereof or a solvate thereof according to [1] or [2] as an active ingredient.

[5] A preventive and/or therapeutic agent for a cardiovascular disease, which contains the α-phenoxybenzeneacetic acid derivative, or a salt thereof or a solvate thereof according to [1] or [2] as an active ingredient.

[6] The preventive and/or therapeutic agent according to [5] wherein the cardiovascular disease is hypertension, a cardiac disease, angina pectoris, a cerebrovascular disorder, a cerebral circulatory disorder, an ischemic peripheral circulatory disorder, a renal disease, or arteriosclerosis.

[7] A preventive and/or therapeutic agent for a metabolic disease, which contains the α-phenoxybenzeneacetic acid derivative, or a salt thereof or a solvate thereof according to [1] or [2] as an active ingredient.

[8] The preventive and/or therapeutic agent according to [7] wherein the metabolic disease is Type II diabetes mellitus, diabetic complication (diabetic retinopathy, a diabetic nerve disorder, or diabetic nephropathy), insulin resistance syndrome, metabolic syndrome, or hyperinsulinemia.

[9] A method of preventing and/or treating a cardiovascular disease, which is characterized by administering an effective amount of the α-phenoxybenzeneacetic acid derivative, or a salt thereof or a solvate thereof according to [1] or [2] to a patient in need of the treatment.

[10] A method of preventing and/or treating a metabolic disease, which is characterized by administering an effective amount of the α-phenoxybenzeneacetic acid derivative, or a salt thereof or a solvate thereof according to [1] or [2] to a patient in need of the treatment.

[11] Use of the α-phenoxybenzeneacetic acid derivative, or a salt thereof or a solvate thereof according to [1] or [2] for manufacture of a preparation for prevention and/or treatment of a cardiovascular disease.

[12] Use of the α-phenoxybenzeneacetic acid derivative, or a salt thereof or a solvate thereof according to [1] or [2] for manufacture of a preparation for prevention and/or treatment of a metabolic disease.

[13] The α-phenoxybenzeneacetic acid derivative, or a salt thereof or a solvate thereof according to [1] or [2] as a preventive and/or therapeutic agent that has both activities of angiotensin II receptor-antagonistic activity and PPARγ activation activity in combination.

### EFFECTS OF THE INVENTION

The α-phenoxybenzeneacetic acid derivative represented by the general formula (I) of the present invention, or a salt thereof or a solvate thereof shows strong antagonistic activity for angiotensin II receptor, and can be suitably used as an active ingredient of a preventive and/or therapeutic agent for diseases with which angiotensin II is involved, for example, cardiovascular diseases such as hypertension, cardiac diseases, angina pectoris, cerebrovascular disorders, cerebral circulatory disorders, ischemic peripheral circulatory disorders, renal disease, and arteriosclerosis.

In addition, the α-phenoxybenzeneacetic acid derivative represented by the general formula (I) of the present invention, or a salt thereof or a solvate thereof represents PPARγ activation activity, and can be suitably used as an active ingredient of a preventive and/or therapeutic agent for diseases with which PPARγ is involved, for example, metabolic diseases such as arteriosclerosis, Type II diabetes mellitus, diabetic complication (diabetic retinopathy, a diabetic nerve disorder, or diabetic nephropathy), insulin resistance syndrome, syndrome X, metabolic syndrome, and hyperinsulinemia.

Furthermore, the α-phenoxybenzeneacetic acid derivative represented by the general formula (I) of the present invention, or a salt thereof or a solvate thereof has angiotensin II receptor-antagonistic activity and PPARγ activation activity in combination, can be suitably used as an active ingredient of a preventive and/or therapeutic agent for diseases with which both of angiotensin II and PPARγ are involved, for example, diseases such as arteriosclerosis, diabetic nephropathy, insulin resistance syndrome, syndrome X, and metabolic syndrome.

### MODES FOR CARRYING OUT THE INVENTION

Examples of the "halogen atom" when used in the present specification include, for example, a fluorine atom, a chlorine atom, a bromine atom, or an iodine atom and the like.

The "C₁₋₆ alkyl group" and the "C₁₋₆ alkyl" when used in the present specification mean a straight or branched C₁₋₆ hydrocarbon group, and examples thereof include, for example, a methyl group, an ethyl group, a n-propyl group, an isopropyl group, a n-butyl group, an isobutyl group, a t-butyl group, a n-pentyl group, a 2-methylbutyl group, a 2,2-dimethylpropyl group, a n-hexyl group, and the like.

The "C₁₋₆ alkoxy group" and the "C₁₋₆ alkoxy" when used in the present specification mean a straight or branched C₁₋₆ alkoxy group, and examples thereof include, for example, a methoxy group, an ethoxy group, a propoxy group, an isopropoxy group, a butoxy group, an isobutoxy group, a sec-butoxy group, a tert-butoxy group, a pentoxy group, an isopentoxy group, a neopentoxy group, a hexyloxy group or an isohexyloxy group, and the like.

Examples of the "C₃₋₈ cycloalkyl group" and the "C₃₋₈ cycloalkyl" when used in the present specification include a C₃₋₈, preferably C₅₋₈ saturated or unsaturated monocyclic, polycyclic, or condensed cyclic cycloalkyl group. Examples of such cycloalkyl group include, for example, a cyclopropyl group, a cyclobutyl group, a cyclopentyl group, a cyclohexyl group, a cyclopentyl group, and a cyclooctyl group.

Examples of the "C₆₋₁₀ aryl group" and the "C₆₋₁₀ aryl" when used in the present specification include a C₆₋₁₀ monocyclic or condensed cyclic carbocyclic aryl group. Examples of such aryl group include, for example, a phenyl group, a naphthyl group, and, an azulenyl group.

The "C₁₋₆ alkylene chain" when used in the present specification means a straight or branched C₁₋₆ divalent hydrocarbon group, and examples thereof include, for example, a methylene chain, an ethylene chain, a trimethylene chain, tetramethylene chain, a pentamethylene chain, a hexamethylene chain, a 2,2-propylene chain, and the like.

The "C₁₋₆ alkylthio group" when used in the present specification means a straight or branched C₁₋₆, preferably C₁₋₄ alkylthio group, and examples thereof include, for example, a methylthio group, an ethylthio group, a n-propylthio group, an isopropylthio group, a n-butylthio group, an isobutylthio group, a sec-butylthio group, a tert-butylthio group, a n-pentylthio group, an isopentylthio group, a neopentylthio group, a 1-methylbutylthio group, a 1-ethylpropylthio group, a n-hexylthio group, an isohexylthio group, a 4-methylpentylthio group, a 3-methylpentylthio group, a 2-methylpentylthio group, a 1-methylpentylthio group, a 3,3-dimethylbutylthio group, a 2,2-dimethylbutylthio group, a 1,1-dimethylbutylthio group, a 1,2-dimethylbutylthio group, a 1,3-dimethylbutylthio group, a 2,3-dimethylbutylthio group, a 1-ethylbutylthio group, a 2-ethylbutylthio group, and the like.

The "C₁₋₆ alkylsulfinyl group" when used in the present specification means a straight or branched C₁₋₆, preferably C₁₋₄ alkylsulfinyl group, and examples thereof include, for example, a methylsulfinyl group, an ethylsulfinyl group, a n-propylsulfinyl group, an isopropylsulfinyl group, a n-butylsulfinyl group, an isobutylsulfinyl group, a sec-butylsulfinyl group, a tert-butylsulfinyl group, a n-pentylsulfinyl group, an isopentylsulfinyl group, a neopentylsulfinyl group, a 1-methylbutylsulfinyl group, a 1-ethylpropylsulfinyl group, a n-hexylsulfinyl group, an isohexylsulfinyl group, a 4-methylpentylsulfinyl group, a 3-methylpentylsulfinyl group, a 2-methylpentylsulfinyl group, a 1-methylpentylsulfinyl group, a 3,3-dimethylbutylsulfinyl group, a 2,2-dimethylbutylsulfinyl group, a 1,1-dimethylbutylsulfinyl group, a 1,2-dimethylbutylsulfinyl group, a 1,3-dimethylbutylsulfinyl group, a 2,3-dimethylbutylsulfinyl group, a 1-ethylbutylsulfinyl group, a 2-ethylbutylsulfinyl group, and the like.

The "C₁₋₆ alkylsulfonyl group" and the "C₁₋₆ alkylsulfonyl" when used in the present specification mean a straight or branched C₁₋₆, preferably C₁₋₄ alkylsulfonyl group, and examples thereof include, for example, a methylsulfonyl group, an ethylsulfonyl group, a n-propylsulfonyl group, an isopropylsulfonyl group, a n-butylsulfonyl group, an isobutylsulfonyl group, a sec-butylsulfonyl group, a tert-butylsulfonyl group, a n-pentylsulfonyl group, an isopentylsulfonyl group, a neopentylsulfonyl group, a 1-methylbutylsulfonyl group, a 1-ethylpropylsulfonyl group, a n-hexylsulfonyl group, an isohexylsulfonyl group, a 4-methylpentylsulfonyl group, a 3-methylpentylsulfonyl group, a 2-methylpentylsulfonyl group, a 1-methylpentylsulfonyl group, a 3,3-dimethylbutylsulfonyl group, a 2,2-dimethylbutylsulfonyl group, a 1,1-dimethylbutylsulfonyl group, a 1,2-dimethylbutylsulfonyl group, a 1,3-dimethylbutylsulfonyl group, a 2,3-dimethylbutylsulfonyl group, a 1-ethylbutylsulfonyl group, a 2-ethylbutylsulfonyl group, and the like.

The "C₂₋₇ alkoxycarbonyl group" when used in the present specification means a group by binding of the "C₁₋₆ alkoxy group" to a carbonyl group. Specifically, examples thereof include, for example, a methoxycarbonyl group, an ethoxycarbonyl group, a n-propoxycarbonyl group, an isopropoxycarbonyl group, a n-butoxycarbonyl group, an isobutoxycarbonyl group, a sec-butoxycarbonyl group, a tert-butoxycarbonyl group, a n-pentoxycarbonyl group, an isopentoxycarbonyl group, a neopentoxycarbonyl group, a 4-methylbutoxycarbonyl group, a 1-ethylpropoxycarbonyl group, a n-hexyloxycarbonyl group, an isohexyloxycarbonyl group, a 3-methylpentoxycarbonyl group, a 2-methylpentoxycarbonyl group, a 1-methylpentoxycarbonyl group, a 3,3-dimethylbutoxycarbonyl group, a 2,2-dimethylbutoxycarbonyl group, a 1,1-dimethylbutoxycarbonyl group, a 1,2-dimethylbutoxycarbonyl group, a 1,3-dimethylbutoxycarbonyl group, a 2,3-dimethylbutoxycarbonyl group, a 1-ethylbutoxycarbonyl group, a 2-ethylbutoxycarbonyl group, and the like. The carbon number herein means a carbon number containing the carbons of the carbonyl group.

The "C₁₋₆ alkyl-pyridinyl-C₁₋₆ alkyl group" when used in the present specification means a C₁₋₆ alkyl group substituted with a pyridinyl group that is substituted with one to four C₁₋₆ alkyls. The substitution position in the pyridine ring is not particularly limited, but an alkyl-substituted pyridin-2-yl-alkyl group is preferable. Specifically, examples thereof include, for example, a 3-methylpyridin-2-ylmethyl group, a 3-ethylpyridin-2-ylmethyl group, a 3-methylpyridin-2-ylethyl group, a 3-ethylpyridin-2-ylethyl group, a 4-ethylpyridin--2-ylethyl group, a 5-ethylpyrldin-2-ylethyl group, a 6-ethylpyridin-2-ylethyl group, a 3,4-dimethylpyridin-2-ylethyl group, a 3,5-dimethylpyridin-2-ylethyl group, and the like.

The "C₁₋₆ alkoxy-C₁₋₆ alkyl group" when used in the present specification means a C₁₋₆ alkyl group that is substituted with one to three C₁₋₆ alkoxys. Specifically, examples thereof include, for example, a methoxymethyl group, an ethoxymethyl group, an ethoxyethyl group, a propoxymethyl group, a propoxyethyl group, a butoxymethyl group, a butoxyethyl group, a 2,2-dimethoxyethyl group, and the like.

The "C₃₋₈ cycloalkyl-C₁₋₆ alkyl group" when used in the present specification means a C₁₋₆ alkyl group substituted with C₃₋₈ cycloalkyl. Specifically, examples thereof include, for example, a cyclopropylmethyl group, a cyclopropylethyl group, a cyclobutylmethyl group, a cyclohexylmethyl group, a cyclopentylmethyl group, a cyclooctylmethyl group, a cyclohexylethyl group, a cyclohexylpropyl group, a cyclohexylbutyl group, and the like.

The "C₆₋₁₀ aryl-C₁₋₆ alkyl group"' when used in the present specification means a C₁₋₆ alkyl group substituted with C₆₋₁₀ aryl. Specifically, examples thereof include, for example, a benzyl group, a phenethyl group, a phenylpropyl group, a phenylbutyl group, a phenylpentyl group, a phenylhexyl group, a 1-naphthylmethyl group, a 2-naphthylmethyl group, and the like.

The "C₁₋₆ alkylsulfonyl-carbamoyl group" when used in the present specification means a carbamoyl group in which one or two of the above C₁₋₆ alkylsulfonyl group are substituted on the nitrogen atom. Specifically, examples thereof include, for example, a N-methylsulfonylcarbamoyl group, a N,N-di(methylsulfonyl)carbamoyl group, a N-ethylsulfonylcarbamoyl group, a N,N-di(ethylsulfonyl)carbamoyl group, a N-propylsulfonylcarbamoyl group, a N-isopropylsulfonylcarbamoyl group, a N,N-di(propylsulfonyl)carbamoyl group, a N-butylsulfonylcarbamoyl group, a N,N-di(butylsulfonyl)carbamoyl group, and the like.

Preferable modes of the present invention are as follows.
The C₁₋₆ alkyl group in R¹ and R^{1'} in the general formulae (II) or (III), is preferably a C₁₋₄ alkyl group or a C₂₋₄ alkyl group which is a straight or branched C₁₋₄, preferably C₂₋₄ hydrocarbon group, and more preferably an ethyl group, a n-propyl group, or a n-butyl group.
The C₁₋₆ alkyl group in R² and R²' in the general formulae (II) or (III) is preferably a C₁₋₄ alkyl group or a C₁₋₃ alkyl group that is a straight or branched C₁₋₄, preferably a C₁₋₃ hydrocarbon group, and more preferably a methyl group.
The "C₁₋₆ alkyl-pyridinyl-C₁₋₆ alkyl group" in R² and R² in the general formulae (II) or (III) is preferably the "C₁₋₄ alkyl-pyridinyl-C₁₋₄ alkyl group", more preferably the "C₁₋₄ alkyl-pyridin-2-yl-C₁₋₄ alkyl group", and further preferably a 5-ethylpyridin-2-ylethyl group.

The "C₁₋₆ alkoxy-C₁₋₆ alkyl group" in R³ in the general formula (II) is preferably the "C₁₋₄ alkoxy-C₁₋₄ alkyl group", more preferably a group in which the end of the C₁₋₄ alkyl group is substituted with a C₁₋₄ alkoxy group, further preferably a methoxyethyl group.
The "C₃₋₈ cycloalkyl-C₁₋₆ alkyl group" in R³ in the general formula (II) is preferably the "C₃₋₈ alkoxy-C₁₋₄ alkyl group", and more preferably a cyclohexylmethyl group.
The "C₆₋₁₀ aryl-C₁₋₆ alkyl group" in R³ in the general formula. (II) is preferably the "C₆₋₁₀ aryl-C₁₋₄ alkyl group", and more preferably a benzyl group.

The C₁₋₆ alkylene chain in Z in the general formula (IV) is preferably a methylene chain, an ethylene chain, or a trimethylene chain.
The C₆₋₁₀ aryl group in R¹¹ in the general formula (IV) is preferably a phenyl group.
The C₁₋₆ alkylthio group in R¹¹ in the general formula (IV) is preferably a C₁₋₄ alkylthio group, and more preferably a methylthio group.
The C₁₋₆ alkylsulfinyl group in R¹¹ in the general formula (IV) is preferably a C₁₋₄ alkylsulfinyl group, and more preferably a methylsulfinyl group.
The C₁₋₆ alkylsulfonyl group in R¹¹ in the general formula (IV) is preferably a C₁₋₄ alkylsulfonyl group, and more preferably a methylsulfonyl group.
The m in the general formula (IV) is preferably 1.

Preferable specific examples of the formula (IV) in R³ in the general formula (II) include a 2-pyridyl group, a 4-benzyloxyphenyl group, a 4-ethoxyphenyl group, a 4-isopropoxyphenyl group, a 5-methoxypyrimidin-2-yl group, a 5-(benzyloxy)pyrimidin-2-yl group, a 5-ethoxypyrimidin-2-yl group, a 5-(4-amino-4-oxobutoxy)pyrimidin-2-yl group, a 5-{2-(methylthio)ethoxy}pyrimidin-2-yl group, a 5-{2-(methylsulfinyl)ethoxy}pyrimidin-2-yl group, a 5-{2-(methylsulfonyl)ethoxy}pyrimidin-2-yl group, a 5-hydroxypyrimidin-2-yl group, and the like.

The "C₁₋₆ alkoxy-C₁₋₆ alkyl group" in R⁴ in the general formula (III) is preferably the "C₁₋₄ alkoxy-C₁₋₄ alkyl group", and more preferably a methoxyethyl group.
The "C₆₋₁₀ aryl-C₁₋₆ alkyl group" in R⁴ in the general formula (III) is preferably the "C₆₋₁₀ aryl-C₁₋₄ alkyl group", and more preferably a benzyl group.

The C₁₋₆ alkyl group in R⁵ and R⁶ in the general formula (I) is preferably a C₁₋₄ alkyl group, and more preferably a n-propyl group.

The "C₂₋₇ alkoxycarbonyl group" in R⁷ in the general formula (I) is preferably a methoxycarbonyl group or an ethoxycarbonyl group.
The "C₁₋₆ alkylsulfonyl-carbamoyl group" in R⁷ in the general formula (I) is preferably a N-(C₁₋₄ alkylsulfonyl)carbamoyl group, and more preferably a N-(methylsulfonyl)carbamoyl group.
The tetrazolyl group in R⁷ in the general formula (I) is preferably a 1H-tetrazol-5-yl group.
The oxooxadiazolyl group in R⁷ in the general formula (I) is preferably a 1,2,4-oxadiazol-5(4H)-on-3-yl group.

The halogen group in R⁸ R⁹, and R¹⁰ in the general formula (I) is preferably a chlorine atom.
The C₁₋₆ alkoxy group in. R⁸, R⁹, and R¹⁰ in the general formula (I) is preferably a C₁₋₄ alkoxy group, and more preferably a methoxy group.

The n in the general formula (I) is preferably 1.

More preferable compounds of the α-phenoxybenzeneacetic acid derivative represented by the general formula (I) include compounds that are selected from a group consisting of:
2-[4-[{4-butyl-2-methyl-6-oxo-1-(pyridin-2-yl)-1,6-di hydropyrimidin-5-yl}methyl]-2,6-dipropylphenoxy]-2-phenylac etic acid (refer to Example 1),
2-[4-[{2-methyl-6-oxo-4-propyl-1-(pyridin-2-yl)-1,6-d ihydropyrimidin-5-yl}methyl]-2,6-dipropylphenoxy]-2-phenyla cetic acid (refer to Example 2),
2-[4-[{4-ethyl-2-methyl-6-oxo-1-(pyridin-2-yl)-1,6-di hydropyrimidin-5-yl}methyl]-2,6-dipropylphenoxy]-2-phenylac etic acid (refer to Example 3),
6-butyl-2-methyl-5-[4-{phenyl(1H-tetrazol-5-yl)methox y}-3,5-dipropylbenzyl]-3-(pyridin-2-yl)pyrimidin-4(3H)-one (refer to Example 4),
3-[[4-[{4-butyl-2-methyl-6-oxo-1-(pyridin-2-yl)-1,6-d ihydropyrimidin-5-yl}methyl]-2,6-dipropylphenoxy](phenyl)me thyl]-1,2,4-oxadiazol-5(4H)-one (refer to Example 5),
2-[4-[{4-butyl-2--methyl-6-oxo-1-(pyridin-2-yl)-1,6-di hydropyrimidin-5-yl}methyl]-2,6-dipropylphenoxy]-N-(methyls ulfonyl)-2-phenyl acetoamide (refer to Example 6),
2-[4-[{4-butyl-2-methyl-6-oxo-1-(pyridin-2-yl)-1,6-di hydropyrimidin-5-yl}methyl]-2,6-dipropylphenoxy]-2-(2-chlor ophenyl)acetic acid (refer to Example 7),
2-[4-[{4-butyl-2-methyl-6-oxo-1-(pyridin-2-yl)-1,6-di hydropyrimidin-5-yl}methyl]-2,6-dipropylphenoxy]-2-(3-chlor ophenyl)acetic acid (refer to Example 8),
2-[4-[{4-butyl-2-methyl-6-oxo-1-(pyridin-2-yl)-1,6-di hydropyrimidin-5-yl}methyl]-2,6-dipropylphenoxy]-2-(4-chlor ophenyl)acetic acid (refer to Example 9),
2-[4-[{4-butyl-2-methyl-6-oxo-1-(pyridin-2-yl)-1,6-di hydropyrimidin-5-yl}methyl]-2,6-dipropylphenoxy]-2-(2-metho xyphenyl)acetic acid (refer to Example 10),
6-butyl-5-[4-{(2-chlorophenyl)(1H-tetrazol-5-yl)metho xy}-3,5-dipropylbenzyl]-2-methyl-3-(pyridin-2-yl-)pyrimidin-4(3H)-one (refer to Example 11),

2-[4-[[1-{4-(benzyloxy)phenyl}-4-butyl-2-methyl-6-oxo -1,6-dihydropyrimidin-5-yl]methyl]-2,6-dipropylphenoxy]-2-p henylacetic acid (refer to Example 12),
2-[4-[{4-butyl-1-(4-ethoxyphenyl)-2-methyl-6-oxo-1,6-dihydropyrimidin-5-yl}methyl]-2,6-dipropylphenoxy]-2-phenyl acetic acid (refer to Example 13),
2-[4-[{4-butyl-1-(4-isopropoxyphenyl)-2-methyl-6-oxo-1,6-dihydropyrimidin-5-yl}methyl]-2,6-dipropylphenoxy]-2-ph enylacetic acid (refer to Example 14),
2-[4-[{4-butyl-1-(5-methoxypyrimidin-2-yl)-2-methyl-6 -oxo-1,6-dihydropyrimidin-5-yl}methyl]-2,6-dipropylphenoxy] -2-phenylacetic acid (refer to Example 15),
(-)-2-[4-[{4-butyl-1-(5-methoxypyrimidin-2-yl)-2-meth yl-6-oxo-1,6-dihydropyrimidin-5-yl}methyl]-2,6-dipropylphen oxy]-2-phenylacetic acid (refer to Example 16),
(+)-2-[4-[{4-butyl-1-(5-methoxypyrimidin-2-yl)-2-meth yl-6-oxo-1,6-dihydropyrimidin-5-yl}methyl]-2,6-dipropylphen oxy]-2-phenylacetic acid (refer to Example 17),
2-[4-[{4-butyl-1-(5-methoxypyrimidin-2-yl)-2-methyl-6 -oxo-1,6-dihydropyrimidin-5-yl}methyl]-2,6-dipropylphenoxy] -2-(4-chlorophenyl)acetic acid (refer to Example 18),
2-[4-[[1-{5-(benzyloxy)pyrimidin-2-yl}-4-butyl-2-meth yl-6-oxo-1,6-dihydropyrimidin-5-yl]methyl]-2,6-dipropylphen oxy]-2-phenylacetic acid (refer to Example 19),
2-[4-[{4-butyl-1-(5-ethoxypyrimidin-2-yl)-2-methyl-6-oxo-1,6-dihydropyrimidin-5-yl}methyl]-2,6-dipropylphenoxy]-2-phenylacetic acid (refer to Example 20),
(-)-2-[4-[{4-butyl-1-(5-ethoxypyrimidin-2-yl)-2-methy 1-6-oxo-1,6-dihydropyrimidin-5-yl}methyl]-2,6-dipropylpheno xy]-2-phenylacetic acid (refer to Example 21),
(+)-2-[4-[{4-butyl-1-(5-ethoxypyrimidin-2-yl)-2-methy 1-6-oxo-1,6-dihydropyrimidin-5-yl}methyl]-2,6-dipropylpheno xy]-2-phenylacetic acid (refer to Example 22),

2-[4-[[1-{5-(4-amino-4-oxobutoxy)pyrimidin-2-yl}-4-bu tyl-2-methyl-6-oxo-1,6-dihydropyrimidin-5-yl]methyl]-2,6-di propylphenoxy]-2-phenylacetic acid (refer to Example 23),
2-[4-[[4-butyl-2-methyl-1-[5-{2-(methylthio)ethoxy}py rimidin-2-yl]-6-oxo-1,6-dihydropyrimidin-5-yl]methyl]-2,6-d ipropylphenoxy]-2-phenylacetic acid (refer to Example 24),
2-[4-[[4-butyl-2-methyl-1-[5-{2-(methylsulfinyl)ethox y}pyrimidin-2-yl]-6-oxo-1,6-dihydropyrimidin-5-yl]methyl]-2 ,6-dipropylphenoxy]-2-phenylacetic acid (refer to Example 25),
2-[4-[[4-butyl-2-methyl-1-[5-{2-(methylsulfonyl)ethox y}pyrimidin-2-yl]-6-oxo-1,6-dihydropyrimidin-5-yl]methyl]-2 ,6-dipropylphenoxy]-2-phenylacetic acid (refer to Example 26),
2-[4-[{4-butyl-1-(5-hydroxypyrimidin-2-yl)-2-methyl-6 -oxo-1,6-dihydropyrimidin-5-yl}methyl]-2,6-dipropylphenoxy] -2-phenylacetic acid (refer to Example 27),
2-[4-{(1-benzyl-4-butyl-2-methyl-6-oxo-1,6-dihydropyr imidin-5-yl)methyl}-2,6-dipropylphenoxy]-2-phenylacetic acid (refer to Example 28),
2-[4-[{4-(benzyloxy)-6-butyl-2-methylpyrimidin-5-yl}m ethyl]-2,6-dipropylphenoxy]-2-phenylacetic acid (refer to Example 29),
2-[4-[{4-butyl-1-(cyclohexylmethyl)-2-methyl-6-oxo-1, 6-dihydropyrimidin-5-yl}methyl]-2,6-dipropylphenoxy]-2-phen ylacetic acid (refer to Example 30),
2-[4-[[4-butyl-2-{2-(5-ethylpyridin-2-yl)ethyl}-6-oxo -1,6-dihydropyrimidin-5-yl]methyl]-2,6-dipropylphenoxy]-2-p henylacetic acid (refer to Example 31),
2-[4-[{4-butyl-6-(2-methoxyethoxy)-2-methylpyrimidin-5-yl}methyl]-2,6-dipropylphenoxy]-2-phenylacetic acid (refer to Example 32), and
2-[4-[{4-butyl-1-(2-methoxyethyl)-2-methyl-6-oxo-1,6-dihydropyrimidin-5-yl}methyl]-2,6-dipropylphenoxy]-2-phenyl acetic acid (refer to Example 33).

Further preferable compounds of the α-phenoxybenzeneacetic acid derivative represented by the general formula (I) include compounds that are selected from a group consisting of:
2-[4-[{4--butyl-2-methyl-6-oxo-1-(pyridin-2-yl)-1,6-di hydropyrimidin-5-yl}methyl]-2,6-dipropylphenoxy]-2-phenylac etic acid (refer to Example 1),
2-[4-[{2-methyl-6-oxo-4-propyl-1-(pyridin-2-yl)-1,6-d ihydropyrimidin-5-yl}methyl]-2,6-dipropylphenoxy]-2-phenyla cetic acid (refer to Example 2),
2-[4-[{4-butyl-2-methyl-6-oxo-1-(pyridin-2-yl)-1,6-di hydropyrimidin-5-yl}methyl]-2,6-dipropylphenoxy]-2-(4-chlor ophenyl)acetic acid (refer to Example 9),
2-[4-[{4-butyl-1-(4-isopropoxyphenyl)-2-methyl-6-oxo-1,6-dihydropyrimidin-5-yl}methyl]-2,6-dipropylphenoxy]-2-ph enylacetic acid (refer to Example 14),
2-[4-[{4-butyl-1-(5-methoxypyrimidin-2-yl)-2-methyl-6 -oxo-1,6-dihydropyrimidin-5-yl}methyl]-2,6-dipropylphenoxy] -2-phenylacetic acid (refer to Example 15),
(-)-2-[4-[{4-butyl-1-(5-methoxypyrimidin-2-yl)-2-meth yl-6-oxo-1,6-dihydropyrimidin-5-yl}methyl]-2,6-dipropylphen oxy]-2-phenylacetic acid (refer to Example 16),
(+)-2-[4-[{4-butyl-1-(5-methoxypyrimidin-2-yl)-2-meth yl-6-oxo-1,6-dihydropyrimidin-5-yl}methyl]-2,6-dipropylphen oxy]-2-phenylacetic acid (refer to Example 17),
2-[4-[{4-butyl-1-(5-ethoxypyrimidin-2-yl)-2-methyl-6-oxo-1,6-dihydropyrimidin-5-yl}methyl]-2,6-dipropylphenoxy] - 2-phenylacetic acid (refer to Example 20),
(-)-2-[4-[{4-butyl-1-(5-ethoxypyrimidin-2-yl)-2-methy 1-6-oxo-1,6-dihydropyrimidin-5-yl}methyl]-2,6-dipropylpheno xy]-2-phenylacetic acid (refer to Example 21),
(+)-2-[4-[{4-butyl-1-(5-ethoxypyrimidin-2-yl)-2-methy 1-6-oxo-1,6-dihydropyrimidin-5-yl}methyl]-2,6-dipropylpheno xy]-2-phenylacetic acid (refer to Example 22),
2-[4-[[4-butyl-2-methyl-1-[5-{2-(methylthio)ethoxy}py rimidin-2-yl]-6-oxo-1,6-dihydropyrimidin-5-yl]methyl]-2,6-d ipropylphenoxy]-2-phenylacetic acid (refer to Example 24),
2-[4-{(1-benzyl-4-butyl-2-methyl-6-oxo-1,6-dihydropyr imidin-5-yl)methyl}-2,6-dipropylphenoxy]-2-phenylacetic acid (refer to Example 28),
2-[4-[{4-butyl-1-(cyclohexylmethyl)-2-methyl-6-oxo-1, 6-dihydropyrimidin-5-yl}methyl]-2,6-dipropylphenoxy]-2-phen ylacetic acid (refer to Example 30),
2-[4-[{4-butyl-6-(2-methoxyethoxy)-2-methylpyrimidin-5-yl}methyl]-2,6-dipropylphenoxy]-2-phenylacetic acid (refer to Example 32), and
2-[4-[{4-butyl-1-(2-methoxyethyl)-2-methyl-6-oxo-1,6-dihydropyrimidin-5-yl}methyl]-2,6-dipropylphenoxy]-2-phenyl acetic acid (refer to Example 33).

When the compound of the present invention has geometrical isomers or optical isomers, such isomers fall within the scope of the present invention. Isolation of these isomers is performed by an ordinary method.

The salt of the compound represented by the general formula (I) is not particularly limited as long as it is a phamaceutically acceptable salt. When the compound is handled as an acidic compound, examples of the salt include, for example, metal salts such as sodium, potassium, magnesium, and calcium; salts with an organic base such as trimethyl amine, triethyl amine, pyridine, picoline, N-methylpyrrolidine, N-methylpiperidine, and N-methylmorpholine; and the like. When the compound is handled as a base compound, examples of the salt include, for example, acid addition salts of a mineral acid such as hydrochloric acid salt, hydrobromic acid salt, hydriodic acid salt, sulfuric acid salt, nitric acid salt, and phosphoric acid salt; acid addition salts of an organic acid such as benzoic acid salt, methane sulfonic acid salt, ethane sulfonic acid salt, benzene sulfonic acid salt, p-toluene sulfonic acid salt, maleic acid salt, fumaric acid salt, tartaric acid salt, citric acid salt, and acetic acid salt; and the like.

Examples of the solvate of the compound represented by the general formula (I) or a salt thereof include, for example, hydrates and the like, but are not limited thereto.

In addition, compounds which are metabolized in a living body and converted into the compounds represented by the aforementioned general formula (I), which are so-called prodrugs, also fall within the scope of the present invention. Examples of groups which form the prodrugs of the compounds of the present invention include the groups described in "Progress in Medicine", vol. 5, pp. 2157 to 2161, 1985, Life Science Medica, and the groups described in "Development of Drugs", vol. 7, Molecular Designs, pp. 163 to 198, 1990, Hirokawa Shoten.

The compound represented by the general formula (I), or a salt thereof, or a solvate thereof can be prepared by various known methods, which methods are not particularly limited. For example, the compound can be prepared according to the following reaction processes. Further, when the following reactions are performed, functional groups other than the reactionsites may be protected beforehand as required, and deprotected in an appropriate stage. Furthermore, each reaction may be performed by an ordinarily used method in each process, and isolation and purification can be performed by a means suitably selected from conventional methods such as crystallization, recrystallization, chromatography, and the like, or a combination thereof.

Preparation method of the compound represented by the general formula (I), or a salt thereof, or a solvate thereof
Among the compounds represented by the general formula (I) of the present invention, a compound represented by formula (Ia) can be prepared by the method described below, but the preparation method is not limited thereto.
Specifically, as shown in the Reaction Scheme 1 described below, a hydroxyl group of an alcoholic derivative (V) is halogenated, and subsequently reacted with β-ketoester (VII), whereby to give oxocarboxylic acid ester (VIII). Subsequently, the oxocarboxylic acid ester (VIII) is reacted with ammonium acetate, and is reacted with acid anhydride (IX), whereby to give acylamino compound (X). The acylamino compound (X) is reacted with an amine compound (XI), whereby to give a pyrimidinone derivative (XII). The pyrimidinone derivative (XII) is deprotected, and then reacted with α-halophenylacetic acid ester (XIV), whereby to give α-phenoxyphenylacetic acid ester (XV). The α-phenoxyphenylacetic acid ester (XV) is hydrolyzed, whereby to prepare the compound represented by formula (Ia) among the compounds represented by the general formula (I) of the present invention.

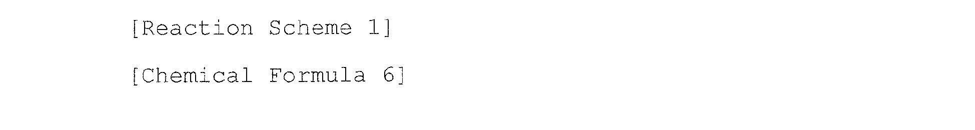

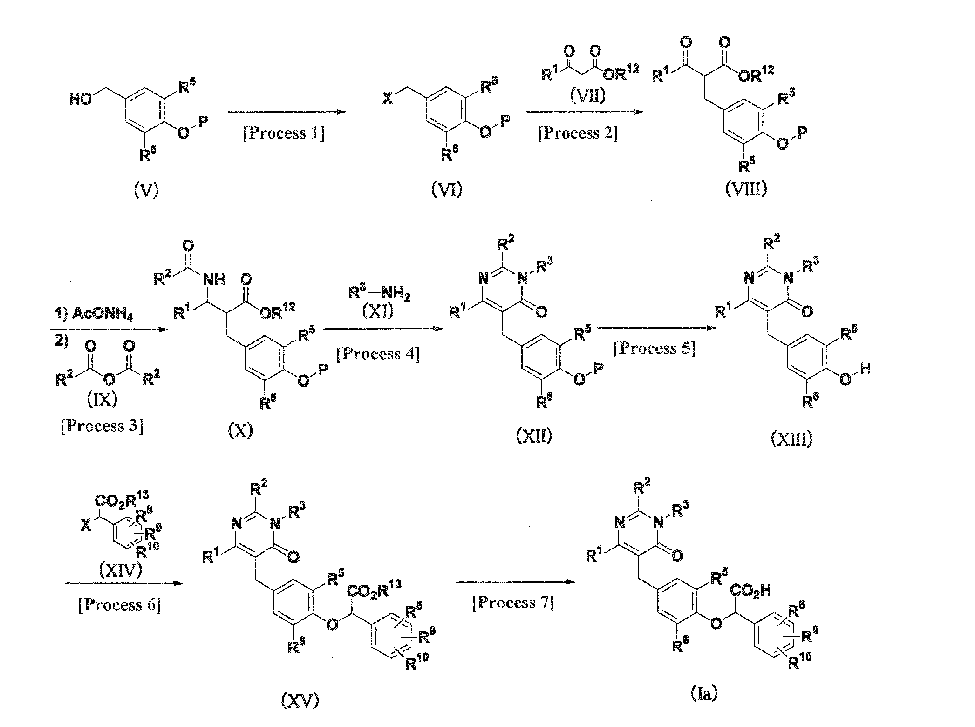

(wherein R¹, R², R³, R⁵, R⁶, R⁸, R⁹, and R¹⁰ are as described above, R¹² and R¹³ represent a protective group of a C₁₋₄ alkyl group or a carboxyl group, P represents a protective group of a hydroxyl group, and X represents a leaving group such as a halogen atom.)

### [Process 1]

The halogenation reaction of the hydroxyl group of the alcoholic derivative represented by the general formula (V) may be performed by reaction with a halogenating agent in a solvent or in the absence of a solvent and in the presence or absence of a base. The halogenating agent is not particularly limited, but examples thereof include a chlorinating agent, a brominating agent, or an iodinating agent such as phosphorus oxychloride, phosphorus pentachloride, triphenyl phosphine dichloride, triphenyl phosphine dibromide, triphenyl phosphite dichloride, triphenyl phosphite dibromide, phosphorus tribromide, thionyl chloride, triphenyl phosphine and carbon tetrachloride, triphenyl phosphine and carbon tetrabromide, triphenyl phosphine, iodine, and imidazole, triphenyl phosphine and N-iodosuccinic acid imide (NIS), and methanesulfonyl chloride and 4-dimethylaminopyridine (DMAP). The solvent is not particularly limited, but use can be made of 1,2-dichloroethane, chloroform, dichloromethane, diethyl ether, ethyl acetate, isopropyl acetate, toluene, benzene, tetrahydrofuran, dioxane, acetonitrile, propionitrile, and the like, which may be used alone or in combination. The base is not particularly limited, but use can be made of organic bases such as pyridine, DMAP, collidine, lutidine, 1,8-diazabicyclo[5.4.0]-7-undecene (DBU), 1,5-asiazabicyclo[4.3.0]-5-nonene (DBN), 1,4-diazabicyclo[2.2.2]octane (DABCO), triethylamine, diisopropylamine, diisopropylethylamine, diisopropylpentylamine, and trimethylamine; alkali metal hydrides such as lithium hydride, sodium hydride, and potassium hydride; alkali metal hydroxides such as lithium hydroxide, sodium hydroxide, and potassium hydroxide; alkali metal carbonates such as lithium carbonate, sodium carbonate, potassium carbonate, and cesium carbonate; and alkali metal bicarbonates such as sodium hydrogencarbonate. The reaction conditions vary depending on raw materials to be used, but generally the reaction is performed at -20 to 180°C, preferably 0°C to 100°C for 1 minute to 24 hours, more preferably 5 minutes to 5 hours, whereby to give a halide (VI).

### [Process 2]

The reaction of the halide (VI), which is obtained by the above-mentioned method, with β-ketoester (VII), may be performed in a solvent in the presence of a base. The solvent is not particularly limited, but use can be made of N,N-dimethylformamide, N-methyl pyrrolidone, dimethyl sulfoxide, dioxane, tetrahydrofuran, acetonitrile, propionitrile, and the like, which may be used alone or in combination. The base is not particularly limited, but for example, use can be made of organic bases such as pyridine, DMAP, collidine, lutidine, DBU, DBN, DABCO, triethylamine, diisopropylamine, diisopropylethylamine, diisopropylpentylamine, and trimethylamine; alkali metal hydrides such as lithium hydride, sodium hydride, and potassium hydride; alkali metal hydroxides such as lithium hydroxide, sodium hydroxide, and potassium hydroxide; alkali metal carbonates such as lithium carbonate, sodium carbonate, potassium carbonate, and cesium carbonate; and alkali metal bicarbonates such as sodium hydrogencarbonate. The reaction conditions vary depending on raw materials to be used, but the reaction is performed generally at --20 to 120°C, preferably 20°C to 100°C for 1 minute to 2 days, more preferably 5 minutes to 24 hours, whereby to give an oxocarboxylic acid ester (VIII).

### [Process 3-1]

The reaction of the oxocarboxylic acid ester (VIII) with ammonium acetate may be performed in a solvent in the presence of an acid. The solvent is not particularly limited, but use can be made of methanol, ethanol, isopropanol, ethyl acetate, isopropyl acetate, toluene, benzene, dioxane, tetrahydrofuran, acetonitrile, propionitrile, N,N-dimethylforma.mide, N-methyl pyrrolidone, dimethyl sulfoxide, and the like, which may be used alone or in combination. The acid is not particularly limited, but for example, use can be made of protonic acids such as acetic acid, trifluoroacetic acid, propionic acid, and benzoic acid, and Lewis acids such as titanium tetrachloride, boron trifluoride, and tin chloride. The reaction conditions vary depending on raw materials to be used, but the reaction is performed generally at 0 to 180°C, preferably 50°C to 150°C for 1 minute to 24 hours, more preferably 5 minutes to 18 hours.

### [Process 3-2]

The reaction of a crude product obtained by distilling the solvent away with acid anhydride (IX) may be performed in the presence of an acid. The acid is not particularly limited, but for example, use can be made of protonic acids such as acetic acid, trifluoroacetic acrid, propionic acid, and benzoic acid. The reaction conditions vary depending on raw materials to be used, but the reaction is performed generally at 0 to 180°C, preferably 50°C to 120°C for 1 minute to 2 days, more preferably 5 minutes to 24 hours, whereby to give acylamino compound (X).

### [Process 4]

The reaction of the acylamino compound (X) obtained by the above-mentioned method with an amine compound (XI) may be performed in a solvent in the presence of trialkyl aluminum. The solvent is not particularly limited, but use can be made of 1,2-dichloroethane, chloroform, dichloromethane, ethyl acetate, isopropyl acetate, toluene, benzene, tetrahydrofuran, dioxane, acetonitrile, propionitrile, and the like, which may be used alone or in combination. As the trialkyl aluminum, use can be made of trimethylaluminum, triethyl aluminum, tripropyl aluminum, and the like. The reaction conditions vary depending on raw materials to be used, but generally the reaction is performed at 0 to 120°C, preferably 50°C to 100°C for 1. minute to 24 hours, more preferably 5 minutes to 20 hours, whereby to give a pyrimidinone derivative (XII).

### [Process 5]

Deprotection of the protective group P of the amine derivative (XII) obtained by the above-mentioned method is not particularly limited, but can be performed by referring to a method generally used for deprotection conditions of the protective group (Protective Groups in Organic Synthesis Fourth Edition, John Wiley & Sons, Inc.), to give a phenol derivative (XIII).

### [Process 6]

The reaction of the phenol derivative (XIII) with α-halophenylacetic acid ester (XIV) may be performed in a solvent and in the presence or absence of a base. The solvent is not particularly limited, but for example, use can be made of tetrahydrofuran, toluene, dioxane, N,N-dimethylformamide, N-methyl pyrrolidone, dichloromethane, chloroform, acetonitrile, propionitrile, and the like, which may be used alone or in combination. The base is not particularly limited, but for example, use can be made of organic bases such as pyridine, DMAP, collidine, lutidine, DBU, DBN, DABCO, triethylamine, diisopropylamine, diisopropylethylamine, diisopropylpentylamine, and trimethylamine; alkali metal hydrides such as lithium hydride, sodium hydride, and potassium hydride; alkali metal hydroxides such as lithium hydroxide, sodium hydroxide, and potassium hydroxide; alkali metal carbonates such as lithium carbonate, sodium carbonate, potassium carbonate, and cesium carbonate; sodium hydrogencarbonate; and the like, The reaction conditions vary depending on raw materials to be used, but generally the reaction is performed at -20 to 100°C, preferably 15 to 80°C for 5 minutes to 2 days, preferably 30 minutes to 24 hours, whereby to give α-phenoxyphenylacetic acid ester (XV).

### [Process 7]

The α-phenoxyphenylacetic acid ester (XV) is subjected to ordinary hydrolysis reaction if necessary, to obtain a compound (Ia). The present reaction may be performed in a solvent and in the presence of a base or acid. The solvent is not particularly limited, but for example, use can be made of tetrahydrofuran, dioxane, methanol, ethanol, water, and the like, which may be used alone or in combination. The base is not particularly limited, but for example, use can be made of alkali metal hydroxides such as lithium hydroxide, sodium hydroxide, and potassium hydroxide; alkali metal carbonates such as lithium carbonate, sodium carbonate, potassium carbonate, and cesium carbonate; trimethylsilyloxy potassium; and the like. The acid is not particularly limited, but use can be made of hydrochloric acid, acetic acid, trifluoroacetic acid, boron tribromide, aluminum chloride, and the like. The reaction conditions vary depending on raw materials to be used, but generally the reaction is performed at -20 to 100°C, preferably 15 to 80°C for 5 minutes to 1 day, preferably 30 minutes to 13 hours, whereby to give a compound (Ia).

In addition, among the compounds (XII), a compound represented by formula (XIIa) can be prepared by the method described below, but the preparation method is not limited thereto.

### [Reaction Scheme 2]

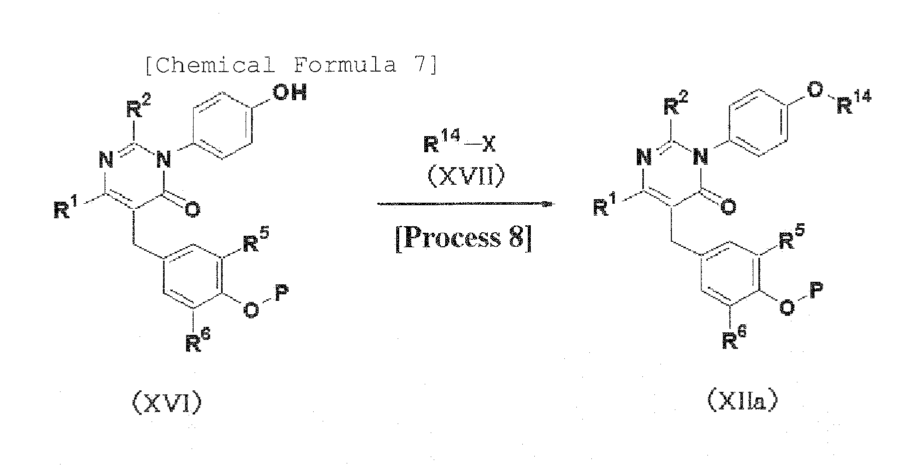

(wherein R¹, R², R⁵, R⁶, P, and X are as described above, and R¹⁴ represents an optionally substituted C₁₋₆ alkyl group.)

### [Process 8]

The reaction of the compound (XVI) with alkyl halide (XVII) may be performed in a solvent and in the presence or absence of a base. The solvent is not particularly limited, but for example, use can be made of tetrahydrofuran, toluene, dioxane, N,N-dimethylformamide, N-methyl pyrrolidone, dichloromethane, chloroform, acetonitrile, propionitrile, and the like, which may be used alone or in combination. The base is not particularly limited, but for example, use can be made of organic bases such as pyridine, DMAP, collidine, lutidine, DBU, DBN, DABCO, triethylamine, diisopropylamine, diisopropylethylamine, diisopropylperltylamine, and trimethylamine; alkali metal hydrides such as lithium hydride, sodium hydride, and potassium hydride; alkali metal hydroxides such as lithium hydroxide, sodium hydroxide, and potassium hydroxide; alkali metal carbonates such as lithium carbonate, sodium carbonate, potassium carbonate and cesium carbonate; and alkali metal bicarbonates such as sodium hydrogencarbonate. The reaction conditions vary depending on raw materials to be used, but generally the reaction is performed at -20 to 150°C, preferably 15 to 100°C for 5 minutes to 2 days, preferably 30 minutes to 24 hours, whereby to give a compound (XIIa).

In addition, among the compounds (XII), a compound represented by formula (XIIb) can be prepared by the method described below, but the preparation method is not limited thereto.

### [Reaction Scheme 3]

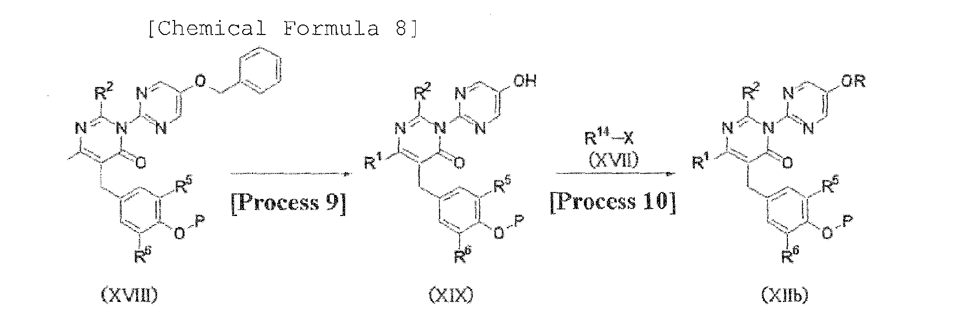

(wherein R¹, R², R⁵, R⁶, R¹⁴, P, and X are as described above.)

### [Process 9]

Deprotection of the benzyl group introduced into the oxygen functional group of the compound (XVTII) may be performed by a method of catalytic hydrogenation. The hydrogen source is not particularly limited, but use can be made of hydrogen, formic acid, ammonium formate, cyclohexadiene, and the like. The catalyst is not particularly limited, but use can be made of palladium carbon, palladium black, platinum black, platinum dioxide, Raney nickel, palladium hydroxide, and the like. The solvent is not particularly limited, but use can be made of methanol, ethanol, isopropanol, ethyl acetate, isopropyl acetate, N,N-dimethylformamide, tetrahydrofuran, dioxane, acetonitrile, acetic acid, water, and the like, which may be used alone or in combination. The reaction conditions vary depending on raw materials to be used, but generally the reaction is performed at 0 to 150°C, preferably 0 to 100°C for 30 minutes to 3 days, preferably 30 minutes to 50 hours, whereby to give a compound (XIX).

### [Process 10]

The reaction of the compound (XIX) with alkyl halide (XVII) may be performed in a solvent and in the presence or absence of a base. The reagent, the solvent, the reaction conditions, and the like used in the reaction are as shown in the above Process 8.

In addition, among the compounds (XII), a compound represented by formula (XIIc) can be prepared by the method described below, but the preparation method is not limited thereto.

### [Reaction Scheme 4]

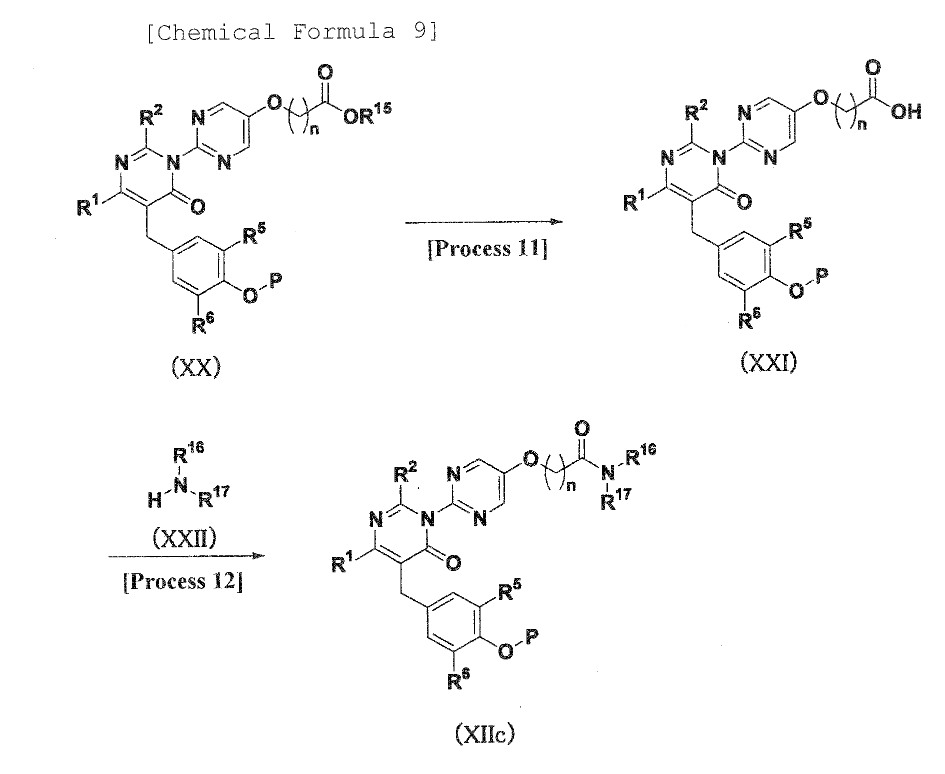

(wherein R¹, R², R⁵, R⁶, P, and X are as described above, R¹⁵ represents a C₁₋₄ alkyl group, and R¹⁶ and R¹⁷ may be the shame or different, and represents a. hydrogen atom or a C₁₋₆ alkyl group.)

### [Process 11]

The hydrolysis reaction of the carboxylic acid ester (XX) may be performed in a solvent and in the presence of a base or acid. The reagent, the solvent, the reaction conditions, and the like used for the reaction are as shown in the above Process 7.

### [Process 12]

The dehydration reaction of the carboxylic acid compound (XXI) with the amine compound (XXII) may be performed with use of a condensation agent in a solvent and in the presence or absence of a base and in the presence or absence of a condensation promoter. The solvent is not particularly limited, but for example, use can be made of 1,2-dichloroethane, chloroform, dichloromethane, ethyl acetate, isopropyl acetate, toluene, benzene, tetrahydrofuran, dioxane, acetonitrile, propionitrile, N,N-dimethylformamide, N-methyl pyrrolidone, and the like. The base is not particularly limited, but for example, use can be made of organic bases such as pyridine, DMAP, collidine, lutidine, DBU, DBN, DABCO, triethylamine, diisopropylethylamine, diisopropylpentylamine, and trimethylamine; alkali metal hydrides such as lithium hydride, sodium hydride, and potassium hydride; alkali metal hydroxides such as lithium hydroxide, sodium hydroxide, and potassium hydroxide; alkali metal carbonates such as lithium carbonate, sodium carbonate, potassium carbonate, and cesium carbonate; alkali metal bicarbonates such as sodium hydrogencarbonate and potassium hydrogencarbonate; and the like. The condensation promoter is not particularly limited, but use can be made of DMAP, 1-hydroxy-7-azabenzotriazole (HOAt), 1-hydroxybenzotriazole (HOBt), 3-hydroxy-3,4-dihydro-4-oxo-1,2,3-benzotriazole (HODhbt), N-hydroxy-5-norbornen-2,3-dicarboxyimide (HONB), pentafluorophenol (HOPfp), N-hydroxyphthalimide (HOPht), N-hydroxysuccinic acid imide (HOSu), and the like. The condensation agent is not particularly limited, but use can be made of N,N'-dicyclohexylcarbodiimide (DCC), N,N'-diisopropyl carbodiimide (DIPCI), 1-ethyl-3-(3-dimethylaminopropyl) carbodiimide (WSCI), 1-ethyl-3-(3-dimethylaminopropyl) carbodiimide hydrochloride (WSC·HCl), diethyl cyanophosphate (DEPC), benzatriazol-1-yloxy-tris(dimethylamino)phosphonium hexafluorophosphate (BOP), benzotriazol-1-yloxy-tris(pyrrolidinylamino)phosphonium hexafluorophosphate (PyBOP), 2-(1H-benzotriazol-1-yl)-1,1,3,3-tetramethyluronium tetrafluoroborate (TBTU), and the like. The reaction conditions vary depending on raw materials to be used, but generally the reaction is performed at-20 to 100°C, preferably 0 to 40°C for 5 minutes to 30 hours, preferably for 2 hours to 20 hours, whereby to give a compound (XIIc) . In addition, the carboxylic acid compound (XXI) used at this time may be induced into acid halide, and then reacted with the amine compound (XXII).

In addition, among the compounds (XII), compounds represented by formulae (XIID) and (XIIe) and a compound represented by formula (XXVII) can be prepared by the method described below, but the preparation method is not limited thereto.

### [Reaction Scheme 5]

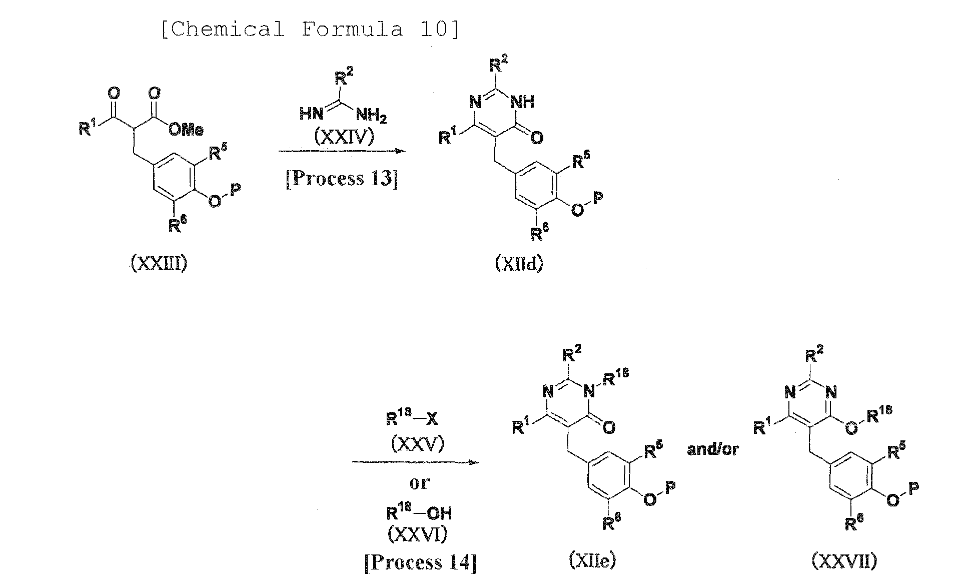

(wherein R¹, R², R⁵, R⁶, P, and X are as described above, and R¹⁸ is as described above for R³ or R⁴.)

### [Process 13]

The reaction of the oxocarboxylic acid ester (XXIII) with the amidine derivative (XXIV) may be performed in a solvent and in the presence of a base. The solvent is not particularly limited, but for example, use can be made of methanol, ethanol, isopropanol, ethyl acetate, isopropyl acetate, toluene, benzene, dioxane, tetrahydrofuran, acetonitrile, propionitrile, N,N-dimethylformamide, N-methyl pyrrolidone, dimethyl sulfoxide, and the like, which may be used alone or in combination. The base is not particularly limited, but for example, use can be made of alkali metal hydrides such as lithium hydride, sodium hydride, and potassium hydride; alkali metals such as metal lithium, metal sodium, and metal potassium; alkali metal hydroxides such as lithium hydroxide, sodium hydroxide, and potassium hydroxide; alkali metal carbonates such as lithium carbonate, sodium carbonate, potassium carbonate, and cesium carbonate; metal amides such as lithium diisopropyl amide, sodium diisopropyl amide, potassium diisopropyl amide, lithium hexamethyl disilazide, sodium hexamethyl disilazide, and potassium hexamethyl disilazide; and metal alcoholates such as sodium methoxide, potassium methoxide, sodium t-butoxide, potassium t-butoxide, n-butyl lithium, s-butyl lithium, and t-butyl lithium. The reaction conditions vary depending on raw materials to be used, but generally the reaction is performed at 0 to 180°C, preferably 50 to 120°C for 1 minute to 24 hours, more preferably 5 minutes to 12 hours, whereby to give a pyrimidinone derivative (XIId).

### [Process 14]

The reaction of a pyrimidinone derivative (XIId) with alkyl halide (XXV), or Mitsunobu reaction of a pyrimidinone derivative (XIId) with an alcohol compound (XXVI) gives a compound (Xlle) and/or compound (XXVII). The reaction of the pyrimidinone derivative (XIId) with alkyl halide (XXV) may be performed in a solvent and in the presence or absence of a base. The solvent is not particularly limited, but for example, use can be made of tetrahydrofuran, toluene, dioxane, N,N-dimethylformamide, N-methyl pyrrolidone, dichloromethane, chloroform, acetonitrile, propionitrile, and the like, which may be used alone or in combination. The base is not particularly limited, but for example, use can be made of organic bases such as pyridine, DMAP, collidine, lutidine, DBU, DBN, DABCO, triethylamine, diisopropylamine, diisopropylethylamine, diisopropylpentylamine, and trimethylamine; alkali metal hydrides such as lithium hydride, sodium hydride and potassium hydride; alkali metal hydroxides such as lithium hydroxide, sodium hydroxide, and potassium hydroxide; alkali metal carbonates such as lithium carbonate, sodium carbonate, potassium carbonate, and cesium carbonate; sodium hydrogencarbonate; and the like. The reaction conditions vary depending on raw materials to be used, but generally the reaction is performed at -20 to 150°C, preferably 15 to 100°C for 5 minutes to 2 days, preferably 30 minutes to 24 hours, whereby to give a compound (XIIe) and/or a compound (XXVII).

The reaction of the pyrimidinone derivative (XIId) with the alcohol compound (XXVI) may be performed with use of a phosphine reagent and an azo reagent or ethylene dicarboxylic acid reagent, or with use of a phosphonium ylide reagent in a solvent. The phosphine reagent is not particularly limited, but use can be made of trialkyl phosphine or triaryl phosphine, specifically trimethyl phosphine, triethyl phosphine, tripropyl phosphine, triisopropyl phosphine, tributyl phosphine, triisobutyl phosphine, tricyclohexyl phosphine, triphenyl phosphine, diphenylphosphino polystyrene, and the like The azo reagent is not particularly limited, but use can be made of diethyl azodicarboxylate (DEAD), diisopropyl azodicarboxylate, 1,1-(azodicarbonyl) piperidine (ADDP), 1,1'-azobis(N,N'-diisopropyl formamide) (TIPA), 1 , 6-dimethyl-1, 5, 7-hexahydro-1,4, 6-tetrazocin-2,5-dione (DHAD), and the like. The ethylene dicarboxylic acid reagent is not particularly limited, but use can be made of dimethyl maleate, diethyl maleate, dimethyl fumarate, diethyl fumarate, and the like. The solvent is not particularly limited, but use can be made of N,N'-dimethylformamide, tetrahydrofuran, dioxane, acetonitrile, propionitrile, nitromethane, acetone, ethyl acetate, isopropyl acetate, benzene, toluene, chlorobenzene, chloroform, dichloromethane, 1,2-dichloroethane, and the like, which may be used alone or in combination. The reaction conditions vary depending on raw materials to be used, but generally the reaction is performed at 0 to 120°C, preferably 0 to 100°C for 30 minutes to 3 days, preferably 30 minutes to 50 hours, whereby to give a compound (XIIe) and/or a compound (XXXVII).

The compound (XXVII) can be converted to a compound represented by the general formula (I) of the present invention wherein Q is formula (III), by using similar methods to those of Processes 5 to 7.

In addition, among the compounds (I), a compound represented by formula (Ib) or formula (Ic) can be prepared by the method described below, but the preparation method is not limited thereto.

### [Reaction Scheme 6]

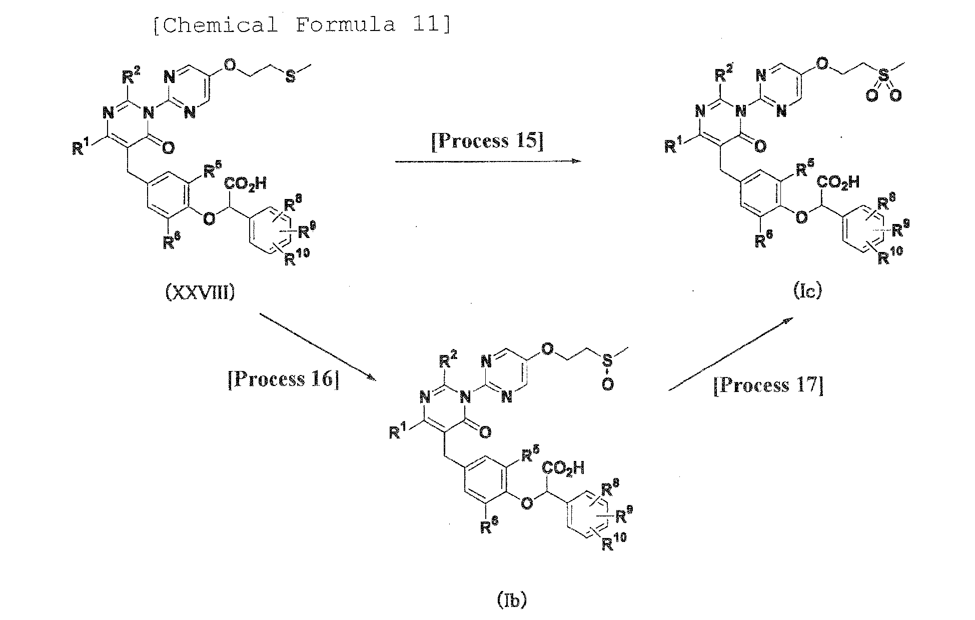

(wherein R¹, R², R⁵, R⁶, R⁸, R⁹, and R¹⁰ are as described above.)

### [Processes 15 to 17]

Oxidation reaction of the compound (XXVIII) may be performed by adopting an ordinary method of converting a sulfur atom to a sulfinyl group or sulfonyl group, for example, oxidation reaction by hydrogen peroxide solution using a catalytic amount of sodium tungstate, molybdenum dichloride dioxide, or tantalum pentachloride, or by using sodium periodinate, potassium periodinate, meta-chloroperbenzoic acid (mCPBA), pyridinium chlorochromate (PCC), pyridinium dichromate (PDC), NCS, NBS, NIS, iodine, bromine, and the like. The solvent is not particularly limited, but for example, use can be made of water, methanol, ethanol, isopropanol, acetonitrile, acetone, tetrahydrofuran, dichloromethane, chloroform, 1,2-dichloroethane, carbon tetrachloride, N,N-dimethylformamide, acetic acid, and the like, which may be used alone or in combination.

In addition, (Ic) can be also prepared from (Ib) obtained in the above-mentioned method using similar oxidation reaction conditions.

In addition, among the compounds (I), a compound represented by formula (Id) can be prepared by the method described below, but the preparation method is not limited thereto.

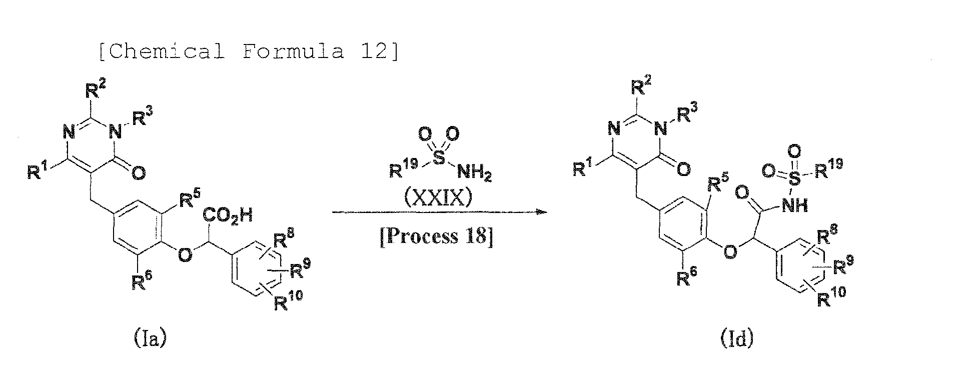

(wherein R¹, R², R³, R⁵, R⁶, R⁸, R⁹, and R¹⁰ are as described above and R¹⁹ represents a C₁₋₆ alkyl group.)

### [Process 18]

The dehydration reaction of the compound (Ia) with alkylsulfonic amide (XXIX) may be performed with use of a condensation agent in a solvent and in the presence or absence of a base and in the presence or absence of a condensation promoter. The solvent is not particularly limited, but for example, use can be made of 1,2-dichloroethane, chloroform, dichloromethane, ethyl acetate, isopropyl acetate, toluene, benzene, tetrahydrofuran, dioxane, acetonitrile, propionitrile, N,N-dimethylformamide, N-methyl pyrrolidone, and the like. The base is not particularly limited, but for example, use can be made of organic bases such as pyridine, DMAP, collidine, lutidine, DBU, DBN, DABCO, triethylamine, diisopropylethylamine, diisopropylpentylamine, and trimethylamine; alkali metal hydrides such as lithium hydride, sodium hydride, and potassium hydride; alkali metal hydroxides such as lithium hydroxide, sodium hydroxide, and potassium hydroxide; alkali metal carbonates such as lithium carbonate, sodium carbonate, potassium carbonate, and cesium carbonate; alkali metal bicarbonates such as sodium hydrogencarbonate and potassium hydrogencarbonate; and the like. The condensation promoter is not particularly limited, but use can be made of DMAP, 1-hydroxy-7-azabenzotriazole (HOAt), 1-hydroxybenzotriazole (HOBt) , 3-hydroxy-3,4-dihydro-4-oxo-1,2,3-benzotriazole (HODhbt), N-hydroxy-5-norbornen-2,3-dicarboxyimide (HONB), pentafluorophenol (HOPfp), N-hydroxyphthalimide (HOPht), N-hydroxysuccinic acid imide (HOSu), and the like. The condensation agent is not particularly limited, but use can be made of N, N' -dicyclohexylcarbodiimide (DCC), N,N' -diisopropyl carbodiimide (DIPCI), 1-ethyl-3-(3-dimethylamiriopropyl) carbodiimide (WSCI), 1-ethyl-3-(3-dimethylaminopropyl) carbodiimide hydrochloride (WSC·HCl), diethyl cyanophosphate (DEPC), benzotriazol-1-yloxy-tris(dimethylamino)phosphonium hexafluorophosphate (BOP), benzotriazol-1-yloxy-tris(pyrrolidinylamino)phosphonium hexafluorophosphate (PyBOP), 2-(1H-benzotriazol-1-yl)-1,1,3,3-tetramethyluronium tetrafluoroborate (TBTU), and the like. The reaction conditions vary depending on raw materials to be used, but generally the reaction is performed at -20 to 100°C, preferably 0 to 40°C for 5 minutes to 30 hours, preferably for 2 hours to 20 hours, whereby to give a compound (Id). In addition, the carboxylic acid compound (Ia) used at this time may be induced into acid halide, and then reacted with alkylsulfonic amide (XXIX).

In addition, among the compounds (I), a. compound represented by formula (Ie) can be prepared by the method described below, but the preparation method is not limited thereto.

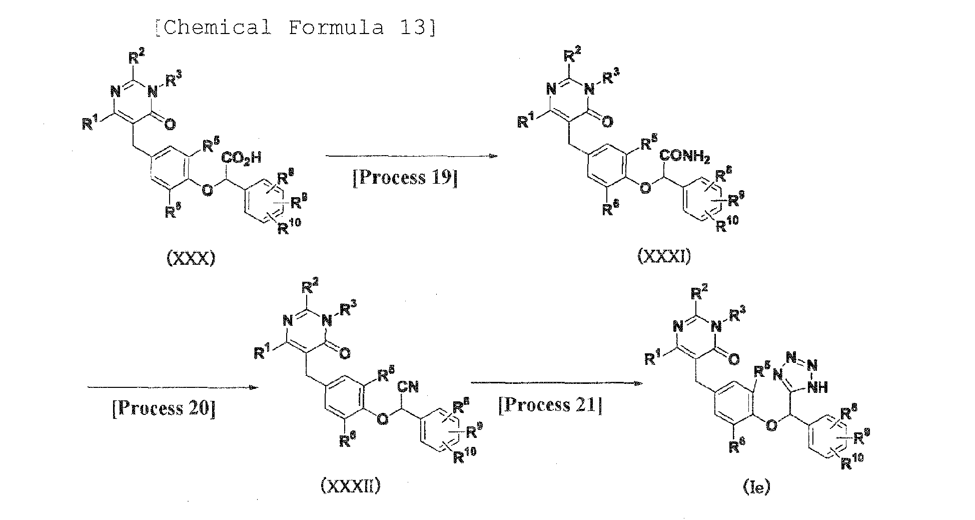

(wherein R¹, R², R³, R⁵, R⁶, R⁸, R⁹, and R¹⁰ are as described above.)

### [Process 19]

The dehydration reaction of the carboxylic acid compound (XXX) with ammonia may be performed with use of a condensation agent in a solvent and in the presence or absence of a base and in the presence or absence of a condensation promoter. The solvent is not particularly limited, but for example, use can be made of 1,2-dichloroethane, chloroform, dichloromethane, ethyl acetate, isopropyl acetate, toluene, benzene, tetrahydrofuran, dioxane, acetonitrile, propionitrile, N,N-dimethylformamide, N-methyl pyrrolidone, and the like. The base is not particularly limited, but for example, use can be made of organic bases such as pyridine, DMAP, collidine, lutidine, DBU, DBN, DABCO, triethylamine, diisopropylethylamine, diisopropylpentylamine, and trimethylamine; alkali metal hydrides such as lithium hydride, sodium hydride, and potassium hydride; alkali metal hydroxides such as lithium hydroxide, sodium hydroxide and potassium hydroxide; alkali metal carbonates such as lithium carbonate, sodium carbonate, potassium carbonate, and cesium carbonate; alkali metal bicarbonates such as sodium hydrogencarbonate and potassium hydrogencarbonate; and the like. The condensation promoter is not particularly limited, but use can be made of DMAP, 1-hydroxy-7-azabenzotriazole (HOAt), 1-hydroxybenzotriazole (HOBt), 3-hydroxy-3,4-dihydro-4-oxo-1,2,3-benzotriazole (HODhbt), N-hydroxy-5-norbornen-2,3-dicarboxyimide (HONB), pentafluorophenol (HOPfp), N-hydroxyphthalimide (HOPht), N-hydroxysuccinic acid imide (HOSu), and the like. The condensation agent is not particularly limited, but use can be made of N,N'-dicyclohexylcarbodiimide (DCC), N,N'-diisopropyl carbodiimide (DIPCI), 1-ethyl-3-(3-dimethylaminopropyl) carbodiimide (WSCI), 1-ethyl-3-(3-dimethylaminopropyl) carbodiimide hydrochloride (WSC·HCl), diethyl cyanophosphate (DEPC), benzotriazol-1-yloxy-tris(dimethylamino)phosphonium hexafluorophosphate (BOP), benzotriazol-1-yloxy-tris(pyrrolidinylamino)phosphonium hexafluorophosphate (PyBOP), 2-(1H-benzotriazol-1-yl)-1,1,3,3-tetramethyluronium tetrafluoroborate (TBTU), and the like. The reaction conditions vary depending on raw materials to be used, but generally the reaction is performed at -20 to 100°C, preferably 0 to 40°C for 5 minutes to 30 hours, preferably for 1 hour to 20 hours, whereby to give an amide compound (XXXI) . In addition, the carboxylic acid compound (XXX) used at this time may be induced into acid halide, and then reacted with ammonia.

### [Process 20]

The conversion of the amide compound (XXXI) into a nitrile derivative (XXXII) may be performed in the presence of a dehydration agent in a solvent or in the absence of a solvent and in the presence or absence of a base. The solvent is not particularly limited, but use can be made of 1, 2-dichloroethane, chloroform, dichloromethane, ethyl acetate, isopropyl acetate, toluene, benzene, tetrahydrofuran, dioxane, acetonitrile, propionitrile, N,N-dimethylformamide, N-methyl pyrrolidone, and the like. The base is not particularly limited, but for example, use can be made of pyridine, DMAP, collidine, lutidine, DBU, DBN, DABCO, triethylamine, diisopropylethylamine, diisopropylpentylamine, trimethylamine, and the like. The dehydration agent is not particularly limited, but use can be made of thionyl chloride, oxalyl chloride, benzene sulfonyl chloride, anhydrous trifluoroacetic acid, diphosphorus pentaoxide, phosphorus oxychloride, carbon tetrachloride and triphenyl phosphine, trichloroacetyl chloride, and the like. The reaction conditions vary depending on raw materials to be used, but generally the reaction is performed at -20 to 180°C, preferably 50 to 120°C for 5 minutes to 30 hours, preferably for 1 hour to 20 hours, whereby to give the nitrile derivative (XXXII).

### [Process 21]

The reaction of the nitrile derivative (XXXII) with an azide compound may be performed in a solvent. As the azide compound, use can be made of trimethyltin azide, tributyl tin azide, triphenyl tin azide, sodium azide, hydrazoic acid, and the like. In addition, trimethylsilyl azide may be also used in the presence of dibutyltin oxide. The solvent is not particularly limited, but use can be made of methanol, ethanol, isopropanol, ethyl acetate, isopropyl acetate, toluene, benzene, dioxane, tetrahydrofuran, acetonitrile, propionitrile, N,N-dimethylformamide, N-methyl pyrrolidone, dimethyl sulfoxide, and the like, which may be used alone or in combination. The reaction conditions vary depending on raw materials to be used, but generally the reaction is performed at 0 to 180°C, preferably 20 to 120°C for 1 minute to 2 weeks, preferably for 1 hour to 3 days, whereby to give a compound (Ie) .

In addition, among the compounds (I), a compound represented by formula (If) can be prepared by the method described below, but the preparation method is not limited thereto.

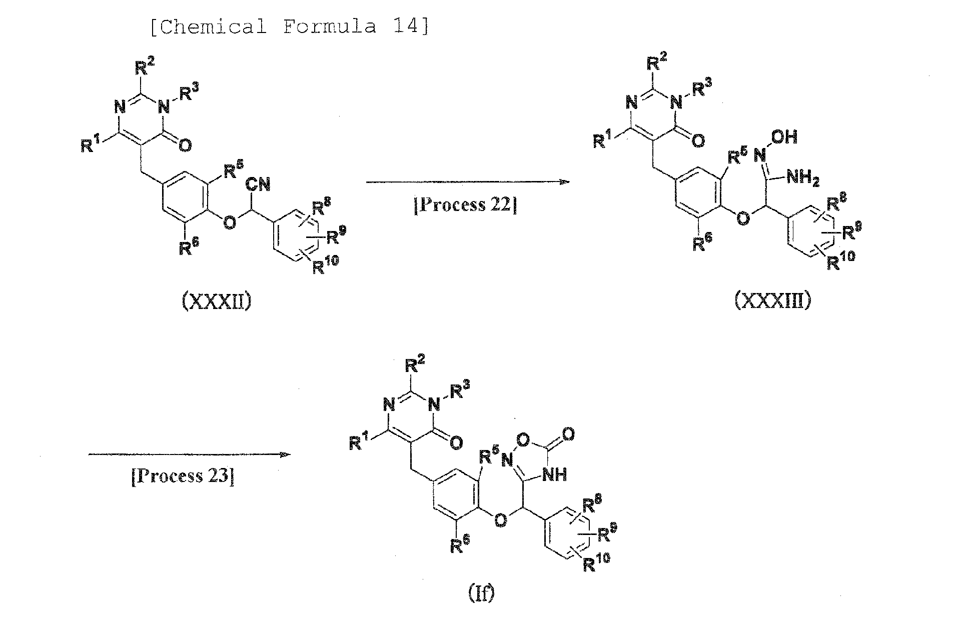

(wherein R¹, R² , R³, R⁵, R⁶, R⁸, R⁹ , and R¹⁰ are as described above.)

### [Process 22]

The reaction of the nitrile derivative (XXXII) with hydroxyl amine may be performed in a solvent. The solvent is not particularly limited, but use can be made of N,N-dimethylformamide, N,N-dimethyl acetoamide, N-methyl pyrrolidone, dimethyl sulfoxide, methanol, ethanol, isopropanol, 1,4-dioxane, tetrahydropyran, and the like, which may be used alone or in combination. When acid salts such as hydroxyl amine hydrochloride, hydroxyl amine sulfate, and hydroxyl amine oxalate is used as the hydroxyl amine, the reaction may be performed under coexistence of a suitable base, for example, potassium carbonate, sodium hydrogencarbonate, sodium hydroxide, triethylamine, sodium methoxide, sodium hydride, and the like in an equal or a bit excessive amount. The reaction conditions vary depending on raw materials to be used, but generally the reaction is performed at 0 to 180°C, preferably 50 to 120°C for 1 minute to 3 days, preferably for 1 hour to 12 hours, whereby to give an amide oxime derivative (XXXIII).

### [Process 23]

The conversion of the amide oxime derivative (XXXIII) to the compound (If) may be performed by using a carbonylation reagent in a solvent and in the presence of a base. The solvent is not particularly limited, but use can be made of 1,2-dichloroethane, chloroform, dichloromethane, ethyl acetate, isopropyl acetate, toluene, benzene, tetrahydrofuran, dioxane, acetonitrile, propionitrile, N,N-dimethylformamide, N,N-dimethyl acetoamide, N-methyl pyrrolidone, diethyl ether, and the like, which may be used alone or in combination. The base is not particularly limited, but for example, use can be made of pyridine, DMAP, collidine, lutidine, DBU, DBN, DABCO, triethylamine, diisopropylethylamine, diisopropylpentylamine, trimethylamine, lithium carbonate, sodium carbonate, potassium carbonate, and cesium carbonate, sodium hydrogencarbonate, potassium hydrogencarbonate, and the like. The carbonylation reagent is not particularly limited, but use can be made of N,N'-carbonyl diimidazole, triphosgene, methyl chlorocarbonate, ethyl chlorocarbonate, and the like. The reaction conditions vary depending on raw materials to be used, but generally the reaction is performed at 0 to 120°C, preferably 15 to 80°C for 5 minutes to 3 days, preferably for 1 hour to 12 hours, whereby to give a compound (If).

Intermediate compounds and target compounds obtained by the aforementioned reactions can be isolated and purified as required by purification methods that are commonly used in the field of synthetic organic chemistry, for example, filtration, extraction, washing, drying, concentration, recrystallization, various types of chromatography, and the like. Intermediate compounds may also be used for successive reactions without particular purification.

Further, various kinds of isomers can be isolated by applying conventional methods utilizing differences of physicochemical properties of the isomers. For example, a racemic mixture can be derived into optically pure isomers by a usual racemic resolution method such as a method of forming diastereomeric salts with a common optically active acid such as tartaric acid, and performing optical resolution, or a method of using optically active column chromatography. Moreover, the resolution of a diastereomer mixture can also be attained by, for example, fractivityal crystallization, various chromatography techniques, and the like. Further, an optically active compound can also be prepared by using a suitable optically active starting material.

The resulting compound (I) can be made into a salt by an ordinary method. The compound may also be obtained as a solvate with a solvent such as a reaction solvent and a recrystallization solvent or a hydrate.

Examples of a dosage form of the pharmaceutical agent comprising the compound of the present invention, a salt thereof or a solvate thereof as an active ingredient include, for example, those for oral administration such as a tablet, a capsule, granules, powders, and a syrup, and those for parenteral administration such as an intravenous injection, an intramuscular injection, a suppository, an inhalant, a transdermal preparation, an eye drop, and a nasal drop. In order to prepare medicinal formulations in such various dosage forms, the active ingredient may be used alone, or may be used in appropriate combination with other pharmaceutically acceptable carriers such as excipients, binders, fillers, disintegrating agents, surfactants, lubricants, dispersing agents, buffering agents, preservatives, corrigents, perfumes, coating agents, and diluents to obtain as a pharmaceutical composition.

Although a dose of the pharmaceutical agent of the present invention may vary depending on the weight, age, sexuality, and symptoms of a patient, and the like, it is generally preferred that 0.1 to 1000 mg, especially 1 to 300 mg, in terms of the compound represented by the general formula (I), may be orally or parenterally administered at one time or several times as divided portions per day for an adult.

### EXAMPLES

The present invention will be further explained with reference to examples. However, the present invention is not limited to these examples. The abbreviations used in the following examples have the following meanings.
s: Singlet
d: Doublet
t: Triplet
q: Quartet
m: Multiplet
br: Broad
J: Coupling constant
Hz: Hertz
CDCl₃: Deuterated chloroform
DMSO-d₆: Deuterated dimethyl sulfoxide
¹H-NMR: Proton nuclear magnetic resonance
IR: Infrared absorption spectrum

### Example 1

### Preparation of 2-[4-[{4-butyl-2-methyl-6-oxo-1-(pyridin-2-yl)-1,6-dihydrop yrimidin-5-yl}methyl]-2,6-dipropylphenoxy]-2-phenylacetic acid

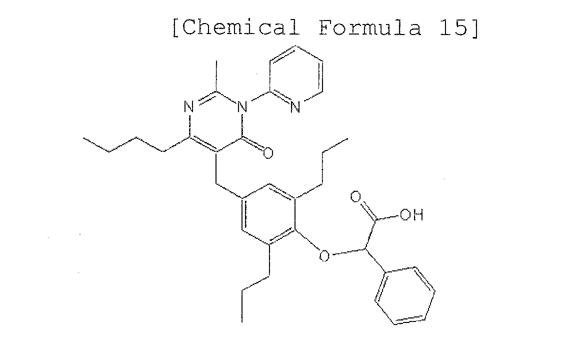

### Process 1:

To a solution of {4-(tert-butyldimethylsilyloxy)-3,5-dipropylphenyl} methanol (3.25 g, 10.1 mmol) in toluene (30 mL), phosphorus tribromide (1.41 mL, 15.0 mmol) was dropped under ice cooling, and the solution was stirred at room temperature for 1 hour. The reaction mixture was added water under ice cooling, and extracted with ethyl acetate. The organic layers were combined, washed with water and brine, dried over anhydrous sodium sulfate, and concentrated in vacuo, to give a crude purified product of 4-(bromomethyl-2,6-diisopropylphenoxy)(tert-butyl)dimethyl silane.

### Process 2:

To a solution of methyl 3-oxoheptanate (1. 90 g, 12.0 mmol) in N,N-dimethylformamide (15 mL), sodium hydride (436 mg, 10.0 mmol) was added under ice cooling, and the mixture was stirred at 0°C for 30 minutes, and added the crude purified product of 4-(bromomethyl-2,6-diisopropylphenoxy)(tert-butyl)dimethyl silane in N,N-dimethylformamide (15 mL), and stirred at room temperature for 18 hours. The reaction mixture was added water, and extracted with ethyl acetate. The organic layers were combined, washed with water and brine, dried over anhydrous sodium sulfate, and concentrated in vacuo. The obteined residue was subjected to a silica gel column chromatography (hexane/ethyl acetate), to give methyl 2-{4-(tert-butyldimethylsilyloxy)-3,5-dipropylbenzyl}-3-oxo heptanate (3.06 g, 66.1%) as a colorless transparent oil.

¹H-NMR(CDCl₃) δ: 0.17 (6H,s), 0.83 (3H, t, J=7 Hz), 0.90 (6H, t, J=7 Hz), 1.00 (9H, s), 1.15-1.24 (2H, m), 1.39-1.58 (6H, m), 2.43-2.52 (6H, m), 3.05 (2H, d, J=8 Hz), 3.67 (3H, s), 3.77 (1H, t, J=8 Hz), 6.75 (2H, s).

### Process 3:

A solution of methyl 2-{4-(tert-butyldimethylsilyloxy)-3,5-dipropylbenzyl}-3-oxo heptanate (3.00 g, 6.60 mmol) and ammonium acetate (3.10g, 39.6 mmol) in toluene (40 mL)-acetic acid (5.0 mL) was refluxed by heating for 5 hours. The solvent was distilled away, and then the residue was added anhydrous acetic acid (1.5 mL) and acetic acid (5.0 mL) at room temperature, and stirred at 70°C for 14 hours. The reaction mixture was added water under ice cooling, and extracted with ethyl acetate. The organic layers were combined, washed with water and brine, dried over anhydrous sodium sulfate, and concentrated in vacuo. The obtained residue was subjected to a silica gel column chromatography (hexane/ethyl acetate), to give methyl 3-acetamido-2-{4-(tert-butyldimethylsilyloxy)-3,5-dipropylb enzyl}-2-heptanate (2.05 g, 61.7%) as a pale yellow oil.

¹H-NMR(CDCl₃) δ: 0.17 (6H,s), 0.86 (3H, t, J=7 Hz), 0.91 (6H, t, J=7 Hz), 1.01 (9H, s), 1.28-1.37 (2H, m), 1.39-1.47 (2H, m), 1.48-1.58 (4H, m), 2.16 (3H, s), 2.43-2.52 (4H, t, J=8 Hz), 2.89 (2H, t, J=8 Hz), 3.58 (2H, s), 3.67 (3H, s), 6.69 (2H, s), 11.8 (1H, s).

### Process 4:

To a solution of 2-aminopyridine (0.64 g, 6.85 mmol) in 1,2-dichloroethane (50 mL), trimethylaluminum (2 mol/L heptane solution, 3.43 mL, 6.85 mmol) was added at room temperature under argon atmosphere, and the solution was stirred at the same temperature for 1 hour. Methyl 3-acetamido-2-{4-(tert-butyldimethylsilyloxy)-3,5-dipropylb enzyl}-2-heptanate (1.15 g, 2.28 mmol) in 1,2-dichloroethane solution (25 mL) was dropped thereto at room temperature, and the reaction mixture was refluxed by heating for 12 hours. To the reaction mixture were added an aqueous solution of ammonium chloride, and chloroform, and the reaction mixture was filtered through a pad of elite. The organic layers of the filtrate were isolated, and the aqueous layer was extracted with chloroform. The organic layers were combined, washed with water and brine, dried over anhydrous sodium sulfate, and concentrated in vacuo. The obtained residue was subjected to a silica gel column chromatography (hexane/acetone), to give 6-butyl-5-{4-(tert-butyldimethylsilyloxy)-3,5-dipropylbenzy l}-2-methyl-3-(pyridin-2-yl)pyrimidin-4(3H)-one (863.3 mg, 69.1%) as pale yellow amorphous.

### Process 5:

To a solution of the obtained 6-butyl-5-{4-(tert-butyldimethylsilyloxy)-3,5-dipropylbenzy l}-2-methyl-3-(pyridin-2-yl)pyrimidin-4(3H)-one in te-trahydrofuran (20 mL), tetra-n-butyl ammonium fluoride (1 mol/L solution in tetrahydrofuran, 2.36 mL, 2.36 mmol) was dropped under ice cooling, and the solution was stirred at room temperature for 1 hour. The reaction mixture was added water, and extracted with ethyl acetate. The organic layers were combined, washed with water and brine, dried over anhydrous sodium sulfate, and concentrated in vacuo. The obtained residue was subjected to a silica gel column chromatography (hexane/acetone), to give 6-butyl-5-(4-hydroxy-3,5-dipropylbenzyl)-2-methyl-3-(pyridi n-2-yl)pyrimidin-4(3H)-one (517.8 mg, 75.6%) as pale yellow amorphous.

¹H-NMR(CDCl₃) δ: 0.91 (3H, t, J=7 Hz), 0.95 (6H, t, J=7 Hz), 1.33-1.42 (2H, m), 1.53-1.64 (6H, m), 2.14 (3H, s), 2.49 (4H, t, J=8 Hz), 2.62 (2H, t, J=8 Hz), 3.80 (2H, s), 4.61 (1H, s), 6.86 (2H, s), 7.35 (1H, d, J=8 Hz), 7.40-7.44 (1H, m), 7.90 (1H, td, J=8, 2 Hz), 8.65-8.67 (1H, m).

### Process 6:

To a solution of 6-butyl-5-(4-hydroxy-3,5-dipropylbenzyl)-2-methyl-3-(pyridi n-2-yl)pyrimidin-4(3H)-one (130.0 mg, 0.30 mmol) in N,N-dimethylformamide (6.0 mL), methyl α-bromophenyl acetate (82.5 mg, 0.36 mmol) and cesium carbonate (146.6 mg, 0.45 mmol) were added, and the solution was stirred at room temperature for 15 hours. The reaction mixture was added water, and extracted with ethyl acetate. The organic layers were combined, washed with water and brine, dried over anhydrous sodium sulfate, and concentrated in vacuo. The obtained residue was subjected to a silica gel column chromatography (hexane/acetone), to give methyl 2-[4-[{4-butyl-2-methyl-6-oxo-1-(pyridin-2-yl)-1,6-dihydrop yrimidin-5-yl}methyl]-2,6-dipropylphenoxy]-2-phenyl acetate (174.5 mg, 100.0%) as pale yellow amorphous.

¹H-NMR(CDCl₃) δ: 0.77 (6H, t, J=7 Hz), 0.90 (3H, t, J=7 Hz), 1.30-1.59 (8H, m), 2.15 (3H, s), 2.24-2.34 (4H, m), 2.59 (2H, t, J=8 Hz), 3.72 (3H, s), 3.81 (2H, s), 5.07 (1H, s), 6.86 (2H, s), 7.33-7.51 (7H, m), 7.92 (1H, td, J=8, 2 Hz), 8.65-8.67 (1H, m) .

### Process 7:

To a solution of methyl 2-[4-[{4-butyl-2-methyl-6-oxo-1-(pyridin-2-yl)-1,6-dihydrop yrimidin-5-yl}methyl]-2,6-dipropylphenoxy]-2-phenyl acetate (174.5 mg, 0.30 mmol) in methanol (12 mL)-water (6 mL), lithium hydroxide monohydrate (125.9 mg, 3.0 mmol) was added, and the solution was stirred at room temperature for 2 hours. The reaction mixture was added water and 1 mol/L hydrochloride, and extracted with ethyl acetate. The organic layers were combined, washed with water and brine, dried over anhydrous sodium sulfate, and concentrated in vacuo. The residue was isolated and purified with silica gel column chromatography (hexane:acetone=2:1), to give the titled compound (147.1 mg, 86.4%) as white amorphous.

¹H-NMR(CDCl₃) δ: 0.73 (6H, t, J=7 Hz), 0.86 (3H, t, J=7 Hz), 1.25-1.53 (8H, m), 2.13 (3H, s), 2.25 (4H, t, J=8 Hz), 2.57 (2H, t, J=8 Hz), 3.79 (2H, s), 5.04 (1H, s), 6.83 (2H, s), 7.31-7.33 (4H, m), 7.39-7.45 (3H, m), 7.89 (1H, td, J=8, 2 Hz), 8.64 (1H, d, J=4 Hz).

### Example 2

### Preparation of 2-[4-[{2-methyl-6-oxo-4-propyl-1-(pyridin-2-yl)-1,6-dihydro pyrimidin-5-yl}methyl]-2,6-dipropylphenoxy]-2-phenylacetic acid

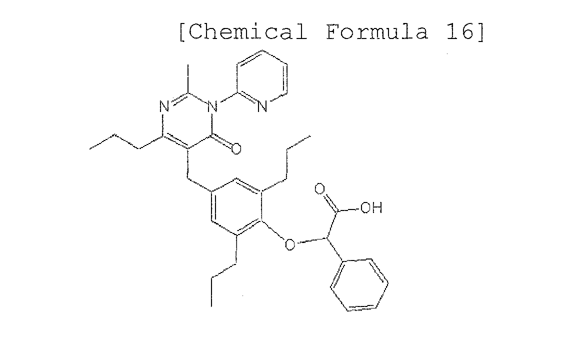

### Process 1:

With use of methyl 3-oxohexanate instead of methyl 3-oxoheptanate, similar reactions and treatments were performed to those of Process 2 of Example 1, to give methyl 2-{4-(tert-butyldimethylsilyloxy)-3,5-dipropylbenzyl}-3-oxo hexanate.

¹H-NMR(CDCl₃) δ: 0.16 (6H,s), 0.89 (6H, t, J=7 Hz), 0.94 (3H, t, J=7 Hz), 0.99 (9H, s), 1.48-1.58 (4H, m), 2.18-2.28 (2H, m), 2.44-2.56 (2H, m), 2.49 (4H, t, J=7 Hz), 3.04 (2H, d, J=7 Hz), 3.67 (3H, s), 3.76 (1H, t, J=7 Hz), 6.74 (2H, s).

### Process 2:

With use of methyl 2-{4-(tert-butyldimethylsilyloxy)-3,5-dipropylbenzyl}-3-oxo hexanate instead of methyl 2-{4-(tert-butyldimethylsilyloxy)--3,5-dipropylbenzyl}-3-oxo heptanate, similar reactions and treatments were performed to those of Process 3 of Example 1, to give methyl 3-acetamido-2-{4-(tert-butyldimethylsilyloxy)-3,5-dipropylb enzyl}-2-hexanate.

¹H-NMR(CDCl₃) δ: 0.17 (6H,s), 0.90 (9H, t, J=7 Hz), 1.00 (9H, s), 1.39-1.49 (2H, m), 1.48-1.58 (4H, m), 2.15 (3H, s), 2.49 (4H, t, J=7 Hz), 2.86 (2H, t, J=8 Hz), 3.58 (2H, s), 3.67 (3H, s), 6.69 (2H, s), 11.78 (1H, s).

### Process 3:

With use of methyl 3-acetamido-2-{4-(tert-butyldimethylsilyloxy)-3,5-dipropylb enzyl}-2-hexanate instead of methyl 3-acetamido-2-{4-(tert-butyldimethylsilyloxy)-3,5-dipropylb enzyl}-2-heptanate, similar reactions and treatments were performed to those of Processes 4 and 5 of Example 1, to give 5-(4-hydroxy-3,5-dipropylbenzyl)-2-methyl-6-propyl-3-(pyrid in-2-yl)pyrimidin-4(3H)-one.

¹H-NMR(CDCl₃) δ: 0.90-0.97 (9H, m), 1.51-1.68 (6H, m), 2.14 (3H, s), 2.46 (4H, t, J=8 Hz), 2.60 (2H, t, J=8 Hz), 3.81 (2H, s), 5.11 (1H, s), 6.84 (2H, s), 7.33 (1H, d, 8Hz), 7.37-7.40 (1H, m), 7.87 (1H, td, J=8, 2 Hz), 8.62-8.64 (1H, m).

### Process 4:

With use of 5-(4-hydroxy-3,5-dipropylbenzyl)-2-methyl-6-propyl-3-(pyrid in-2-yl)pyrimidin-4(3H)-one instead of 6-butyl-5-(4-hydroxy-3,5-dipropylbenzyl)-2-methyl-3-(pyridi n-2-yl)pyrimidin-4(3H)-one, similar reactions and treatments were performed to those of Process 6 of Example 1, to give methyl 2-[4-[{2-methyl-6-oxo-4-prapyl-1-(pyridin-2-yl)-1,6-dihydro pyrimidin-5-yl}methyl]-2,6-dipropylphenoxy]-2-phenyl acetate.

¹H-NMR(CDCl₃) δ: 0.77 (6H, t, J=7 Hz), 0.93 (3H, t, J=7 Hz), 1.37-1.69 (6H, m), 2.15 (3H, s), 2.25-2.36 (4H, m), 2.57 (2H, t, J=8 Hz), 3.73 (3H, s), 3.81 (2H, s), 5.07 (1H, s), 6.86 (2H, s), 7.34-7.51 (7H, m), 7.92 (1H, td, J=8, 2 Hz), 8.67 (1H, dd, J=5, 1 Hz).

### Process 5:

With use of methyl 2-[4-[{2-methyl-6-oxo-4-propyl-l-(pyridin-2-yl)-1,6-dihydro pyrimidin-5-yl}methyl]-2,6-dipropylphenoxy]-2--phenyl acetate instead of methyl 2-[4-[{4-butyl-2-methyl-6-oxo-1-(pyridin-2-yl)-1,6-dihydrop yrimidin-5-yl}methyl]-2,6-dipropylphenoxy]-2-phenyl acetate, similar reactions and treatments were performed to those of Process 7 of Example 1, to give the titled compound (yield: 71.9%).

¹H-NMR(CDCl₃) δ: 0.72 (6H, br), 0.88 (3H, t, J=8 Hz), 1.26-1.57 (6H, m), 2.13 (3H, s), 2.24 (4H, br), 2.55 (2H, t, J=8 Hz), 3.78 (2H, s), 5.03 (1H, s), 6.82 (2H, s), 7.31-7.42 (7H, m), 7.88 (1H, td, J=8, 2 Hz), 8.63-8.67 (1H, m).

### Example 3

Preparation of 2-[4-[{4-ethyl-2-methyl-6-oxo-1-(pyridin-2-yl)-1,6-dihydrop yrimidin-5-yl}methyl]-2,6-dipropyphenoxy]-2-phenylacetic acid

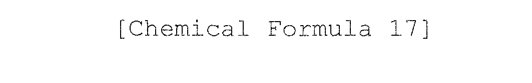

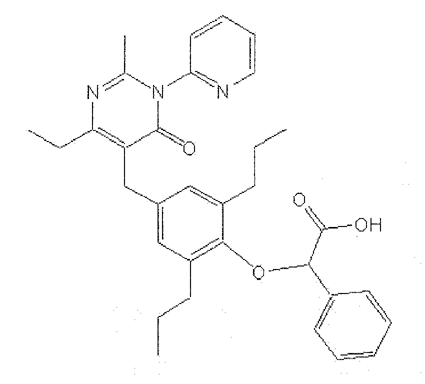

### Process 1:

With use of methyl 3-oxopentanate instead of methyl 3-oxoheptanate, similar reactions and treatments were performed to those of Process 2 of Example 1, to give methyl 2-{4-(tert-butyldimethylsilyloxy)-3,5-dipropylbenzyl}-3-oxo pentanate.

¹H-NMR(CDCl₃) δ: 0.16 (6H,s), 0.89 (6H, t, J=7 Hz), 0.94 (3H, t, J=8 Hz), 0.99 (9H, s), 1.48-1.58 (4H, m), 2.17-2.50 (2H,m), 2.49 (4H, t, J=7 Hz), 3.04 (2H, d, J=7 Hz), 3.67 (3H, s), 3.74 (1H, t, J=7 Hz), 6.74 (2H, s).

### Process 2:

With use of methyl 2-{4-(tert-butyldimethylsilyloxy)-3,5-dipropylbenzyl}-3-oxo pentanate instead of methyl 2-{4-(tert-yldimethylsilyloxy)-3,5-dipropylbenzyl}-3-oxo heptanate, similar reactions and treatments were performed to those of Process 3 of Example 1, to give methyl 3-acetamido-2-{4-(tert-butyldimethylsilyloxy)-3,5-dipropylb enzyl}-2-pentanate.

¹H-NMR(CDCl₃) δ: 0.17 (6H,s), 0.90 (6H, t, J=7 Hz), 1.00 (9H, s), 1.07 (3H, t, J=7 Hz), 1.48-1.58 (4H, m), 2.16 (3H, s), 2.44-2.54 (4H, t, J=7 Hz), 2.93 (2H, q, J=7 Hz), 3.58 (2H, s), 3.67 (3H, s), 6.69 (2H, s), 11.78 (1H, s).

### Process 3:

With use of methyl 3-acetamido-2-{4-(tert-butyldimethylsilyloxy)-3,5-dipropylb enzyl}-2-pentanate instead of methyl 3-acetamido-2-{4-(tert-butyldimethylsilyloxy)-3,5-dipropylb enzyl}-2-heptanate, similar reactions and treatments were performed to those of Processes 4 and 5 of Example 1, to give 6-ethyl-5-(4-hydroxy-3,5-dipropylbenzyl)-2-methyl-3-(pyridi n-2-yl)pyrimidin-4(3H)-one.

¹H-NMR(CDCl₃) δ: 0.93 (6H, t, J=7 Hz), 1.16 (3H, t, J=8 Hz), 1.53-1.62 (4H, m), 2.15 (3H, s), 2.48 (4H, t, J=8 Hz), 2.66 (2H, dd, J=15, 8 Hz), 3.80 (2H, s), 4.83 (1H, s), 6.86 (2H, s), 7.34 (1H, d, 8Hz), 7.41 (1H, dd, J=7, 5Hz), 7.89 (1H, td, J=8, 2 Hz), 8.65 (1H, dd, J=5, 1 Hz).

### Process 4:

With use of 6-ethyl-5-(4-hydroxy-3,5-dipropylbenzyl)-2-methyl-3-(pyridi n-2-yl)pyrimidin-4(3H)-one instead of 6-butyl-5-(4-hydroxy-3,5-dipropylbenzyl)-2-methyl-3-(pyridi n-2-yl)pyrimidin-4 (3H) -one, similar reactions and treatments were performed to those of Process 6 of Example 1, to give methyl 2-[4-[{4-ethyl-2-methyl-6-oxo-1-(pyridin-2-yl)-1,6-dihydrop yrimidin-5-yl}methyl]-2,6-dipropylphenoxy]-2-phenyl acetate.

¹H-NMR(CDCl₃) δ: 0.77 (6H, t, J=7 Hz), 1.13 (3H, t, J=7 Hz), 1.37-1.51 (4H, m), 2.15 (3H, s), 2.28-2.37 (4H, m), 2.62 (2H, dd, J=15, 7 Hz), 3.72 (3H, s), 3.82 (2H, s), 5.08 (1H, s), 6.87 (2H, s), 7.33-7.36 (4H, m), 7.42-7.44 (1H, m), 7.49-7.51 (2H, m), 7.91 (1H, td, J=8, 2 Hz), 8.66 (1H, dd, J=5, 1 Hz).

### Process 5:

With use of methyl 2-[4-[{4-ethyl-2-methyl-6-oxo-1-(pyridin-2-yl)-1,6-dihydrop yrimidin-5-yl}methyl]-2,6-dipropylphenoxy]-2-phenyl acetate instead of methyl 2-[4-[{4-butyl-2-methyl-6-oxo-1-(pyridin-2-yl)-1,6-dihydrop yrimidin-5-yl}methyl]-2,6-dipropylphenoxy]-2-phenyl acetate, similar reactions and treatments were performed to those of Process 7 of Example 1, to give the titled compound (yield: 87.1%).

¹H-NMR(CDCl₃) δ: 0.72 (6H, t, J=7 Hz), 1.08 (3H, t, J=7 Hz), 1.25-1.45 (4H, m), 2.14 (3H, s), 2.25 (4H, br), 2.61 (2H, dd, J=14, 7 Hz), 3.79 (2H, s), 5.03 (1H, s), 6.83 (2H, s), 7.31-7.42 (7H, m), 7.88 (1H, td, J=8, 2 Hz), 8.64 (1H, d, J=4 Hz).

### Example 4

### Preparation of 6-butyl-2-methyl-5-[4-{phenyl(1H-tetrazol-5-yl)methoxy}-3,5 -dipropylbenzyl]-3-(pyridin-2-yl)pyrimidin-4(3H)-one

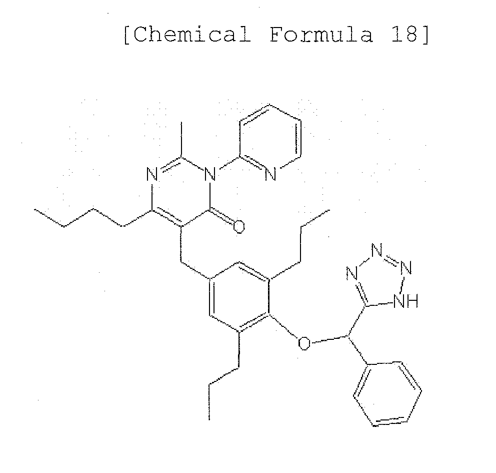

### Process 1:

To a solution of 2-[4-[{4-butyl-2-methyl-6-oxo-1-(pyridin-2-yl)-1,6-dihydrop yrimidin-5-yl}methyl]-2,6-dipropylphenoxy]-2-phenylacetic acid (147.1 mg, 0.26 mmol) obtained in Example 1 in dichloromethane (6.0 mL), 1-hydroxybenzotriazole hydrate (54.3 mg, 0.39 mmol) and 1-ethyl-3-(3-dimethylaminopropyl) carbodiimide hydrochloride (74.8 mg, 0.39 mmol) were added, and the solution was stirred at room temperature for 2 hours. To the reaction mixture was added an aqueous solution of ammonia (1.3 mL), and the solution was stirred at room temperature for 4 hours. The reaction mixture was added water, and extracted with chloroform. The organic layers were combined, washed with an aqueous solution of saturated sodium hydrogencarbonate, water, and brine, dried over anhydrous sodium sulfate, and concentrated in vacuo. The residue was subjected to a silica gel column chromatography (hexane/acetone), to give 2-[4-[{4-butyl-2-methyl-6-oxo-1-(pyridin-2-yl)-1,6-dihydrop yrimidin-5-yl}methyl]-2,6-dipropylphenoxy]-2-phenyl acetoamide (124.0 mg, 84.2%) as pale yellow amorphous.

¹H-NMR(CDCl₃) δ: 0.78 (6H, t, J=7 Hz), 0.89 (3H, t, J=7 Hz), 1.25-1.57 (8H, m), 2.13-2.19 (7H, m), 2,57 (2H, t, J=8 Hz), 3.81 (2H, s), 4.96 (1H, s), 6.58 (1H, d, J=3 Hz), 6.84 (2H, s), 7.16 (1H, d, J=4 Hz), 7.29-7.35 (6H, m), 7.42 (1H, dd, J=7, 5 Hz), 7.93 (1H td, J=8, 2 Hz), 8.66 (1H, dd, J=5, 2 Hz).

### Process 2:

To 2-[4-[{4-butyl-2-methyl-6-oxo-1-(pyridin-2-yl)-1,6-dihydrop yrimidin-5-yl}methyl]-2,6-dipropylphenoxy]-2-phenyl acetoamide (124.0 mg, 0.22 mmol), phosphorus oxychloride (1.0 mL) was added, and the solution was stirred at 100°C for 2 hours. The reaction mixture was cooled, and then added ice water, and extracted with ethyl acetate . The organic layers were combined, washed with water and brine, dried over anhydrous sodium sulfate, and concentrated in vacuo. The residue was subjected to a silica gel column chromatography (hexarie/acetone), to give 2-[4-[{4-butyl-2-methyl-6-oxo-1-(pyridin-2-yl)-1,6-dihydrop yrimidin-5-yl}methyl]-2,6-dipropylphenoxy]-2-phenylacetonit rile (123.4 mg, 102.2%) as pale yellow amorphous.

¹H-NMR(CDCl₃) δ: 0.88 (6H, t, J=7 Hz), 0.91 (3H, t, J=7 Hz), 1.33-1.42 (2H, m), 1.63-1.48 (6H, m), 2.16 (3H, s), 2.43-2.51 (2H, m), 2.58-2.65 (4H, m), 3.85 (2H, s), 5.44 (1H, s), 6.96 (2H, s), 7.37 (1H, d, J=8Hz), 7.42-7.47 (4H, m), 7.58-7.60 (2H, m), 7.89 (1H, td, J=8, 2 Hz), 8.67 (1H, dd, J=5, 1 Hz).

### Process 3:

To a solution of 2-[4-[{4-butyl-2-methyl-6-oxo-1-(pyridin-2-yl)-1,6-dihydrop yrimidin-5-yl}methyl]-2,6-dipropylphenoxy]-2-phenylacetonit rile (19.3 mg, 0.033 mmol) in toluene (1.0 mL), trimethylsilyl azide (213 µL, 1.62 mmol) and dibutyltin oxide (3.9 mg, 0.016 mmol) were added, and the solution was stirred under argon atmosphere at 85°C for 6 hours. The reaction solvent was distilled away and the obtained residue was isolated and purified with silica gel column chromatography (chloroform: methanol = 10 : 1), to give the titled compound (9.0 mg, 46.0%) as pale yellow amorphous.

¹H-NMR (CDCl₃) δ: 0.66 (6H, br), 0.91 (3H, t, J=7 Hz), 1.26-1.41 (6H, m), 1.59 (2H, br), 2.03 (4H, br), 2.16 (3H, s), 2.61 (2H, t, J=8 Hz), 3.78 (2H, s), 6.00 (1H, s), 6.75 (2H, s), 7.52-7.33 (7H, m), 7.85 (1H, br), 8.58 (1H, br).

### Example 5

### Preparation of 3-[[4-[{4-butyl-2-methyl-6-oxo-1-(pyridin-2-yl)-1,6-dihydro pyrimidiri-5-yl}methyl]-2,6-dipropylphenoxy] (phenyl)methyl]-1,2,4-oxadiazol-5 (4H)-one

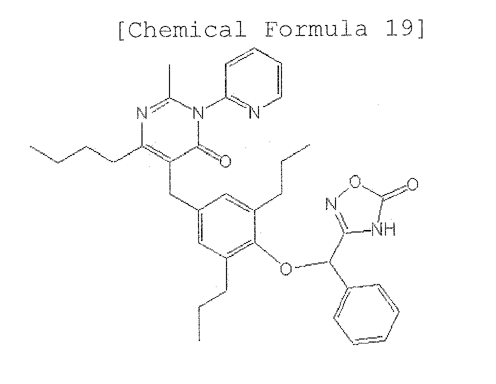

### Process 1:

To a solution of hydroxyl amine hydrochloride (118.1 mg, 1.7 mmol) in dimethyl sulfoxide (1 mL), sodium hydrogencarbonate (168.0 mg, 2.0 mmol) was added, and the solution was stirred at 40°C for 1 hour. To the reaction mixture, a solution of 2-[4-[{4-butyl-2-methyl-6-oxo-1-(pyridin-2-yl)-1,6-dihydrop yrimidin-5-yl}methyl]-2,6-dipropylphenoxy]-2-phenylacetonit rile (50.1 mg, 0.091 mmol) obtained in Process 2 of Example 4 in dimethyl sulfoxide (0.5 mL) was added, and the solution was stirred at 90°C for 5 hours. After completion of the reaction, the reaction mixture was added water and extracted with ethyl acetate. The organic layers were combined, washed with water and brine, dried over anhydrous sodium sulfate, and concentrated in vacuo. The obtained residue was subjected to silica gel column chromatography (chloroform/methanol), to give 2-[4-[{4-butyl-2-methyl-6-oxo-1-(pyridin-2-yl)-1,6-dihydrop yrimidin-5-yl)methyl]-2,6-dipropylphenoxy]-N'-hydroxy-2-phe nylacetoimidamide (39.1 mg, 73.9%) as pale yellow amorphous.

¹H-NMR (CDCl₃) δ: 0.76 (6H, t, J=7 Hz), 0.90 (3H, t, J=7 Hz), 1.31-1.60 (8H, m), 2.14 (3H, s), 2.24-2.39 (4H, m), 2.60 (2H, t, J=8 Hz), 3.80 (2H, s), 4.75 (2H, s), 5.03 (1H, s), 6.86 (2H, s), 7.31-7.50 (7H, m), 7.89 (1H, br), 8.65 (1H, br).

### Process 2:

To a solution of 2-[4-[{4-butyl-2-methyl-6-oxo-1-(pyridin-2-yl)-1,6-dihydrop yrimidin-5-yl}methyl]-2,6-dipropylphenoxy]-N'-hydroxy-2-phe nylacetoimidamide (39.1 mg, 0.067 mmol) in N,N-dimethylformamide (2.0 mL) were added N,N'-carbonyl diimidazole (21.9 mg, 0.14 mmol) and 1,8-diazabicyclo[5.4.0]undeca-7-ene (20. 6 mg, 0.14 mmol), and the solution was stirred at room temperature for 2 hours. After completion of the reaction, the reaction mixture was added water, and extracted with ethyl acetate. The organic layers were combined, washed with water and brine, dried over anhydrous sodium sulfate, and concentrated in vacuo. The obtained residue was isolated and purified with silica gel column chromatography (chloroform : methanol = 10 : 1), to give the titled compound (37.0 mg, 90.9%) as pale yellow amorphous.

¹H-NMR(CDCl₃) δ: 0.72 (6H, t, J= 7Hz), 0.91 (3H, t, J=7 Hz), 1.36-1.42 (6H, m), 1.57 (2H, br), 2.15 (3H, s), 2.23 (4H, t, J=8 Hz), 2.61 (2H, t, J=8 Hz), 5.50 (1H, s), 6.84 (2H, s), 7.32-7.39 (5H, m), 7.49(2H, br), 7.87-7.91 (1H, m), 8.63 (1H, d, J=3 Hz).

### Example 6

### Reparation of 2-[4-[{4-butyl-2-methyl-6-oxo-1-(pyridin-2-yl)-1,6-dihydrop yrimidin-5-yl}methyl]-2,6-dipropylphenoxy]-N-(methylsulfony 1)-2-phenyl acetoamide

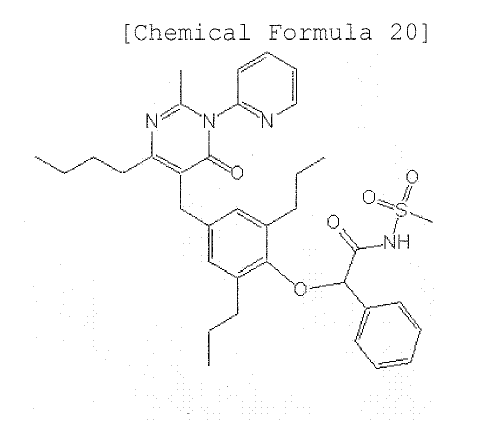

To a solution of 2-[4-[{4-butyl-2-methyl-6-oxo-1-(pyridin-2-yl)-1,6-dihydrop yrimidin-5-yl}methyl]-2,6-dipropylphenoxy]-2-phenylacetic acid (56.7 mg, 0.10 mmol) prepared in Example 1 in N,N-dimethylformamide (1.0 mL) was added N,N'-carbonyl diimidazole (48.6 mg, 0.30 mmol), and the solution was stirred at room temperature for 2 hours. The reaction mixture was added methanesulfonamide (57.1 mg, 0.60 mmol) and 1,8-diazabicyclo[5.4.0]undeca-7-ene (45.7 mg, 0.30 mmol), and stirred at room temperature for 6 hours. The reaction mixture was added 1 mol/L hydrochloric acid, and extracted with chloroform. The organic layers were combined, washed with saline, dried over anhydrous sodium sulfate, and concentrated in vacuo. The residue was isolated and purified with silica gel column chromatography (hexane : acetone = 2 : 1), to give the titled compound (32.5 mg, 50.4%) as pale yellow amorphous.

¹H-NMR(CDCl₃) δ: 0.80 (6H, t, J= 7Hz), 0.90 (3H, t, J=7 Hz), 1.25-1.59 (8H, m), 2.15-2.22 (7H, m), 2.58 (2H, t, J=8 Hz), 3.32 (3H, br), 3.80 (2H, s), 5.02 (1H, s), 6.85 (2H, s), 7.31-7.45 (7H, m), 7.92 (1H, td, J=8 , 2 Hz), 8.67 (1H, dd, J=5, 1 Hz).

### Example 7

### Preparation of 2-[4-[{4-butyl-2-methyl-6-oxo-1-(pyridin-2-yl)-1,6-dihydrop yrimidin-5-yl}methyl]-2,6-dipropylphenoxy]-2-(2-chloropheny l)acetic acid

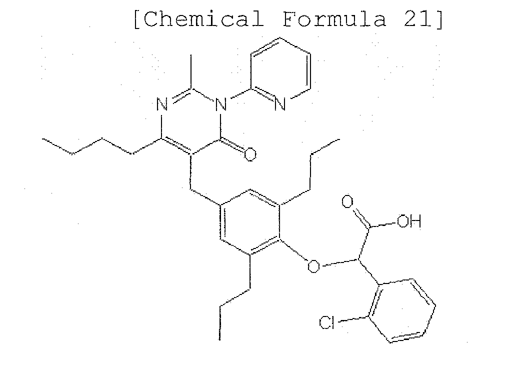

### Process 1:

With use of methyl α-bromo(2-chlorophenyl)acetate instead of methyl α-bromophenyl acetate, similar reactions and treatments were performed to those of Process 6 of Example 1, to give methyl 2-[4-[{4-butyl-2-methyl-6-oxo-1-(pyridin-2-yl)-1,6-dihydrop yrimidin-5-yl}methyl]-2,6-dipropylphenoxy]-2-(2-chloropheny l)acetate.

¹H-NMR(CDCl₃) δ: 0.77 (6H, t, J=7 Hz), 0.90 (3H, t, J=7 Hz), 1.30-1.63 (8H, m), 2.15 (3H, s), 2.25-2.40 (4H, m), 2.59 (2H, t, J=8 Hz), 3.72 (3H, s), 3.83 (2H, s), 5.55 (1H, s), 6.88 (2H, s), 7.27-7.46 (5H, m), 7.79-7.83(1H, m), 7.89-7.96 (1H, m), 8.65-8.68 (1H, m).

### Process 2:

With use of methyl 2-[4-[{4-butyl-2-methyl-6-oxo-1-(pyridin-2-yl)-1,6-dihydrop yrimidin-5-yl}methyl]-2,6-dipropylphenoxy]-2-(2-chloropheny l)acetate instead of methyl 2-[4-[{4-butyl-2-methyl-6-oxo-1-(pyridin-2-yl)-1,6-dihydrop yrimidin-5-yl}methyl]-2,6-dipropylphenoxy]-2-phenyl acetate, similar reactions and treatments were performed to those of Process 7 of Example 1, to give the titled compound (yield: 94.7%).

¹H-NMR(CDCl₃) δ: 0.73 (6H, t, J=7 Hz), 0.85 (3H, t, J=7 Hz), 1.26-1.52 (8H, m), 2.14 (3H, s), 2.27 (4H, br), 2.56 (2H, t, J=8 Hz), 3.79 (2H, s). 5.51 (1H, s), 6.82 (2H, s), 7.26-7.42 (5H, m), 7.66(1H, br), 7.89 (1H, td, J=8, 2 Hz), 8.65 (1H, d, J=3 Hz).

### Example 8

### Preparation of 2-[4-[{4-butyl-2-methyl-6-oxo-1-(pyridin-2-yl)-1,6-dihydrop yrimidin-5-yl}methyl]-2,6-dipropylphenoxy]-2-(3-chloropheny l)acetic acid

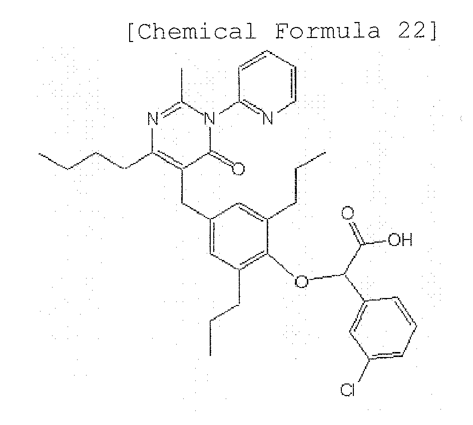

### Process 1:

With use of methyl α-bromo(3-chlorophenyl)acetate instead of methyl α-bromophenyl acetate, similar reactions and treatments were performed to those of Process 6 of Example 1, to give methyl 2-[4-[{4-butyl-2-methyl-6-oxo-1-(pyridin-2-yl)-1,6-dihydrop yrimidin-5-yl}methyl]-2,6-dipropylphenoxy]-2-(3-chloropheny l)acetate.

¹H-NMR(CDCl₃) δ: 0.80 (6H, t., J=7 Hz), 0.90 (3H, t, J=7 Hz), 1.31-1.62 (8H, m), 2.15 (3H, s), 2.24-2.37 (4H, m), 2.59 (2H, t, J=8 Hz), 3.74 (3H, s), 3.81 (2H, s), 5.04 (1H, s), 6.87 (2H, s), 7.28-7.44 (5H, m), 7.56 (1H s), 7.89-7.94 (1H, m), 8.66 (1H d, J=5 Hz).

### Process 2:

With use of methyl 2-[4-[{4-butyl-2-methyl-6-oxo-1-(pyridin-2-yl)-1,6-dihydrop yrimidin-5-yl}methyl]-2,6-dipropylphenoxy]-2-(3-chloropheny l)acetate instead of methyl 2-[4-[{4-butyl-2-methyl-6-oxo-1-(pyridin-2-yl)-1,6-dihydrop yrimidin-5-yl}methyl]-2,6-dipropylphenoxy]-2-phenyl acetate, similar reactions and treatments were performed to those of Process 7 of Example 1, to give the titled compound (yield: 73.7%).

¹H-NMR(CDCl₃) δ: 0.75 (6H, t, J=7 Hz), 0.86 (3H, t, J=7 Hz), 1.24-1.54 (8H, m), 2.14 (3H, s), 2.27 (4H, br), 2.58 (2H, t, J=8 Hz), 3.79 (2H, s), 5.00 (1H, s), 6.83 (2H, s), 7.26-7.33 (4H, m), 7.40-7.43 (1H, m), 7.52 (1H, br), 7.89 (1H td, J=8, 2 Hz), 8.64 (1H, d, J=3 Hz).

### Example 9

### Preparation of 2-[4-[{4-butyl-2-methyl-6-oxo-1-(pyridin-2-yl)-1,6-dihydrop yrimidin-5-yl}methyl]-2,6-dipropylphenoxy]-2-(4-chloropheny l)acetic acid

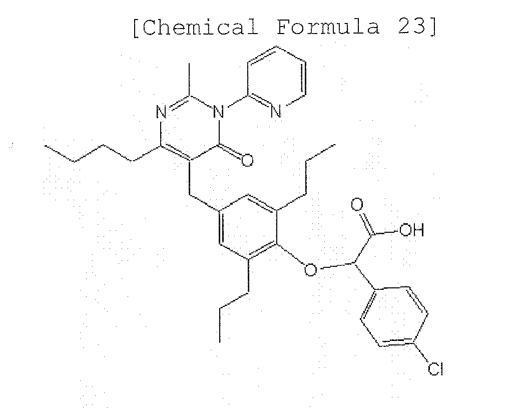

### Process 1:

With use of methyl α-bromo(4-chlorophenyl)acetate instead of methyl α-bromophenyl acetate, similar reactions and treatments were performed to those of Process 6 of Example 1, to give methyl 2-[4-[{4-butyl-2-methyl-6-oxo-1-(pyridin-2-yl)-1,6-dihydrop yrimidin-5-yl}methyl]-2,6-dipropylphenoxy]-2-(4-chloropheny l)acetate.

¹H-NMR(CDCl₃) δ: 0.79 (6H, t, J=7 Hz), 0.86-0.97 (3H, m), 1.33-1.64 (8H, m), 2.15 (3H, s), 2.24-2.36 (4H, m), 2.59 (2H, t, J=8 Hz), 3.72 (3H, s), 3.81 (2H, s), 5.04 (1H, s), 6.86 (2H, s), 7.34-7.46 (6H, m), 7.89-7.94 (1H, m), 8.66-8.67 (1H, m).

### Process 2:

With use of methyl 2-[4-[{4-butyl-2-methyl-6-oxo-1-(pyridin-2-yl)-1,6-dihydrop yrimidin-5-yl}methyl]-2,6-dipropylphenoxy]-2-(4-chloropheny l)acetate instead of methyl 2-[4-[{4-butyl-2-methyl-6-oxo-1-(pyridin-2-yl)-1,6-dihydrop yrimidin-5-yl}methyl]-2,6-dipropylphenoxy]-2-phenyl acetate, similar reactions and treatments were performed to those of Process 7 of Example 1, to give the titled compound (yield: 70.9%).

¹H-NMR(CDCl₃) δ: 0.73 (6H, br), 0.87 (3H, t, J=7 Hz), 1.29-1.51 (8H, m), 2.14 (3H, s), 2.25 (4H, br), 2.57 (2H, t, J=8 Hz), 3.79 (2H, s), 5.02 (1H, s), 6.83 (2H, s), 7.26-7.43 (6H, m), 7.90 (1H, td, J=8,2 Hz), 8.65 (1H, d, J=4 Hz).

### Example 10

### Preparation of 2-[4-[{4-butyl-2-methyl-6-oxo-1-(pyridin-2-yl)-1,6-dihydrop yrimidin-5-yl}methyl]-2,6-dipropylphenoxy]-2-(2-methoxyphen yl)acetic acid

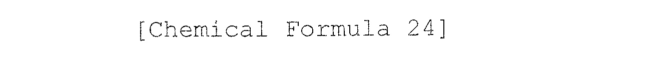

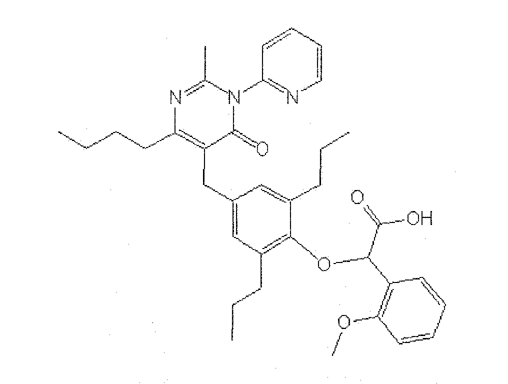

### Process 1:

With use of methyl α-bromo(2-methoxyphenyl)acetate instead of methyl α-bromophenyl acetate, similar reactions and treatments were performed to those of Process 6 of Example 1, to give methyl 2-[4-[{4-butyl-2-methyl-6-oxo-1-(pyridin-2-yl)-1,6-dihydrop yrimidin-5-yl}methyl]-2,6-dipropylphenoxy]-2-(2-methoxyphen yl)acetate.

¹H-NMR(CDCl₃) δ: 0.76 (6H, t, J=7 Hz), 0.89 (3H, t, J=7 Hz), 1.32-1.57 (8H, m), 2.15 (3H, s), 2.29 (4H, t, J=8 Hz), 2.59 (2H, t, J=8 Hz), 3.73 (3H, s), 3.74 (3H, s), 3.81 (2H, s), 5.46 (1H, s), 6.84-6.87 (3H, m), 7.29-7.36 (2H, m), 7.43 (1H, dd, J=7, 5 Hz), 7.51 (1H, dd, J=8, 1 Hz), 7.92 (1H, td, J=8, 2 Hz), 8.67 (1H, d, J=4 Hz).

### Process 2:

With use of methyl 2-[4-[{4-butyl-2-methyl-6-oxo-1-(pyridin-2-yl)-1,6-dihydrop yrimidin-5-yl}methyl]-2,6-dipropylphenoxy]-2-(2-methoxyphen yl)acetate instead of methyl 2-[4-[{4-butyl-2-methyl-6-oxo-1-(pyridin-2-yl)-1,6-dihydrop yrimidin-5-yl}methyl]-2,6-dipropylphenoxy]-2-phenyl acetate, similar reactions and treatments were performed to those of Process 7 of Example 1, to give the titled compound (yield: 89.5%).

¹H-NMR(CDCl₃) δ: 0.73 (6H, br), 0.87 (3H, t, J=7 Hz), 1.29-1.51 (8H, m), 2.14 (3H, s), 2.25 (4H, br), 2.57 (2H, t, J=8 Hz), 3.79 (2H, s), 5.02 (1H, s), 6.83 (2H, s), 7.26-7.43 (6H, m), 7.90 (1H, td, J=8, 2 Hz), 8.65 (1H, d, J=4 Hz).

### Example 11

### Preparation of 6-butyl-5-[4-{(2-chlorophenyl)(1H-tetrazol-5-yl)methoxy}-3, 5-dipropylbenzyl]-2-methyl-3-(pyridin-2-yl)pyrimidin-4(3H)-one

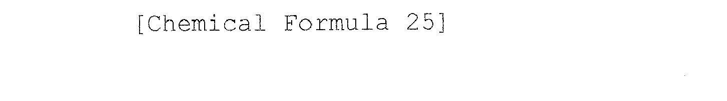

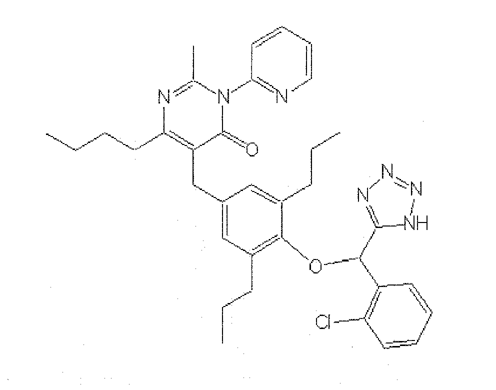

### Process 1:

With use of 2-[4-[{4-butyl-2-methyl-6-oxo-1-(pyridin-2-yl)-1,6-dihydrop yrimidin-5-yl}methyl]-2,6-dipropylphenoxy]-2-(2-chloropheny l)acetic acid instead of 2-[4-[{4-butyl-2-methyl-6-oxo-1-(pyridin-2-yl)-1,6-dihydrop yrimidin-5-yl}methyl]-2,6-dipropylphenoxy]-2-phenylacetic acid, similar reactions and treatments were performed to those of Process 1 of Example 4, to give 2-[4-[{4-butyl-2-methyl-6-oxo-1-(pyridin-2-yl)-1,6-dihydrop yrimidin-5-yl}methyl]-2,6-dipropylphenoxy]-2-(2-chloropheny l) acetoamide.

¹H-NMR (CDCl₃) δ: 0.80 (6H, t, J=7 Hz), 0.89 (3H, t, J=7 Hz), 1.26-1.39 (4H, m), 1.44-1.52 (4H, m), 2.15(3H, s), 2.17-2.27 (4H, m), 2.56 (2H, t, J=8 Hz), 3.81 (2H, s), 5.44 (1H, s), 6.39 (1H, br), 6.84 (2H, s), 7.14 (1H, br), 7.25-7.45 (6H, m), 7.90-7.94 (1H, m), 8.66-8.67 (1H, m).

### Process 2:

With use of 2-[4-[{4-butyl-2-methyl-6-oxo-1-(pyridin-2-yl)-1,6-dihydrop yrimidin-5-yl}methyl]-2,6-dipropylphenoxy]-2-(2-chloropheny l) acetoamide instead of 2-[4-[{4-butyl-2-methyl-6-oxo-1-(pyridin-2-yl)-1,6-dihydrop yrimidin-5-yl}methyl]-2,6-dipropylphenoxy]-2-phenyl acetoamide, similar reactions and treatments were performed to those of Process 2 of Example 4, to give 2-[4-[{4-butyl-2-methyl-6-oxo-1-(pyridin-2-yl)-1,6-dihydrop yrimidin-5-yl}methyl]-2,6-dipropylphenoxy]-2-(2-chloropheny l)acetonitrile.

¹H-NMR(CDCl₃) δ: 0.86 (6H, t, J=7 Hz), 0.92 (3H, t, J=7 Hz), 1.34-1.43 (2H, m), 1.54-1.61 (6H, m), 2.17 (3H, s), 2.43-2.50 (2H, m), 2.61-2.69 (4H, m), 3.86 (2H, s), 5.80 (1H, s), 6.97 (2H, s), 7.36-7.47 (5H, m), 7.88-7.94 (2H, m), 8.67-8.69 (1H, m).

### Process 3:

With use of 2-[4-[{4-butyl-2-methyl-6-oxo-1-(pyridin-2-yl)-1,6-dihydrop yrimidin-5-yl}methyl]-2,6-dipropylphenoxy]-2-(2-chloropheny l)acetonitrile instead of 2-[4-[{4-butyl-2-methyl-6-oxo-1-(pyridin-2-yl)-1,6-dihydrop yrimidin-5-yl}methyl]-2,6-dipropylphenoxy]-2-phenylacetonit rile, similar reactions and treatments were performed to those of Process 3 of Example 4, to give the titled compound (yield: 91.9%).

¹H-NMR(CDCl₃) δ: 0.63 (6H, br), 0.90 (3H, t, J=7 Hz), 1.32-1.42 (6H, m), 1.57-1.62 (2H, m), 2.03 (4H, br), 2.13 (3H, s), 2.63 (2H, t, J=8 Hz), 3.74 (2H, s), 6.35 (1H, s), 6.74 (2H, s), 7.24-7.36 (5H, m), 7.76 (1H, br), 8.12 (1H, d, J=7 Hz), 8.50 (1H, br).

### Example 12

### Preparation of 2-[4-[[1-{4-(benzyloxy)phenyl}-4-butyl-2-methyl-6-oxo-1,6-d ihydropyrimidin-5-yl]methyl]-2,6-dipropylphenoxy]-2-phenyla cetic acid

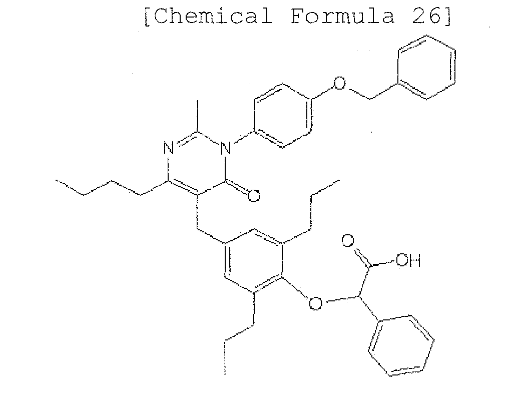

### Process 1:

With use of 4-(benzyloxy)aniline instead of 2-aminopyridine, similar reactions and treatments were performed to those of Processes 4 and 5 of Example 1, to give 3-{4-(benzyloxy)phenyl}-6-butyl-5-(4-hydroxy-3,5-dipropylbe nzyl)-2-methylpyrimidin-4(3H)-one.

¹H-NMR(CDCl₃) δ: 0.90-0.96 (9H, m), 1.35-1.44 (2H, m), 1.53-1.62 (6H, m), 2.14 (3H, s), 2.49 (4H, t, J=8 Hz), 2.62 (2H, t, J=8 Hz), 3.79 (2H, s), 4.76 (1H, s), 5.07 (2H,s), 6.88 (2H, s), 7.01-7.11 (4H, m), 7.31-7.44 (5H, m).

### Process 2:

With use of 3-{4-(benzyloxy)phenyl}-6-butyl-5-(4-hydroxy-3,5-dipropylbe nzyl)-2-methylpyrimidin-4(3H)-one instead of 6-butyl-5-(4-hydroxy-3,5-dipropylbenzyl)-2-methyl-3-(pyridi n-2-yl)pyrimidin-4(3H)-one, similar reactions and treatments were performed to those of Process 6 of Example 1, to give methyl 2-[4-[[1-{4-(benzyloxy)phenyl}-4-butyl-2-methyl-6-oxo-1,6-d ihydropyrimidin-5-yl]methyl]-2,6-dipropylphenoxy]-2-phenyl acetate.

¹H-NMR(CDCl₃) δ: 0.77 (6H, t, J=7 Hz), 0.91 (3H, t, J=7 Hz), 1.32-1.62 (8H, m), 2.16 (3H, s), 2.25-2.37 (4H, m), 2.59 (2H, t, J=8 Hz), 3.72 (3H, s), 3.80 (2H, s), 5.07 (1H, s), 5.09 (2H, s), 6.87 (2H, s), 7.10 (4H, s), 7.34-7.44 (8H, m), 7.49-7.52 (2H, m).

### Process 3:

With use of methyl 2-[4-[[1-{4-(benzyloxy)phenyl}-4-butyl-2--methyl-6-oxo-1,6-d ihydropyrimidin-5-yl]methyl]-2,6-dipropylphenoxy]-2-phenyl acetate instead of methyl 2-[4-[{4-butyl-2-methyl-6-oxo-1-(pyridin-2-yl)-1,6-dihydrop yrimidin-5-yl}methyl]-2,6-dipropylphenoxy]-2-phenyl acetate, similar reactions and treatments were performed to those of Process 7 of Example 1, to give the titled compound (yield: 36.6%).

¹H-NMR(CDCl₃) δ: 0.75 (6H, t, J=7 Hz), 0.88 (3H, t, J=7 Hz), 1.26-1.53 (8H, m), 2.15 (3H, s), 2.24 (4H, br), 2.56 (2H, t, J=8 Hz), 3.79 (2H, s), 5.09 (3H, s), 6.85 (2H, s), 7.09 (4H, s), 7.34-7.45 (10H, m).

### Example 13

### Preparation of 2-[4-[{4-butyl-1-(4-ethoxyphenyl)-2-methyl-6-oxo-1,6-dihydr opyrimidin-5-yl}methyl]-2,6-dipropylphenoxy]-2-phenylacetic acid

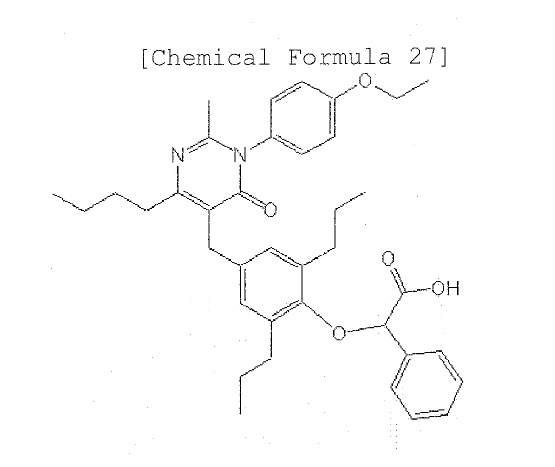

### Process 1:

To a solution of 4-aminophenol (218 mg, 2.0 mmol) in 1,2-dichloroethane (10 mL), trimethylaluminum (2 mol/L heptane solution, 1.0 mL, 2.0 mmol) was added at room temperature under argon atmosphere, and the solution was stirred at the same temperature for 1 hour. Methyl 3-acetamido-2-{4-(tert-butyldimethylsilyloxy)-3,5-dipropylb enzyl}-2-heptanate (251 mg, 2.0 mmol) obtained in Process 3 of Example 1 in 1,2-dichloroethane solution (25 mL) was dropped thereto at room temperature, and the reaction mixture was refluxed by heating for 15 hours. To the reaction mixture were added an aqueous solution of ammonium chloride and chloroform, and the reaction mixture was filtered through a pad of celite. The organic layer of the filtrate was isolated, and the aqueous layer was extracted with chloroform. The organic layers were combined, washed with water and brine, dried over anhydrous sodium sulfate, and concentrated in vacuo. The obtained residue was subjected to a silica gel column chromatography (hexane/acetone), to give 6-butyl-5-{4-(tert-butyldimethylsilyloxy)-3,5-dipropylbenzy 1}-3-(4-hydroxyphenyl)-2-methylpyrimidin-4(3H)-one (231.6 mg, 82.3%) as pale yellow amorphous.

¹H-NMR(CDCl₃) δ: 0.15 (6H, s), 0.87-0.92 (9H, m), 1.00 (9H, s), 1.31-1.40 (2H, m), 1.52-1.59 (6H, m), 2.16 (3H, s), 2.40 (4H, t, J=8 Hz), 2.52 (2H, t, J=8 Hz), 3.85 (2H, s), 6.65 (2H, d, J=9 Hz), 6.85-6.88 (4H, m).

### Process 2:

To a solution of 6-butyl-5-{4-(tert-butyldimethylsilyloxy)-3,5-dipropylbenzy l}-2-methyl-3-(4-hydroxylphenyl)-2-methylpyrimidin-4(3H)-on e (55.2 mg, 0.098 mmol) and potassium carbonate (27.1mg, 0.196 mmol) in acetonitrile (1.0 mL), ethyl iodide (22.9 mg, 0.147 mmol) was dropped, and the solution was stirred at 60°C for 12 hours. The reaction mixture was filtered, and the crystal was washed with ethyl acetate, and concentrated in vacuo. The obtained residue was subjected to a silica gel column chromatography (hexane/acetone), to give 6-butyl-5-{4-(tert-butyldimethylsilyloxy)-3,5-dipropylbenzy l}-2-methyl-3-(4-ethoxyphenyl)-2-methylpyrimidin-4(3H)-one (50.0 mg, 86.3%) as pale yellow amorphous.

### Process 3:

To a solution of the obtained 6-butyl-5-{4-(tert-butyldimethylsilyloxy)-3,5-dipropylbenzy l}-2-methyl-3-(4-ethoxyphenyl)-2-methylpyrimidin-4(3H)-one in tetrahydrofuran (1.0 mL), tetra-n-butyl ammonium fluoride (1 mol/L solution in tetrahydrofuran, 0.13 mL, 0.13 mmol) was dropped under ice cooling, and the solution was stirred at room temperature for 1 hour. The reaction mixture was added water, and extracted with ethyl acetate. The organic layers were combined, washed with water and brine, dried over anhydrous sodium sulfate, and concentrated in vacuo. The obtained residue was subjected to a silica gel column chromatography (hexane/acetone), to give 6-butyl-3-(4-ethoxyphenyl)-5-(4-hydroxy-3,5-dipropylbenzyl) -2-methylpyrimidin-4(3H)-one (31.6 mg, 78.0%) as pale yellow amorphous.

¹H-NMR(CDCl₃) δ: 0.91-0.97 (9H, m), 1.33-1.46 (5H, m), 1.54-1.64 (6H, m), 2.15 (3H, s), 2.50 (4H, t, J=8 Hz), 2.62 (2H, t, J=8 Hz), 3.79 (2H, s), 4.06 (2H, dd, J=14, 7 Hz), 4.58 (1H, s), 6.89 (2H, s), 6. 99 (2H, d, J=9 Hz), 7.10 (2H, d, J=9 Hz).

### Process 4:

### Preparation of methyl 2--[4-[{4-butyl-1-(4-ethoxyphenyl)-2-methyl-6-oxo-1,6-dihydr opyrimidin-5-yl}methyl]-2,6-dipropylphenoxy]-2-phenyl acetate

With use of 6-butyl -3-(4-ethoxyphenyl)-5-(4-hydroxy-3,5-dipropylbenzyl)-2-meth ylpyrimidin-4(3H)-one instead of 6-butyl-5-(4-hydroxy-3,5-dipropylbenzyl)-2-methyl-3-(pyridi n-2-yl)pyrimidin-4 (3H)-one, similar reactions and treatments were performed to those of Process 6 of Example 1, to give methyl 2-[4-[{4-butyl-1-(4-ethoxyphenyl)-2-methyl-6-oxo-1,6-dihydr opyrimidin-5-yl}methyl]-2,6-dipropylphenoxy]-2-phenyl acetate (quantitative).

¹H-NMR(CDCl₃) δ: 0.77 (6H, t, J=7Hz), 0.91 (3H, t, J=7 Hz), 1.32-1.57 (11H, m), 2.15 (3H, s), 2.28-2.35 (4H, m), 2.56 (2H, t, J=8 Hz), 3.72 (3H, s), 3.79 (2H, s), 4.07 (2H, dd, J=14, 7 Hz), 5.07 (1H, s), 6.87 (2H, s), 7.00 (2H, d, J=9 Hz), 7.09 (2H, d, J=9 Hz), 7.33-7.38 (3H, m), 7.49-7.54 (2H, m).

### Process 5:

With use of methyl 2-[4-[{4-butyl-1-(4-ethoxyphenyl)-2-methyl-6-oxo-1,6-dihydr opyrimidin-5-yl}methyl]-2,6-dipropylphenoxy]-2-phenyl acetate instead of methyl 2-[4-[{4-butyl-2-methyl-6-oxo-1-(pyridin-2-yl)-1,6-dihydrop yrimidin-5-yl}methyl]-2,6-dipropylphenoxy]-2-phenyl acetate, similar reactions and treatments were performed to those of Process 7 of Example 1, to give the titled compound (yield: 68.7%).

¹H-NMR(CDCl₃) δ: 0.74 (6H, t, J=7 Hz), 0.87 (3H, t, J=7 Hz), 1.21-1.55 (11H, m), 2.15 (3H, s), 2.25 (4H, br), 2.56 (2H, t, J=8 Hz), 3.78 (2H, s), 4.06 (2H, dd, J=14, 7 Hz), 5.05 (1H, s), 6.84 (2H, s), 6.98 (2H, d, J=9 Hz), 7.07 (2H, d, J=9 Hz), 7.32 (3H, br), 7.42 (2H, br).

### Example 14

### Preparation of 2-[4-[{4-butyl-1-(4-isopropoxyphenyl)-2-methyl-6-oxo-1,6-di hydropyrimidin-5-yl}methyl]-2,6-dipropylphenoxy]-2-phenylac etic acid

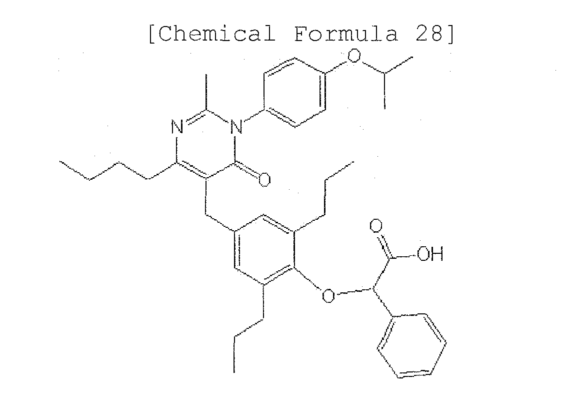

### Process 1:

With use of isopropyl iodide instead of ethyl iodide, similar reactions and treatments were performed similar to Process 2 of Example 13, to give 6-butyl-5-(4-hydroxy-3,5-dipropylbenzyl)-3-(4-isopropoxyphe nyl)-2-methylpyrimidin-4(3H)-one,

¹H-NMR(CDCl₃) δ: 0.91-0.97 (9H, m), 1.35-1.45 (8H, m), 1.54-1.64 (6H, m), 2.15 (3H, s), 2.50 (4H, t, J=8 Hz), 2.62 (2H, t, J=8 Hz), 3.79 (2H, s), 4.52-4.60 (2H, m), 6.89 (2H, s), 6.98 (2H, d, J=9 Hz), 7.07 (2H, d, J=9 Hz).

### Process 2:

With use of 6-butyl-5-(4-hydroxy-3,5-dipropylbenzyl)-3-(4-isopropoxyphe nyl)-2-methylpyrimidin-4(3H)-one instead of 6-butyl-5-(4-hydroxy-3,5-dipropylbenzyl)-2-methyl-3-(pyridi n-2-yl)pyrimidin-4(3H)-one, similar reactions and treatments were performed to those of Process 6 of Example 1, to give methyl 2-[4-[{4-butyl-1-(4-isopropoxyphenyl)-2-methyl-6-oxo-1,6-di hydropyrimidin-5-yl}methyl]-2,6-dipropylphenoxy]-2-phenyl acetate.

¹H-NMR(CDCl₃) δ: 0.77 (6H, t, J=7 Hz), 0.91 (3H, t, J=7 Hz), 1.32-1.59 (14H, m), 2.16 (3H, s), 2.26-2.35 (4H, m), 2.58 (2H, t, J=8 Hz), 3.72 (3H, s), 3.78 (2H, s), 4.54-4.62 (1H, m), 5.07 (1H, s), 6.87 (2H, s), 6.98 (2H, d, J=9 Hz), 7.07 (2H, d, J=9 Hz), 7.32-7.39 (3H, m), 7.49-7.55 (2H, m).

### Process 3:

With use of methyl 2-[4-[{4-butyl-1-(4-isopropoxyphenyl)-2-methyl-6-oxo-1,6-di hydropyrimidin-5-yl}-methyl]-2,6-dipropylphenoxy]-2-phenyl acetate instead of methyl 2-[4-[{4-butyl-2-methyl-6-oxo-1-(pyridin-2-yl)-1,6-dihydrop yrimidin-5-yl}methyl]-2,6-dipropylphenoxy]-2-phenyl acetate, similar reactions and treatments were performed to those of Process 7 of Example 1, to give the titled compound (yield: 72.4%).

¹H-NMR(CDCl₃) δ: 0.73 (6H, t, J=7 Hz), 0.85 (3H, t, J=7 Hz), 1.21-1.55 (14H, m), 2.15 (3H, s), 2.25 (4H, br), 2.56 (2H, t, J=8 Hz), 3.78 (2H, s), 4.53-4.59 (1H, m), 5.03 (1H, s), 6.83 (2H, s), 6.96 (2H, d, J=9 Hz), 7.05 (2H, d, J=9 Hz), 7.31 (3H, br), 7.43 (2H, br).

### Example 15

### Preparation of 2-[4-[{4-butyl-1-(5-methoxypyrimidin-2-yl)-2-methyl-6-oxo-1 ,6-dihydropyrimidin-5-yl}methyl]-2,6-dipropylphenoxy]-2-phe nylacetic acid

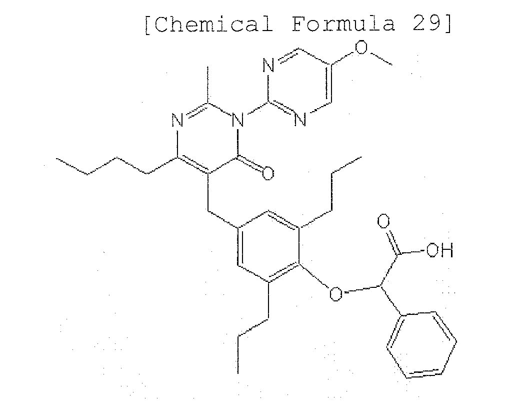

### Process 1:

With use of 2-amino-5-methoxypyrimidine instead of 2-aminopyridine, similar reactions and treatments were performed to those of Processes 4 and 5 of Example 1, to give 6-butyl-5-(4-hydroxy-3,5-dipropylbenzyl)-3-(5-methoxypyrimi din-2-yl)-2-methylpyrimidin-4-(3H)-one.

¹H-NMR(CDCl₃) δ: 0.88-0.96 (9H, m), 1.23 (3H, t, J=7 Hz), 1.31-1.40 (2H, m), 1.50-1.63 (6H, m), 2.13 (3H, s), 2.49 (4H, t, J=8 Hz), 2.60 (2H, t, J=8 Hz), 3.80 (2H, s), 3.98 (3H, s), 4.75 (1H, s), 6.85 (2H, s), 8.52 (2H, s).

### Process 2:

With use of 6-butyl-5-(4-hydroxy-3,5-dipropylbenzyl)-3-(5-methoxypyrimi din-2-yl)-2-methylpyrimidin-4 (3H)-one instead of 6-butyl-5-(4-hydroxy-3,5-dipropylbenzyl)-2-methyl-3-(pyridi n-2-yl)pyrimidin-4(3H)-one, similar reactions and treatments were performed to those of Process 6 of Example 1, to give methyl 2-[4-[{4-butyl-1-(5-methoxypyrimidin-2-yl)-2-methyl-6-oxo-1 ,6-dihydropyrimidin-5-yl}methyl]-2,6-dipropylphenoxy]-2-phe nyl acetate.

¹H-NMR(CDCl₃) δ: 0.77 (6H, t, J=7 Hz), 0.87-0.90 (3H, m), 1.29-1.63 (8H, m), 2.14 (3H, s), 2.24-2.36 (4H, m), 2.58 (2H, t, J=8 Hz), 3.72 (3H, s), 3.81 (2H, s), 3.99 (3H, s), 5.06 (1H, s), 6.85 (2H, s), 7.35-7.37 (3H, m), 7.49-7.51 (2H, m), 8.53 (2H, s).

### Process 3:

With use of methyl 2-[4-[{4-butyl-1-(5-methoxypyrimidin-2-yl)-2-methyl-6-oxo-1 ,6-dihydropyrimidin-5-yl}methyl]-2,6-dipropylphenoxy]-2-phe nyl acetate instead of methyl 2-[4-[{4-butyl-2-methyl-6-oxo-1-(pyridin-2-yl)-1,6-dihydrop yrimidin-5-yl}methyl]-2,6-dipropylphenoxy]-2-phenyl acetate, similar reactions and treatments were performed to those of Process 7 of Example 1, to give the titled compound (yield: 88.5%).

¹H-NMR(CDCl₃) δ: 0.71 (6H, t, J=7 Hz), 0.86 (3H, t, J=7 Hz), 1.27-1.53 (8H, m), 2.15 (3H, s), 2.23 (4H, br), 2.56 (2H, t, J=8 Hz), 3.78 (2H, s), 3.96 (3H, s), 5.01 (1H, s), 6.81 (2H, s), 7.29 (3H, br), 7.40 (2H, br), 8.51 (2H, s).

### Examples 16 and 17

### Preparation of (-)-2-[4-[{4-butyl-1-(5-methoxypyrimidin-2-yl)-2-methyl-6-o xo-1,6-dihydropyrimidin-5-yl}methyl]-2,6-dipropylphenoxy]-2 -phenylacetic acid and (+)-2-[4-[{4-butyl-1-(5-methoxypyrimidin-2-yl)-2-methyl-6-o xo-1,6-dihydropyrimidin-5-yl}methyl]-2,6-dipropylphenoxy]-2 -phenylacetic acid

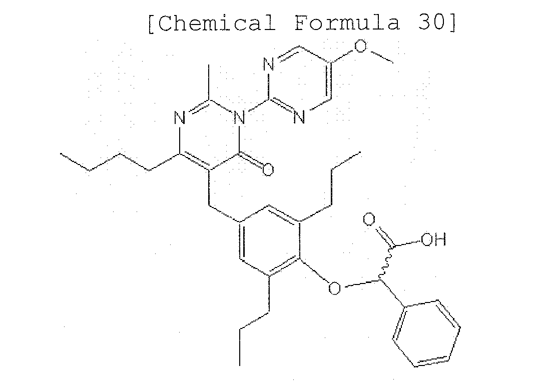

2-[4-[{4-butyl-1-(5-methoxypyrimidin-2-yl)-2-methyl-6 -oxo-1,6-dihydropyrimidin-5-yl}methyl]-2,6-dipropylphenoxy] -2-phenylacetic acid was eluted using HPLC column (Trade name: CHIRALPAK AD-H, manufactured by DAICEL CHEMICAL INDUSTRIES, LTD., 2 cmφ × 25 cm) for isolating optical isomers, and using hexane : 2-propanol : acetic acid (300 : 700 : 1) as a developing solvent, to give the titled compound.

(-)-2-[4-[{4-butyl-1-(5-methoxypyrimidin-2-yl)-2-meth yl-6-oxo-1,6-dihydropyrimidin-5-yl}methyl]-2,6-dipropylphen oxy]-2-phenylacetic acid

¹H-NMR(CDCl₃) δ: 0.71 (6H, t, J=7 Hz), 0.86 (3H, t, J=7 Hz), 1.27-1.53 (8H, m), 2.15 (3H, s), 2.23 (4H, br), 2.56 (2H, t, J=8 Hz), 3.78 (2H, s), 3.96 (3H, s), 5.01 (1H, s), 6.81 (2H, s), 7.29 (3H, br), 7.40 (2H, br), 8.51 (2H, s).
Retention time: 12.21 minutes, flow rate: 1 mL/min, optical purity: >99.9%ee
[α]²⁰_{D} = -10.74°

(+)-2-[4-[{4-butyl-1-(5-methoxypyrimidin-2-yl)-2-meth yl-6-oxo-1,6-dihydropyrimdin-5-yl}methyl]-2,6-dipropylphen oxy]-2-phenylacetic acid

¹H-NMR(CDCl₃) δ: 0.71 (6H, t, J=7 Hz), 0.86 (3H, t, J=7 Hz), 1.27-1.53 (8H, m), 2.15 (3H, s), 2.23 (4H, br), 2.56 (2H, t, J=8 Hz), 3.78 (2H, s), 3.96 (3H, s), 5.01 (1H, s), 6.81 (2H, s), 7.29 (3H, br), 7.40 (2H, br), 8.51 (2H, s).
Retention time: 17.94 minutes, flow rate: 1 mL/min, optical purity: 99.5%ee
[α]²⁰_{D} = +9.34°

### Example 18

### Preparation of 2-[4-[{4-butyl-1-(5-methoxypyrimidin-2-yl)-2-methyl-6-oxo-1 ,6-dihydropyrimidin-5-yl}methyl]-2,6-dipropylphenoxy]-2-(4-chlorophenyl)acetic acid

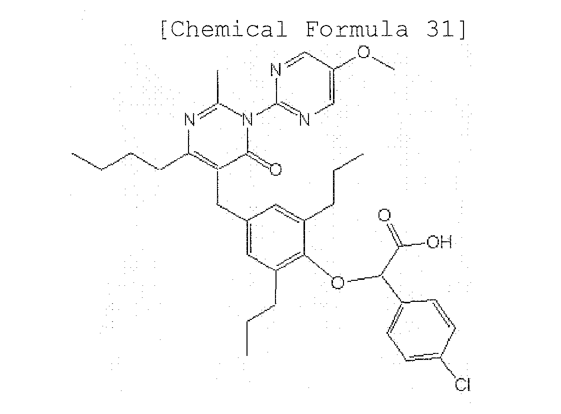

With use of 6-butyl-5-(4-hydroxy-3,5-dipropylbenzyl)-3-(5-methoxypyrimi din-2-yl)-2-methylpyrimidin-4(3H)-one instead of 6-butyl-5-(4-hydroxy-3,5-dipropylbenzyl)-2-methyl-3-(pyridi n-2-yl)pyrimidin-4(3H)-one, and with use of methyl α-bromo(4-chlorophenyl)acetate instead of methyl α-bromophenyl acetate, similar reactions and treatments were performed to those of Process 6 of Example 1, to give methyl 2-[4-[{4-butyl-1-(5-methoxypyrimidin-2-yl)-2-methyl-6-oxo-1 ,6-dihydropyrimidin-5-yl}methyl]-2,6-dipropylphenoxy]-2-(4-chlorophenyl)acetate.

¹H-NMR(CDCl₃) δ: 0.79 (6H, t, J=7 Hz), 0.89 (3H, t, J=7 Hz), 1.29-1.62 (8H, m), 2.14 (3H, s), 2.25-2.34 (4H, m), 2.58 (2H, t, J=8 Hz), 3.72 (3H,s), 3.81 (2H, s), 4.00 (3H, s), 5.04 (1H, s), 6.85 (2H, s), 7.35 (2H, d, J=9 Hz), 7.45 (2H, d, J=8 Hz), 8.54 (2H, s).

With use of methyl 2-[4-[{4-butyl-1-(5-methoxypyrimidin-2-yl)-2-methyl-6-oxo-1 ,6-dihydropyrimidin-5-yl}methyl]-2,6-dipropylphenoxy]-2-(4-chlorophenyl)acetate instead of methyl 2-[4-[{4-butyl-2-methyl-6-oxo-1-(pyridin-2-yl)-1,6-dihydrop yrimidin-5-yl}methyl]-2,6-dipropylphenoxy]-2-phenyl acetate, similar reactions and treatments were performed to those of Process 7 of Example 1, to give the titled compound (quantitative).

¹H-NMR(CDCl₃) δ: 0.74 (6H, br), 0.86 (3H, t, J=7 Hz), 1.28-1.52 (8H, m), 2.15 (3H, s), 2.25 (4H, br), 2.56 (2H, t, J=8 Hz), 3.79 (2H, s), 3.99 (3H, s), 5.01 (1H, s), 6.83 (2H, s), 7.31 (2H, br), 7.38 (2H, br), 8.52 (2H, s).

### Example 19

### Preparation of 2-[4-[[1-{5-(benzyloxy)pyrimidin-2-yl}-4-butyl-2-methyl-6-o xo-1,6-dihydropyrimidin-5-yl}methyl]-2,6-dipropylphenoxy]-2 -phenylacetic acid

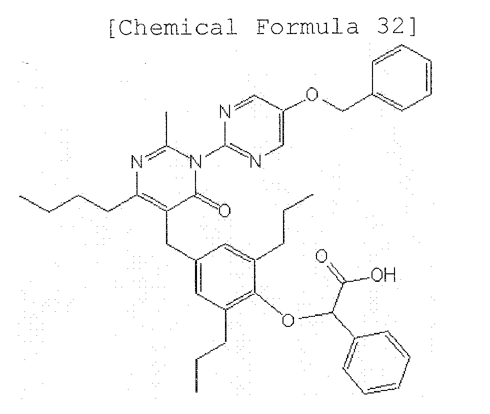

### Process 1:

With use of 2-amino-5-(benzyloxy)pyrimidine instead of 2-aminopyridine, similar reactions and treatments were performed to those of Processes 4 and 5 of Example 1, to give 3-{5-(benzyloxy)pyrimidin-2-yl}-6-butyl-5-(4-hydroxy-3,5-di propylbenzyl)-2-methylpyrimidin-4(3H)-one.

¹H-NMR(CDCl₃) δ: 0.88-0.95 (9H, m), 1.31-1.40 (2H, m), 1.51-1.62 (6H, m), 2.13 (3H, s), 2.47 (4H, t, J=8 Hz), 2.60 (2H, t, J=8 Hz), 3.80 (2H, s), 4.77 (1H, s), 5.19 (2H, s), 6.84 (2H, s), 7.38-7.43 (5H, m), 8.57 (2H, s).

### Process 2:

With use of 3-{(5-benzyloxy)pyrimidin-2-yl}-6-butyl-5-(4-hydroxy-3,5-di propylbenzyl)-2-methylpyrimidin-4(3H)-one instead of 6-butyl-5-(4-hydroxy-3,5-dipropylbenzyl)-2-methyl-3-(pyridi n-2-yl)pyrimidin-4 (3H)-one, similar reactions and treatments were performed to those of Process 6 of Example 1, to give methyl 2-[4-[[1-{5-(benzyloxy)pyrimidin-2-yl}-4-butyl-2-methyl-6-o xo-1,6-dihydropyrimidin-5-yl}methyl]-2,6-dipropylphenoxy]-2 -phenyl acetate.

¹H-NMR(CDCl₃) δ: 0.77 (6H, t, J=7 Hz), 0.88 (3H, t, J=7 Hz), 1.31-1.60 (8H, m), 2.13 (3H, s), 2.23-2.35 (4H, m), 2.57 (2H, t, J=8 Hz), 3.72 (3H, s), 3.81 (2H, s), 5.06 (1H, s), 5.22 (2H, s), 6.85 (2H, s), 7.35-7.50 (10H, m.), 8.59 (2H, s).

### Process 3:

With use of methyl 2-[4-[[1-{5-(benzyloxy)pyrimidin-2-yl}-4-butyl-2-methyl-6-o xo-1,6-dihydropyrimidin-5-yl]methyl]-2,6-dipropylphenoxy]-2 -phenyl acetate instead of methyl 2-[4-[{4-butyl-2-methyl-6-oxo-1-(pyridin-2-yl)-1,6-dihydrop yrimidin-5-yl}methyl]-2,6-dipropylphenoxy]-2-phenyl acetate, similar reactions and treatments were performed to those of Process 7 of Example 1, to give the titled compound (yield: 59.2%).

¹H-NMR(CDCl₃) δ: 0.72 (6H, t, J=7 Hz), 0.85 (3H, t, J=7 Hz), 1.26-1.52 (8H, m), 2.13 (3H, s), 2.23 (4H, br), 2.55 (2H, t, J=8 Hz), 3.78 (2H, s), 5.02 (1H, s), 5.20 (2H, s), 6.81 (2H, s), 7.30 (3H, br), 7.38-7.44 (7H, m), 8.57 (2H, s).

### Example 20

### Preparation of 2-[4-[{4-butyl-1-(5-ethoxypyrimidin-2-yl)-2-methyl-6-oxo-1, 6-dihydropyrimidin-5-yl}methyl]-2,6-dipropylphenoxy]-2-phen ylacetic acid

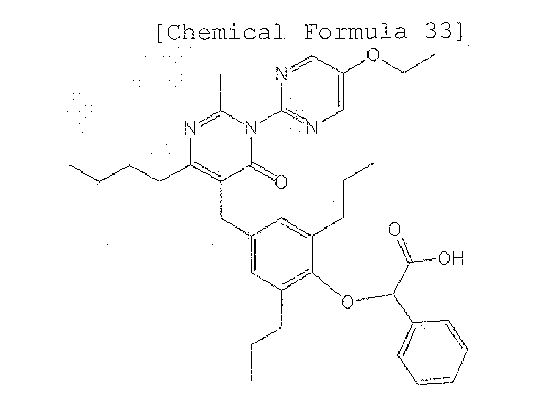

### Process 1:

To a solution of 2-amino-5- (benzyloxy)pyrimidine (1.2 g, 6.0 mmol) in 1,2-dichloroethane (30 mL), trimethylaluminum (2 mol/L heptane solution, 3.0 mL, 6.0 mmol) was added at room temperature under argon atmosphere, and the solution was stirred at the same temperature for 1 hour. A solution of methyl 3-acetamido-2-{4-(tert-butyldimethylsilyloxy)-3,5-dipropylb enzyl}-2-heptanate (1.1 g, 2.0 mmol) prepared in Process 3 of Example 1 in 1,2-dichloroethane (15 mL) was dropped thereto at room temperature, and the reaction mixture was refluxed by heating for 18 hours. To the reaction mixture were added an aqueous solution of ammonium chloride and chloroform, and the reaction mixture was filtered through a pad of celite. The organic layer of the filtrate was isolated, and the aqueous layer was extracted with chloroform. The organic layers were combined, washed with water and brine, dried over anhydrous sodium sulfate, and concentrated in vacuo. The obtained residue was subjected to a silica gel column chromatography (hexane/acetone), to give 6-butyl-5-{4-(tert-butyldimethylsilyloxy)-3,5-dipropylbenzy l}-3-(5-benzyloxypyrimidin-2-yl)-2-methylpyrimidin-4(3H)-on e (1.01 g, 77.1%) as pale yellow amorphous.

### Process 2:

To a solution of the obtained 6-butyl-5-{4-(tert-butyldimethylsilyloxy)-3,5-dipropylbenzy l}-3-(5-benzyloxypyrimidin-2-yl)-2-methylpyrimidin-4(3H)-on e (160.3 mg, 0.245 mmol) in methanol (20 mL) was added palladium carbon (10 wt%, wet, 80 mg), and the atmosphere was substituted with hydrogen atmosphere, and then the solution was stirred at room temperature for 1 hour. The reaction mixture was added ethyl acetate, and filtered through a pad of celite, and the filtrate was concentrated in vacuo. The obtained residue was subjected to a silica gel column chromatography (hexane/acetone), to give 6-butyl-5-{4-(tert-butyldimethylsilyloxy)-3,5-dipropylbenzy l}-3-(5-hydroxypyrimidin-2-yl)-2-methylpyrimidin-4(3H)-one (99.7 mg, 72.0%) as pale yellow amorphous.

¹H-NMR(CDCl₃) δ: 0.16 (6H, s), 0.88-0.92 (9H, m), 1.00 (9H,s), 1.32-1.37 (2H, m), 1.53-1.57 (9H, m), 2.16 (3H, s), 2.50 (4H, t, J=8 Hz), 2.63 (2H, t, J=8 Hz), 3.87 (2H, s), 6.84 (2H, s), 8.17 (2H, s).

### Process 3:

With use of 6-butyl-5-{4-(tert-butyldimethylsilyloxy)-3,5-dipropylbenzy l}-3-(5-hydroxypyrimidin-2-yl)-2-methylpyrimidin-4(3H)-one instead of 6-butyl-5-{4-(tert-butyldimethylsilyloxy)-3,5-dipropylbenzy l}-2-methyl-3-(4-hydroxylphenyl)-2-methylpyrimidin-4(3H)-on e, similar reactions and treatments were performed to those of Process 2 of Example 13, to give 6-butyl-3-(5-ethoxypyrimidin-2-yl)-5-(4-hydroxy-3,5-dipropy lbenzyl)-2-methylpyrimidin-4(3H)-one (yield: 99.3%).

¹H-NMR(CDCl₃) δ: 0.88-0.97 (9H, m), 1.33-1.40 (2H, m), 1.49-1.63 (9H, m), 2.13 (3H, s), 2.45 (4H, t, J=8 Hz), 2.59 (2H, t, J=8 Hz), 3.80 (2H, s), 3.20 (2H, dd, J=14, 7 Hz), 4.60 (1H, s), 6.85 (2H, s), 8.50 (2H, s).

### Process 4:

With use of 6-butyl-3-(5-ethoxypyrimidin-2-yl)-5-(4-hydroxy-3,5-dipropy lbenzyl)-2-methyl-pyrimidin-4(3H)-one instead of 6-butyl-5-(4-hydroxy-3,5-dipropylbenzyl)-2-methyl-3-(pyridi n-2-yl)pyrimidin-4(3H)-one, similar reactions and treatments were performed to those of Process 6 of Example 1, to give methyl 2-[4-[{4-butyl-1-(5-ethoxypyrimidin-2-yl)-2-methyl-6-oxo-1, 6-dihydropyrimidin-5-yl}methyl]-2,6-dipropylphenoxy]-2-phen yl acetate (yield: 90.8%).

¹H-NMR(CDCl₃) δ: 0.77 (6H, t, J=7 Hz), 0.87-0.90 (3H, m), 1.26-1.63 (11H, m), 2.14 (3H, s), 2.24-2.36 (4H, m), 2.57 (2H, t, J=8 Hz), 3.72 (3H, s), 3.81 (2H, s), 4.21 (2H, dd, J=14, 7 Hz), 5.06 (1H, s), 6.85 (2H, s), 7.35-7.43 (3H, m), 7.49-7.51 (2H, m), 8.51 (2H, s).

### Process 5:

With use of methyl 2-[4-[{4-butyl-1-(5-ethoxypyrimidin-2-yl)-2-methyl-6-oxo-1, 6-dihydropyrimidin-5-yl}methyl]-2,6-dipropylphenoxy]-2-phen yl acetate instead of methyl 2-[4-[{4-butyl-2-methyl-6-oxo-1-(pyridin-2-yl)-1,6-dihydrop yrimidin-5-yl}methyl]-2,6-dipropylphenoxy]-2-phenyl acetate, similar reactions and treatments were performed to those of Process 7 of Example 1, to give the titled compound (yield: 86.3%).

¹H-NMR(CDCl₃) δ: 0.73 (6H, t, J=7 Hz), 0.86 (3H, t, J=7 Hz), 1.26-1.33 (4H, m), 1.42-1.53 (7H, m), 2.13 (3H, s), 2.24 (4H, br), 2.56 (2H, t, J=8 Hz), 3.79 (2H, s), 3.96 (2H, dd, J=14, 7 Hz), 5.04 (1H, s), 6.82 (2H, s), 7.32 (3H, br), 7.42 (2H, br), 8.50 (2H, s)

### Examples 21 and 22

### Preparation of (-)-2-[4-[{4-butyl-1-(5-ethoxypyrimidin-2-yl)-2-methyl-6-ox o-1,6-dihydropyrimi-din-5-yl}methyl]-2,6-dipropylphenoxy]-2-phenylacetic acid and (+)-2-[4-[{4-butyl-1-(5-ethoxypyrimidin-2-yl)-2-methyl-6-ox o-1,6-dihydropyrimidin-5-yl}methyl]-2,6-dipropylphenoxy]-2-phenylacetic acid

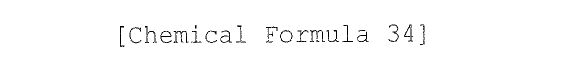

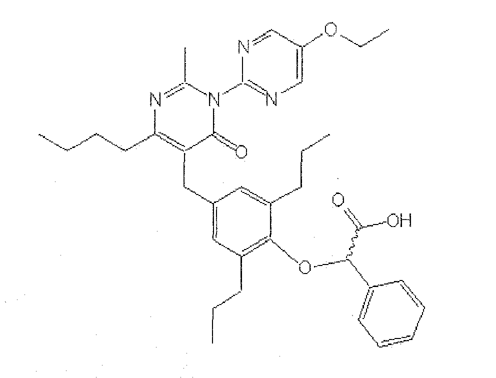

With use of 2-[4-[{4-butyl-1-(5-ethoxypyrimidin-2-yl)-2-methyl-6-oxa-1, 6-dihydropyrimidin-5-yl}methyl]-2,6-dipropylphenoxy]-2-phen ylacetic acid instead of 2-[4-[{4-butyl-1-(5-methoxypyrimidin-2-yl)-2-methyl-6-oxo-1 ,6-dihydropyrimidin-5-yl}methyl]-2,6-dipropylphenoxy]-2-phe nylacetic acid, similar reactions and treatments were performed to those of Examples 16 and 17, to give the titled compound.

(-)-2-[4-[{4-butyl-1-(5-ethoxypyrimidin-2-yl)-2-methy 1-6-oxo-1,6-dihydropyrimidin-5-yl}methyl]-2,6-dipropylpheno xy]-2-phenylacetic acid
¹H-NMR(CDCl₃) δ: 0.73 (6H, t, J=7 H₂), 0.86 (3H, t, J=7 Hz), 1.26-1.33 (4H, m), 1.42-1.53 (7H, m), 2.13 (3H, s), 2.24 (4H, br), 2.56 (2H, t, J=8 Hz), 3.79 (2H, s), 3.96 (2H, dd, J=14, 7 Hz), 5.04 (1H, s), 6.82 (2H, s), 7.32 (3H, br), 7.42 (2H, br), 8.50 (2H, s)
Retention time: 11.73 minutes, flow rate: 1 mL/min, optical purity: >99.9%ee
[α]²⁰_{D} = -11.88°

(+)-2-[4-[{4-butyl-1-(5-ethoxypyrimidin-2-yl)-2-methy 1-6-oxo-1,6-dihydropyrimidin-5-yl}methyl]-2,6-dipropylpheno xy]-2-phenylacetic acid

¹H-NMR(CDCl₃) δ: 0.73 (6H, t, J=7 Hz), 0.86 (3H, t, J=7 Hz), 1.26-1.33 (4H, m), 1.42-1.53 (7H, m), 2.13 (3H, s), 2.24 (4H, br), 2.56 (2H, t, J=8 Hz), 3.79 (2H, s), 3. 96 (2H, dd, J=14, 7 Hz), 5.04 (1H, s), 6.82 (2H, s), 7.32 (3H, br), 7.42 (2H, br), 8.50 (2H, s)
Retention time: 15.79 minutes, flow rate: 1 mL/min, optical purity: >99.9%ee
[α]²⁰_{D} = +10.87°

### Example 23

### Preparation of 2-[4-[[1-{5-(4-amino-4-oxobutoxy)pyrimidin-2-yl}-4-butyl-2-methyl-6-oxo-1,6-dihydropyrimidin-5-yl]methyl]-2,6-dipropyl phenoxy]-2-phenylacetic acid

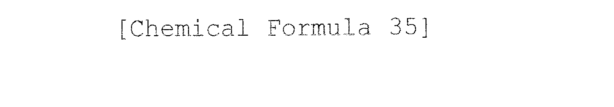

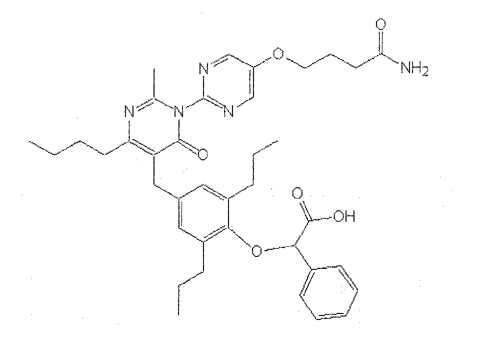

### Process 1:

To a solution of 6-butyl-5-{4-(tert-butyldimethylsilyloxy)-3,5-dipropylbenzy l}-3-(5-hydroxypyrimidin-2-yl)-2-methylpyrimidin-4(3H)-one prepared in Process 2 of Example 20 (50.3 mg, 0.089 mmol) and potassium carbonate (16.0 mg, 0.116 mmol) in acetonitrile (1.0 mL), ethyl 4-bromobutyrate (19.5 mg, 0.10 mmol) was dropped, and the solution was stirred at 80°C for 15 hours. The reaction mixture was filtered, and the crystal was washed with ethyl acetate, and concentrated in vacuo. The obtained residue was subjected to silica gel column chromatography (chloroform/methanol), to give ethyl 3-[2-[4-butyl-5-{4-(tert-butyldimethylsilyloxy)-3,5-dipropy lbenzyl}-2-methyl-6-oxopyrimidin-1(6H)-yl]pyrimidin-5-yloxy ]butanate (67.5 mg, 110.1%) as pale yellow amorphous.

### Process 2:

To a solution of the obtained ethyl 3-[2-[4-butyl-5-{4-(tert-butyldimethylsilyloxy)-3,5-dipropy lbenzyl}-2-methyl-6-oxopyrimidin-1(6H)-yl]pyrimidin-5-yloxy ]propanate in methanol (4 mL)-water (2mL), lithium hydroxide monohydrate (41.7 mg, 0.99 mmol) was added, and the solution was stirred at room temperature for 12 hours. To the reaction mixture, water and 1 mol/L hydrochloric acid were added, and the reaction mixture was extracted with ethyl acetate. The organic layers were combined, washed with water and brine, dried over anhydrous sodium sulfate, and concentrated in vacuo. The residue was subjected to a silica gel column chromatography (hexane/acetone), to give 3-[2-[4-butyl-5-{4-(tert-butyldimethylsilyloxy)-3,5-dipropy lbenzyl}-2-methyl-6-oxopyrimidin-1(6H)-yl]pyrimidin-5-yloxy ]butane acid (38.0 mg, 65.7%) as pale yellow amorphous.

¹H-NMR(CDCl₃) δ: 0.15 (6H, s), 0.96-0.87 (9H, m), 1.00 (6H, s), 1.29-1.40 (2H, m), 1.44-1.62 (6H, m), 2.14-2.18 (5H, m), 2.45-2.50 (4H, m), 2.57-2.62 (4H, m), 3.80 (2H, s), 4.20 (2H, br), 6.83 (2H, s), 8.52 (2H, s).

### Process 3:

To a solution of 3-[2-[4-butyl-5-{4-(tert-butyldimethylsilyloxy)-3,5-dipropy lbenzyl}-2-methyl-6-oxopyrimidin-1(6H)-yl]pyrimidin-5-yloxy ]butane acid (38.0 mg, 0.058 mmol) in dichloromethane (1.0 mL) were added 1-hydroxybenzotriazole hydrate (12.3mg, 0.088 mmol) and 1-ethyl-3-(3-dimethylaminopropyl) carbodiimide hydrochloride (16.8 mg, 0.088 mmol), and the solution was stirred at room temperature for 2 hours. To the reaction mixture was added an aqueous solution of ammonia (1.0 mL), and the solution was stirred at room temperature for 15 hours. The reaction mixture was added water, and extracted with chloroform. The organic layers were combined, washed with an aqueous solution of saturated sodium hydrogencarbonate, water and brine, dried over anhydrous sodium sulfate, and concentrated in vacuo. The residue was subjected to silica gel column chromatography (chloroform/methanol), to give 3-[2-[4-butyl-5-{4-(tert-butyldimethylsilyloxy)-3,5-dipropy lbenzyl}-2-methyl-6-oxopyrimidin-1(6H)-yl]pyrimidin-5-yloxy ]butanamide (18.3 mg, 28.3%) as pale yellow amorphous.

### Process 4:

To a solution of the obtained 3-[2-[4-butyl-5-{4-(tert-butyldimethylsilyloxy)-3,5-dipropy lbenzyl}-2-methyl-6-oxopyrimidin-1(6H)-yl]pyrimidin-5-yloxy ]butanamide in tetrahydrofuran (1.0 mL), tetra-n-butyl ammonium fluoride (1 mol/L solution in tetrahydrofuran, 42.0 µL, 0.042 mmol) was dropped under ice cooling, and the solution was stirred at room temperature for 1 hour. The reaction mixture was added water, and extracted with ethyl acetate. The organic layers were combined, washed with water and brine, dried over anhydrous sodium sulfate, and concentrated in vacuo. The obtained residue was subjected to silica gel column chromatography (chloroform/methanol), to give 4-[2-{4-butyl-5-hydroxy-3,5-dipropylbenzyl}-2-methyl-6-oxop yrimidin-1(6H)-yl]pyrimidin-5-yloxy)butanamide (15.4 mg, quantitative) as pale yellow amorphous.

¹H-NMR(CDCl₃) δ: 0.88-0.97 (9H, m), 1.33-1.40 (2H, m), 1.51-1.63 (6H, m), 2.14 (3H, s), 2.16-2.22 (2H, m), 2.43 (2H, t, J=7 Hz), 2.49 (4H, t, J=8 Hz), 2.60 (2H, t, J=8 Hz), 3.80 (2H, s), 4.20 (2H, t, J=6 Hz), 4.64 (1H, s), 5.46 (1H,s), 5.61 (1H, s), 6.84 (2H, s), 8.51 (2H, s).

### Process 5:

With use of 4-[2-{4-butyl-5-(4-hydroxy-3,5-dipropylbenzyl)-2-methyl-6-o xopyrimidin-1(6H)-yl}pyrimidin-5-yloxy]butanamide instead of 6-butyl-5-(4-hydroxy-3,5-dipropylbenzyl)-2-methyl-3-(pyridi n-2-yl)pyrimidin-4(3H)-one, similar reactions and treatments were performed to those of Process 6 of Example 1, to give methyl 2-[4-[[1-{5-(4-amino-4-oxobutoxy)pyrimidin-2-yl}-4-butyl-2-methyl-6-oxo-1,6-dihydropyrimidin-5-yl]methyl]-2,6-dipropyl phenoxy]-2-phenyl acetate (yield: 41.2%).

¹H-NMR(CDCl₃) δ: 0.77 (6H, t, J=7 Hz), 0.88 (3H, t, J=7 Hz), 1.29-1.56 (8H, m), 2.14 (3H, s), 2.21 (2H, t, J=6 Hz), 2.28-2.34 (4H, m), 2.45 (2H, t, J=7 Hz), 2.57 (2H, t, J=8 Hz), 3.73 (3H, s) 3.80 (2H, s), 4.23 (2H, t, J=6 Hz), 5.05 (1H, s), 5.41 (1H, s), 5.52 (1H, s), 6.84 (2H, s), 7.35-7.38 (3H, m), 7.48-7.51 (2H, m), 8.53 (2H, s).

### Process 6:

With use of methyl 2-[4-[[1-{5-(4-amino-4-oxobutoxy)pyrimidin-2-yl}-4-butyl-2-methyl-6-oxo-1,6-dihydropyrimidin-5-yl]methyl]-2,6-dipropyl phenoxy]-2-phenyl acetate instead of methyl 2-[4-[{4-butyl-2-methyl-6-oxo-1-(pyridin-2-yl)-1,6-dihydrop yrimidin-5-yl}methyl]-2,6-dipropylphenoxy]-2-phenyl acetate, similar reactions and treatments were performed to those of Process 7 of Example 1, to give the titled compound (yield: 59.7%).

¹H-NMR(CDCl₃) δ: 0.76 (6H, br), 0.88 (3H, t, J=7 Hz), 1.25-1.55 (8H, m), 2.04 (2H, br), 2.13 (3H, s), 2.29 (4H, br), 2.59 (2H, br), 3.67 (1H, s), 3.78 (2H, s), 4.09 (2H, br), 5.10 (1H, s), 5.88 (1H, s), 6.81 (2H, s), 7.31 (3H, br), 7.45 (2H, br), 8.45 (2H, s).

### Example 24

### Preparation of 2-[4-[[4-butyl-2-methyl-1-[5-{2-(methylthio)ethoxy}pyrimidi n-2-yl]-6-oxo-1,6-dihydropyrimidin-5-yl]methyl]-2,6-dipropy lphenoxy]-2-phenylacetic acid

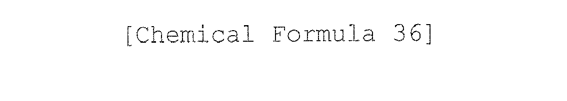

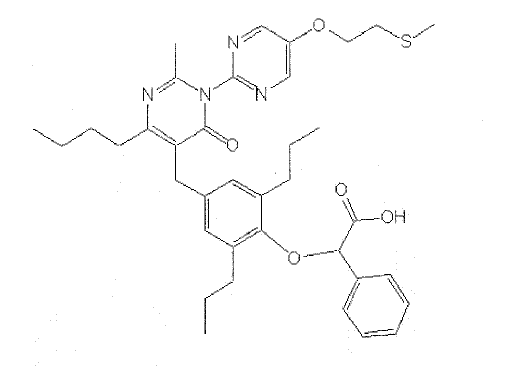

### Process 1:

With use of 2-amino-5-{2-(methylthio)ethoxy}pyrimidine instead of 2-aminopyridine, similar reactions and treatments were performed to those of Processes 4 and 5 of Example 1, to give 6-butyl-5-(4-hydroxy-3,5-dipropylbenzyl)-2-methyl-3-[5-{2-( methylthio)ethoxy}pyrimidin-2-yl]pyrimidin-4(3H)-one.

¹H-NMR(CDCl₃) δ: 0.88-0.97 (9H, m), 1.31-1.40 (2H, m), 1.50-1.63 (6H, m), 2.14 (3H, s), 2.24 (3H, s), 2.49 (4H, t, J=8 Hz), 2.60 (2H, t, J=8 Hz), 2.95 (2H, t, J=7 Hz), 3.78 (2H,s), 4.31 (2H, t, J=7 Hz), 4.59 (1H, s), 6.85 (2H, s), 8.54 (2H, s).

### Process 2:

With use of 6-butyl-5-(4-hydroxy-3,5-dipropylbenzyl)-2-methyl-3-[5-{2-( methylthio)ethoxy}pyrimidin-2-yl]pyrimidin-4(3H)-one instead of 6-butyl-5-(4-hydroxy-3,5-dipropylbenzyl)-2-methyl-3-(pyridi n-2-yl)pyrimidin-4(3H)-one, similar reactions and treatments were performed to those of Process 6 of Example 1, to give methyl 2-[4-[[4-butyl-2-methyl-1-[5-{2-(methylthio)ethoxy}pyrimidi n-2-yl]-6-oxo-1,6-dihydropyrimidin-5-yl]methyl]-2,6-dipropy lphenoxy]-2-phenyl acetate.

¹H-NMR(CDCl₃) δ: 0.77 (6H, t, J=7 Hz), 0.89 (3H, t, J=7 Hz), 1.29-1.56 (8H, m), 2.14 (3H, s), 2.23 (3H, s), 2.27-2.32 (4H, m), 2.57 (2H, t, J=8 Hz), 2.95 (2H, t, J=7 Hz), 3.73 (3H, s), 3.80 (2H,s), 4.33 (2H, t, J=7 Hz), 5.06 (1H, s), 6.85 (2H, s), 7.35-7.39 (3H, m), 7.49-7.51 (2H, m), 8.55 (2H, s).

With use of methyl 2-[4-[[4-butyl-2-methyl-1-[5-{2-(methylthio)ethoxy}pyrimidi n-2-yl]-6-oxo-1,6-dihydropyrimidin-5-yl]methyl]-2,6-dipropy lphenoxy]-2-phenyl acetate instead of methyl 2-[4-[{4-butyl-2-methyl-6-oxo-1-(pyridin-2-yl)-1,6-dihydrop yrimidin-5-yl}methyl]-2,6-dipropylphenoxy]-2-phenyl acetate, similar reactions and treatments were performed to those of Process 7 of Example 1, to give the titled compound (quantitative).

¹H-NMR(CDCl₃) δ: 0.74 (6H, t, J=7 Hz), 0.86 (3H, t, J=7 Hz), 1.25-1.52. (8H, m), 2.13 (3H, s), 2.23-2.27 (7H, m), 2.56 (2H, t, J=8 Hz), 2.95 (2H, t, J=7 Hz), 3.79 (2H,s), 4.31 (2H, t, J=6 Hz), 5.05 (1H, s), 6.83 (2H, s), 7.33 (3H, br), 7.44 (2H, br), 8.53 (2H, s).

### Example 25

### Preparation of 2-[4-[[4-butyl-2-methyl-1-[5-{2-(methylsulfinyl)ethoxy}pyri midin-2-yl]-6-oxo-1,6-dihydropyrimidin-5-yl]methyl]-2,6-dip ropylphenoxy]-2-phenylacetic acid

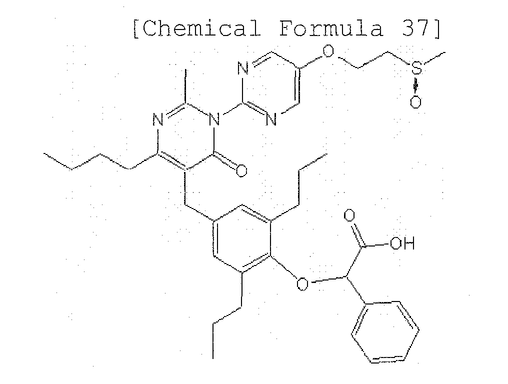

To a solution of 2-[4-[[4-butyl-2-methyl-1-[5-{2-(methylthio)ethoxylpyrimidi n-2-yl]-6-oxo-1,6-dihydropyrimidin-5-yl]methyl]-2,6-dipropy lphenoxy]-2-phenylacetic acid prepared in Example 24 (17.0 mg, 0.025 mmol) in acetic acid (1.0 mL)-methanol (0.2 mL), sodium peroxoborate tetrahydrate (4.7 mg, 0.030 mmol was added, and the solution was stirred at room temperature for 6 hours. The reaction mixture was added an aqueous solution of sodium thiosulfate and an aqueous solution of 2 mol/L sodium hydroxide, and extracted with ethyl acetate. The organic layers were combined, washed with water and brine, dried over anhydrous sodium sulfate, and concentrated in vacuo. The residue was isolated and purified with silica gel column chromatography (chloroform : methanol = 10 : 1), to give the titled compound (10.3 mg, 59.2%) as pale yellow amorphous.

¹H-NMR(CDCl₃) δ: 0.76 (6H, br), 0.88 (3H, t, J=7 Hz), 1.30-1.53 (8H, m), 2.14 (3H, s), 2.28 (4H, br), 2.59 (2H, t, J=8 Hz), 2.70 (3H, s), 3.16 (2H, d, J=27 Hz), 3.79 (2H,s), 4.54 (2H, br), 5.08 (1H, s), 6.83 (2H, s), 7.33 (3H, br), 7.45 (2H, br), 8.55 (2H, s).

### Example 26

### Preparation of 2-[4-[[4-butyl-2-methyl-1-[5-{2-(methylsulfonyl)ethoxy}pyri midin-2-yl]-6-oxo-1,6-dihydropyrimidin-5-yl]methyl]-2,6-dip ropylphenoxy]-2-phenylacetic acid

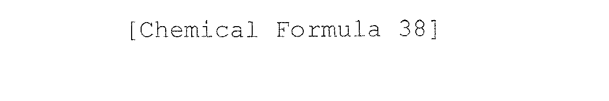

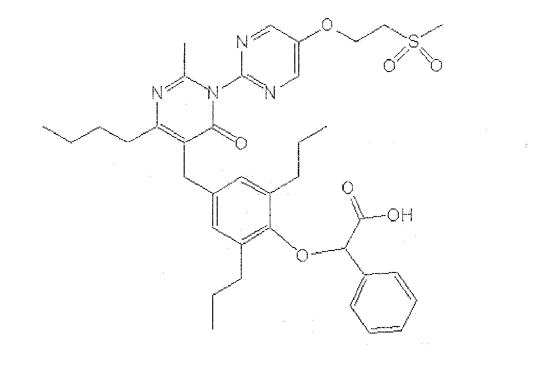

To a solution of 2-[4-[[4-butyl-2-methyl-1-[5-{2-(methylthio)ethoxy}pyrimidi n-2-yl]-6-oxo-1,6-dihydropyrimidin-5-yl]methyl]-2,6-dipropy lphenoxy]-2-phenylacetic acid (17.1 mg, 0.025 mmol) prepared in Example 24 in methanol (1.0 mL) were added 30% hydrogen peroxide solution (14.3 mg, 0.127 mmol) in methanol (1.0 mL) and a solution of tantalum pentachloride (1.1 mg, 0.003 mmol) in methanol (1.0 mL), and the solution was stirred at room temperature for 5 hours. The reaction mixture was concentrated in vacuo, and the residue was isolated and purified with silica gel column chromatography (chloroform : methanol =10 : 1), to give the titled compound (15.3 mg, 88.5%) as pale yellow amorphous.

¹H-NMR(CDCl₃) δ: 0.74 (6H, t, J=7 Hz), 0.87 (3H, t, J=7 Hz), 1.28-1.52 (8H, m), 2.14 (3H, s), 2.25 (4H, br), 2.57 (2H, t, J=8 Hz), 3.06 (3H, s), 3.51 (2H, br), 3.79 (2H,s), 4.59 (2H, br), 5.07 (1H, s), 6.82 (2H, s), 7.35 (3H, br), 7.42 (2H, br), 8.56 (2H, s).

### Example 27

### Preparation of 2-[4-[{4-butyl-1-(5-hydroxypyrimidin-2-yl)-2-methyl-6-oxo-1 ,6-dihydropyrimidin-5-yl}methyl]-2,6-dipropylphenoxy]-2-phe nylacetic acid

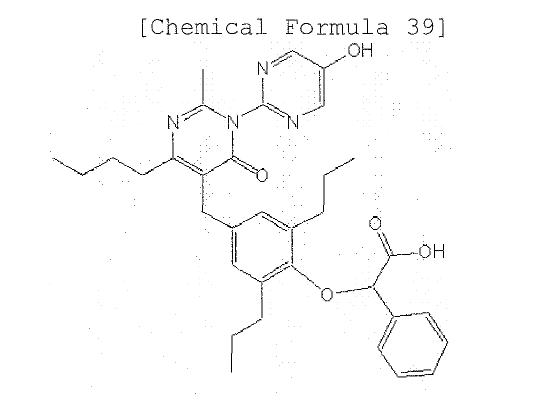

To a solution of 2-[4-[[4-butyl-2-methyl-1-[5-{2-(methylsulfonyl)ethoxy}pyri midin-2-yl]-6-oxo-1,6-dihydropyrimidin-5-yl]methyl]-2,6-dip ropylphenoxy] -2-phenylacetic acid prepared in Example 26 (17.1 mg, 0.025 mmol) in methanol (1.0 mL)-water (0.5 mL) was added lithium hydroxide monohydrate (7.3 mg, 0.173 mmol), and the solution was stirred at room temperature for 12 hours. The reaction mixture was added water and 1 mol/L hydrochloric acid, and extracted with ethyl acetate. The organic layers were combined, washed with water and brine, dried over anhydrous sodium sulfate, and concentrated in vacuo. The residue was isolated and purified with silica gel column chromatography (chloroform : methanol = 10 : 1), to give the titled compound (7.2 mg, 72.4%) as pale yellow amorphous.

¹H-NMR(CDCl₃) δ: 0.78 (6H, t, J=7 Hz), 0.89 (3H, t, J=7 Hz), 1.28-1.53 (8H, m), 2.15 (3H, s), 2.30 (4H, t, J=8 Hz), 2.59 (2H, t, J=8 Hz), 3.83 (2H, s), 5.01 (1H, s), 6.84 (2H, s), 7.36-7.38 (3H, m), 7.45-7.48 (2H, m), 8.45 (2H, s).

### Example 28

### Preparation of 2-[4-{(1-benzyl-4-butyl-2-methyl-6-oxo-1,6-dihydropyrimidin -5-yl)methyl}-2,6-dipropylphenoxy]-2-phenylacetic acid

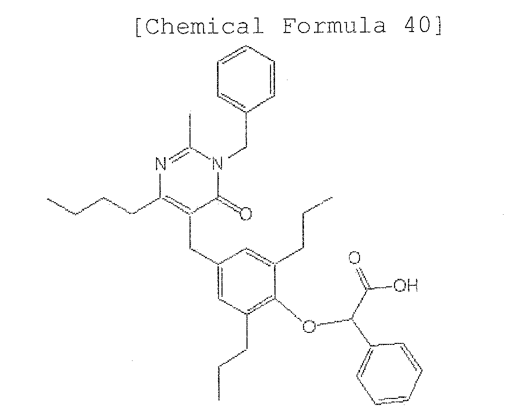

### Process 1:

To a solution of methyl 2-{4-(tert-butyldimethylsilyloxy)-3,5-dipropylbenzyl}-3-oxo heptanate (4.63 g, 10.0 mmol) prepared in Process 2 of Example 1 and acetoamidine hydrochloride (2.84 g, 30.0 mmol) in methanol (40 mL), sodium methoxide (2.16 g, 40.0 mmol) was added, and the reaction mixture was refluxed by heating for 6 hours. The solvent was distilled away, and then water was added to the residue, and the reaction mixture was extracted with ethyl acetate. The organic layers were combined, washed with water and brine, dried over anhydrous sodium sulfate, and concentrated in vacuo. The obtained residue was recrystallized from ethyl acetate, to give 6-butyl-5-{4-(tert-butyldimethylsilyloxy)-3,5-dipropylbenzy l}-2-methylpyrimidin-4(3H)-one (2.40 g, 51.0%) as a colorless leaf-like crystal.

¹H-NMR(CDCl₃) δ: 0.15 (6H, s), 0.86 (3H, t, J=7 Hz), 0.89 (6H, t, J=7 Hz), 1.00 (9H, s), 1.25-1.35 (2H, m), 1.42-1.55 (6H, m), 2.40 (3H, s), 2.47 (4H, t, J=8 Hz), 2.55 (2H, t, J=8 Hz), 3.79 (2H, s), 6.81 (2H, s), 12.71 (1H, br).

### Process 2:

To a solution of 6-butyl-5-{4-(tert-butyldimethylsilyloxy)-3,5-dipropylbenzy l}-2-methylpyrimidin-4(3H)-one (141.0 mg, 0.30 mmol) in N,N-dimethylformamide (5.0 mL), sodium hydride (19.6 g, 0.45 mmol) and benzyl bromide (47.5 µL, 0.40 mmol) were added under ice cooling, and the solution was stirred at 75°C for 15 hours. The reaction mixture was added water, and extracted with ethyl acetate. The organic layers were combined, washed with water and brine, dried over anhydrous sodium sulfate, and concentrated in vacuo. The obtained residue was subjected to a silica gel column chromatography (hexane/acetone), to give 3-benzyl-6-butyl-5-{4-(tert-butyldimethylsilyloxy-3,5-dipro pylbenzyl)-2-methylpyrimidin-4(3H)-one (101.5 mg, 60.3%) as pale yellow amorphous, and 4-benzyloxy-6-butyl-5-{4-(tert-butyldimethylsilyloxy-3,5-di propylbenzyl)-2-methyl pyrimidine (33.1 mg, 19.7%) as pale yellow amorphous.

### Process 3:

To a solution of the obtained 3-benzyl-6-butyl-5-{4-(tert-butyldimethylsilyloxy-3,5-dipro pylbenzyl)-2-methylpyrimidin-4(3H)-one in tetrahydrofuran (3.0 mL), tetra-n-butyl ammonium fluoride (1 mol/L solution in tetrahydrofuran, 0.27 mL, 0.27 mmol) was dropped under ice cooling, and the solution was stirred at room temperature for 1 hour. The reaction mixture was added water, and extracted with ethyl acetate. The organic layers were combined, washed with water and brine, dried over anhydrous sodium sulfate, and concentrated in vacuo. The obtained residue was subjected to a silica gel column chromatography (hexane/acetone), to give 3-benzyl-6-butyl-5-(4-hydroxy-3,5-dipropylbenzyl)-2-methylp yrimidin-4(3H)-one (68.1 mg, 84.7%) as pale yellow amorphous.

¹H-NMR(CDCl₃) δ: 0.89 (3H, t, J=7 Hz), 0.95 (6H, t, J=7 Hz), 1.30-1.40 (2H, m), 1.50-1.64 (6H, m), 2.42 (3H, s), 2.50 (4H, t, J=8 Hz), 2.56 (2H, t, J=8 Hz), 3.84 (2H, s), 4.70 (1H, s), 5.29 (2H, s), 6.85 (2H, s), 7.15-7.18 (2H, m), 7.25-7.33 (3H, m).

### Process 4:

With use of 3-benzyl-6-butyl-5-(4-hydroxy-3,5-dipropylbenzyl)-2-methylp yrimidin-4(3H)-one instead of 6-butyl-5-(4-hydroxy-3,5-dipropylbenzyl)-2-methyl-3-(pyridi n-2-yl)pyrimidin-4(3H)-one, similar reactions and treatments were performed to those of Process 6 of Example 1, to give methyl 2-[4-{(1-benzyl-4-butyl-2-methyl-6-oxo-1,6-dihydropyrimidin -5-yl)methyl}-2,6-dipropylphenoxy]-2-phenyl acetate.

¹H-NMR(CDCl₃) δ: 0.78 (6H, t, J=7 Hz), 0.87 (3H, t, J=7 Hz), 1.27-1.64 (8H, m), 2.27-2.36 (4H, m), 2.42 (3H, s), 2.49-2.58 (2H, m), 3.72 (3H, s), 3.85 (2H, s), 5.08 (1H, s), 5.29 (2H, s), 6.84 (2H, s), 7.15-7.17 (2H, m), 7.24-7.39 (6H, m), 7.49-7.53 (2H, m).

### Process 5:

With use of methyl 2-[4-{(1-benzyl-4-butyl-2-methyl-6-oxo-1,6-dihydropyrimidin -5-yl)methyl}-2,6-dipropylpbenoxy]-2-phenyl acetate instead of methyl 2-[4-[{4-butyl-2-methyl-6-oxo-1-(pyridin-2-yl)-1,6-dihydrop yrimidin-5-yl}methyl]-2,6-dipropylphenoxy]-2-phenyl acetate, similar reactions and treatments were performed to those of Process 7 of Example 1, to give the titled compound (yield: 80.6%).

¹H-NMR(CDCl₃) δ: 0.73 (6H, t, J=7 Hz), 0.82 (3H, t, J=7 Hz), 1.22-1.47 (8H, m), 2.27 (4H, br), 2.42 (3H, s), 2.50 (2H, t, J=8 Hz), 3.82 (2H, s), 5.03 (1H, s), 5.28 (2H, s), 6.80 (2H, s), 7.13-7.16 (2H, m), 7.25-7.30 (6H, m), 7.43 (2H, br).

### Example 29

### Preparation of 2-[4-[{4-(benzyloxy)-6-butyl-2-methylpyrimidin-5-yl}methyl] -2,6-dipropylphenoxy]-2-phenylacetic acid

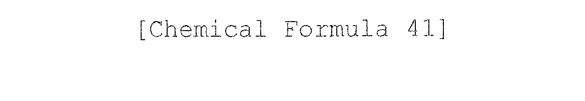

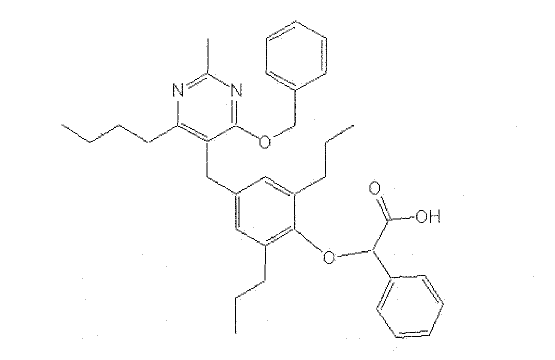

### Process 1:

With use of 4-benzyloxy-6-butyl-5-{4-(tert-butyldimethylsilyloxy-3,5-di propylbenzyl)-2-methyl pyrimidine prepared in Process 2 of Example 28, similar reactions and treatments were performed to those of Process 3 of Example 28, to give 4-[{4-(benzyloxy)-6-butyl-2-methylpyrimidin-5-yl}methyl]-2, 6-dipropyl phenol.

¹H-NMR(CDCl₃) δ: 0.87 (3H, t, J=7 Hz), 0.92 (6H, t, J=7 Hz), 1.30-1.39 (2H, m), 1.49-1.60 (6H, m), 2.45 (4H, t, J=8 Hz), 2.58 (3H, s), 2.69 (2H, t, J=8 Hz), 3.85 (2H, s), 4.58 (1H, s), 5.40 (2H, s), 6.70 (2H, s), 7.28-7.33 (5H, m).

### Process 2:

With use of 4-[{4-(benzyloxy)-6-butyl-2-methylpyrimidin-5-yl}methyl]-2, 6-dipropyl phenol instead of 6-butyl-5-(4-hydroxy-3,5-dipropylbenzyl)-2-methyl-3-(pyridi n-2-yl)pyrimidin-4(3H)-one, similar reactions and treatments were performed to those of Process 6 of Example 1, to give methyl 2-[4-[{4-(benzyloxy)-6-butyl-2-methylpyrimidin-5-yl}methyl] -2,6-dipropylphenoxy]-2-phenyl acetate.

¹H-NMR(CDCl₃) δ: 0.74 (6H, t, J=7 Hz), 0.87 (3H, t, J=7 Hz), 1.26-1.45 (6H, m), 1.48-1.56 (2H, m), 2.20-2.32 (4H, m), 2.59 (3H, s), 2.67 (2H, t, J=8 Hz), 3.72 (3H, s), 3.86 (2H, s), 5.05 (1H, s), 5.39 (2H, s), 6.70 (2H, s), 7.30-7.38 (8H, m), 7.47-7.49 (2H, m).

With use of methyl 2-[4-[{4-(benzyloxy)-6-butyl-2-methylpyrimidin-5-yl}methyl] -2,6-dipropylphenoxy]-2-phenyl acetate instead of methyl 2-[4-[{4-butyl-2-methyl-6-oxo-1-(pyridin-2-yl)-1,6-dihydrop yrimidin-5-yl}methyl]-2,6-dipropylphenoxy]-2-phenyl acetate, similar reactions and treatments were performed to those of Process 7 of Example 1, to give the titled compound (yield: 60.0%).

¹H-NMR(CDCl₃) δ: 0.70 (6H, br), 0.81 (3H, t, J=7 Hz), 1.25-1.45 (8H, m), 2.20 (4H, br), 2.57 (3H, s), 2.64 (2H, br), 3.82 (2H, s), 5.00 (1H, s), 5.38 (2H, s), 6.66 (2H, s), 7.25-7.29 (8H, m), 7.38 (2H, br).

### Example 30

### Preparation of 2-[4-[{4-butyl-1-(cyclohexylmethyl)-2-methyl-6-oxo-1,6-dihy dropyrimidin-5-yl}methyl]-2,6-dipropylphenoxy]-2-phenylacet ic acid

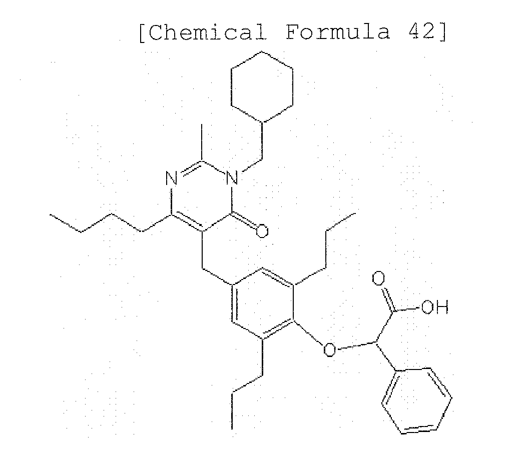

### Process 1:

With use of bromomethyl cyclohexane instead of benzyl bromide, similar reactions and treatments were performed to those of Processes 2 and 3 of Example 28, to give 6-butyl-3-(cyclohexylmethyl)-5-(4-hydroxy-3,5-dipropylbenzy 1)-2-methylpyrimidin-4(3H)-one.

¹H-NMR(CDCl³) δ: 0.88 (3H, t, J=7 Hz), 0.94 (6H, t, J=7 Hz), 1.04-1.25 (4H, m), 1.29-1.38 (2H, m), 1.48-1.65 (10H, m), 1.71-1.74 (2H, m), 1.79-1.85 (1H, m), 2.47-2.54 (9H, m), 3.78 (2H, s), 3.86 (2H, br), 4.73 (1H, s), 6.81 (2H, s).

### Process 2:

With use of 6-butyl-3-(cyclohexylmethyl)-5-(4-hydroxy-3,5-dipropylbenzy 1)-2-methylpyrimidin-4(3H)-one instead of 6-butyl-5-(4-hydroxy-3,5-dipropylbenzyl)-2-methyl-3-(pyridi n-2-yl)pyrimidin-4(3H)-one, similar reactions and treatments were performed to those of Process 6 of Example 1, to give methyl 2-[4-[{4-butyl-1-(cyclohexylmethyl)-2-methyl-6-oxo-1,6-dihy dropyrimidin-5-yl}methyl]-2,6-dipropylphenoxy]-2-phenyl acetate.

¹H-NMR(CDCl₃) δ: 0.76 (6H, t, J= 7 Hz), 0.85-0.89 (3H, m), 1.04-1.82 (19H, m), 2.23-2.33 (4H, m), 2.47-2.54 (5H, m), 3.72 (3H, s), 3.79 (2H, s), 3.81-3.88 (2H, m), 5.08 (1H, s), 6.80 (2H, s), 7.34-'7.38 (3H, m), 7.48-7.53 (2H, m).

### Process 3:

With use of methyl 2-[4-[{4-butyl-1-(cyclohexylmethyl)-2-methyl-6-oxo-1,6-dihy dropyrimidin-5-yl}methyl]-2,6-dipropylphenoxy]-2-phenyl acetate instead of methyl 2-[4-[{4-butyl-2-methyl-6-oxo-1-(pyridin-2-yl)-1,6-dihydrop yrimidin-5-yl}methyl]-2,6-dipropylphenoxy]-2-phenyl acetate, similar reactions and treatments were performed to those of Process 7 of Example 1, to give the titled compound (yield: 48.3%).

¹H-NMR (CDCl₃) δ: 0.73 (6H, br), 0.82 (3H, t, J=7 Hz), 1.04-1.42 (13H, m), 1.60-1.79 (6H, m), 2.25 (4H, br), 2.45-2.51 (5H, m), 3.76 (2H, s), 3.86 (2H, br), 5.03 (1H, s), 6.77 (2H, s), 7.32 (3H, br), 7.43 (2H, br).

### Example 31

### Preparation of 2-[4-[[4-butyl-2-{2-(5-ethylpyridin-2-yl)ethyl}-6-oxo-1,6-d ihydropyrimidin-5-yl]methyl]-2,6-dipropylphenoxy]-2-phenyla cetic acid

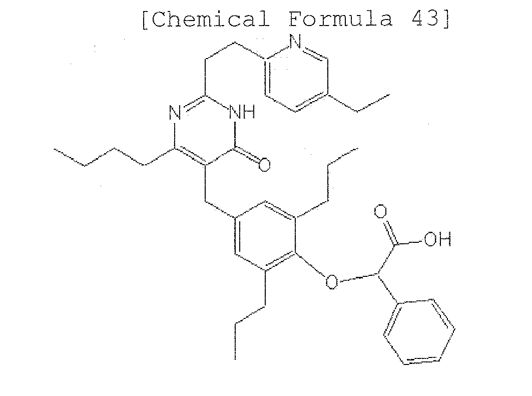

### Process 1:

To a solution of methyl 2-{4-(tert-butyldimethylsilyloxy)-3,5-dipropylbenzyl}-3-oxo heptanate (0.69 g, 1.5 mmol) prepared in Process 2 of Example 1 and 3-(6-ethylpyridin-3-yl)propane imidamide (0.70 g, 3.0 mmol) in methanol (30 mL), sodium methoxide (0.24 g, 4.5 mmol) was added, and the reaction mixture was refluxed by heating for 7 hours. The solvent was distilled away, and then water was added to the residue, and the reaction mixture was extracted with ethyl acetate. The organic layers were combined, washed with water and brine, dried over anhydrous sodium sulfate, and concentrated in vacuo. The obtained residue was subjected to silica gel column chromatography (hexane/acetone), to give 6-butyl-5-{4-(tert-butyldimethylsilyloxy)-3,5-dipropylbenzy 1}-2-{2-(5-ethylpyridin-2-yl)ethyl}pyrimidin-4(3H)-one (0.35 g, 40.0%) as white solid.

### Process 2:

To a solution of the obtained 6-butyl-5-{4-(tert-butyldimethylsilyloxy)-3,5-dipropylbenzy 1}-2-{2-(5-ethylpyridin-2-yl)ethyl}pyrimidin-4(3H)-one (88.5mg, 0.15 mmol) in tetrahydrofuran (4.0 mL), tetra-n-butyl ammonium fluoride (1 mol/L solution in tetrahydrofuran, 0.23 mL, 0.23 mmol) was dropped under ice cooling, and the solution was stirred at room temperature for 1 hour. The reaction mixture was added water, and extracted with ethyl acetate. The organic layers were combined, washed with water and brine, dried over anhydrous sodium sulfate, and concentrated in vacuo. The obtained residue was subjected to a silica gel column chromatography (hexane/acetone), to give 6-butyl-2-{2-(5-ethylpyridin-2-yl)ethyl}-5-(4-hydroxy-3,5-d ipropylbenzyl)pyrimidin-4(3H)-one (71.3 mg, 99.9%) as pale yellow amorphous.

¹H-NMR(CDCl₃) δ: 0.86-0.99 (9H, m), 1.21-1.68 (11H, m), 2.47-2.65 (8H, m), 2.60 (2H, t, J=8 Hz), 3.04-3.22 (4H, m), 3.78 (2H, s), 4.78 (1H, s), 6.84 (2H, s), 7.12 (1H, d, J=8 Hz), 7.44 (1H, dd, J=8, 2 Hz), 8.46 (1H, s), 13.1 (1H, s).

### Process 3:

With use of 6-butyl-3-(cyclohexylmethyl)-5-(4-hydroxy-3,5-dipropylbenzy 1)-2-methylpyrimidin-4(3H)-one instead of 6-butyl-5-(4-hydroxy-3,5-dipropylbenzyl)-2-methyl-3-(pyridi n-2-yl)pyrimidin-4(3H)-one, similar reactions and treatments were performed to those of Process 6 of Example 1, to give methyl 2-[4-[[4-butyl-2-{2-(5-ethylpyridin-2-yl)ethyl}-6-oxo-1,6-d ihydropyrimidin-5-yl]methyl]-2,6-dipropylphenoxy]-2-phenyl acetate (yield: 88.7%).

¹H-NMR(CDCl₃) δ: 0.74 (3H, t, J=7 Hz), 0.83-0.93 (6H, m), 1.21-1.60 (12H, m), 2.25-2.31 (2H, m), 2.46 (2H, t, J=8 Hz), 2.58-2.72 (4H, m), 3.20-3.32 (4H, m), 3.71 (3H, s), 3.87-3.98 (2H, m), 6.18 (1H, s), 6.84 (2H, s), 7.06 (1H, d, J=8 Hz), 7.34-7.53 (6H, m), 8.38 (1H, s).

### Process 4:

With use of methyl 2-[4-[[4-butyl-2-{2-(5-ethylpyridin-2-yl)ethyl}-6-oxo-1,6-d ihydropyrimidin-5-yl]methyl]-2,6-dipropylphenoxy]-2-phenyl acetate instead of methyl 2-[4-[{4-butyl-2-methyl-6-oxo-1-(pyridin-2-yl)-1-1,6-dihydrop yrimidin-5-yl}methyl]-2,6-dipropylphenoxy]-2-phenyl acetate, similar reactions and treatments were performed to those of Process 7 of Example 1, to give the titled compound (yield: 40.8%).

¹H-NMR(CDCl₃) δ: 0.84-0.89 (6H, m), 1.16 (3H, t, J=8 Hz), 1.23-1.36 (2H, m), 1.46-1.54 (6H, m), 2.42 (2H, t, J=8 Hz), 2.53-2.58 (2H, m), 2.62-2.73 (2H, m), 3.12-3.26 (2H, m), 3.35-3.44 (2H, m), 3.78 (2H, dd, J=45, 15 Hz), 6.14 (1H,s), 6.84 (2H, s), 7.13 (1H, d, J=8 Hz), 7.30 (3H, br), 7.48 (1H, d, J=8 Hz), 7.65 (2H, br), 8.37 (1H, s).

### Example 32

### Preparation of 2-[4-[{4-butyl-6-(2-methoxyethoxy)-2-methylpyrimidin-5-yl}m ethyl]-2,6-dipropylphenoxy]-2-phenylacetic acid

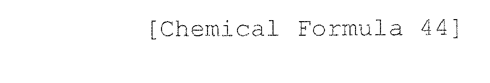

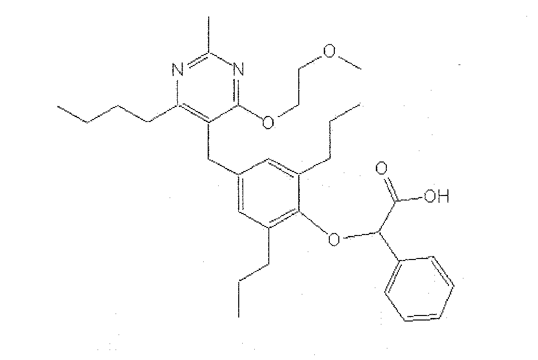

### Process 1:

To a solution of 6-butyl-5-{4-(tert-butyldimethylsilyloxy)-3,5-dipropylbenzy 1}-2-methylpyrimidin-4 (3H) -one (2.13 g, 4.6 mmol) prepared in Process 1 of Example 28, triphenyl phosphine (1.29 g, 4.9 mmol) and 2-methoxyethanol (0.53 g, 6.9 mmol) in tetrahydrofuran (50 mL), diethyl azodicarboxylate (2.2 mL, 4.9 mmol) was added under argon atmosphere, and the solution was stirred at room temperature for 9 hours. The solvent was distilled away, and then the residue was added water, and the reaction mixture was extracted with ethyl acetate. The organic layers were combined, washed with water and brine, dried over anhydrous sodium sulfate, and concentrated in vacuo. The obtained residue was subjected to a silica gel column chromatography (hexane/ethyl acetate), to give 4-butyl-5-{4-(tert-butyldimethylsilyloxy)-3,5-dipropylbenzy 1}-6-(2-methoxyethoxy)-2-methyl pyrimidine (1.58 g, 65.2%) as a pale yellow oil, and 6-butyl-3-(2-methoxyethoxy)-5-{4-(tert-butyldimethylsilylox y)-3,5-dipropylbenzyl}-2-methylpyrimidin-4(3H)-one (0.39 g, 16.3%) as a pale yellow oil.

### Process 2:

To a solution of the obtained 4-butyl-5-{4-(tert-butyldimethylsilyloxy)-3,5-dipropylbenzy 1}-6-(2-methoxyethoxy)-2-methyl pyrimidine (105.8 mg, 0.20 mmol) in tetrahydrofuran (4.0 mL), tetra-n-butyl ammonium fluoride (1 mol/L solution in tetrahydrofuran, 0.30 mL, 0.30 mmol) was dropped under ice cooling, and the solution was stirred at room temperature for 1 hour. The reaction mixture was added water, and extracted with ethyl acetate. The organic layers were combined, washed with water and brine, dried over anhydrous sodium sulfate, and concentrated in vacuo. The obtained residue was subjected to a silica gel column chromatography (hexane/acetone), to give 4-[{4-butyl-6-(2-methoxyethoxy)-2-methylpyrimidin-5-yl}meth yl]-2,6-dipropyl phenol (83.1 mg, 100.2%) as pale yellow amorphous.

¹H-NMR(CDCl₃) δ: 0.86 (3H, t, J=7 Hz), 0.94 (6H, t, J=7 Hz), 1.28-1.37(2H, m), 1.48-1.63 (6H, m), 2.28 (4H, t, J=8 Hz), 2.55 (3H, s), 2.66 (2H, t, J=8 Hz), 3.37 (3H, s), 3.69 (2H, t, J=5 Hz), 3.84 (2H, s), 4.50 (2H, t, J=5Hz), 4.77 (1H, s), 6.75 (2H, s).

### Process 3:

With use of 4-[{4-butyl-6-(2-methoxyethoxy)-2-methylpyrimidin-5-yl}meth yl]-2,6-dipropyl phenol instead of 6-butyl-5-(4-hydroxy-3,5-dipropylbenzyl)-2-methyl-3-(pyridi n-2-yl)pyrimi-din-4(3H)-one, similar reactions and treatments were performed to those of Process 6 of Example 1, to give methyl 2-[4-[{4-butyl-6-(2-methoxyethoxy)-2-methylpyrimidin-5-yl}m ethyl]-2,6-dipropylphenoxy]-2-phenyl acetate (yield: 87.4%).

¹H-NMR(CDCl₃) δ: 0.77 (6H, t, J=7 Hz), 0.86 (3H, t, J=7 Hz), 1.26-1.62 (8H, m), 2.24-2.36 (4H, m), 2.55 (3H, s), 2.64 (2H, t, J=8 Hz), 3.34 (3H, s), 3.67 (2H, t, J=5 Hz), 3.72 (3H, s), 3.85 (2H, s), 4.50 (2H, t, J=5 Hz), 5.07 (1H, s), 6.75 (2H, s), 7.34-7.38 (3H, m), 7.48-7.53 (2H, m).

### Process 4:

With use of methyl 2-[4-[{4-butyl-6-(2-methoxyethoxy)-2-methylpyrimidin-5-yl}m ethyl]-2,6-dipropylphenoxy]-2-phenyl acetate instead of methyl 2-[4-[{4-butyl-2-methyl-6-oxo-1-(pyridin-2-yl)-1,6-dihydrop yrimidin-5-yl}methyl]-2,6-dipropylphenoxy]-2-phenyl acetate, similar reactions and treatments were performed to those of Process 7 of Example 1, to give the titled compound (yield: 71.5%).

1H-NMR(CDCl₃) δ: 0.72 (6H, br), 0.77 (3H, t, J=7 Hz), 1.15-1.43 (8H, m), 2.28 (4H, br), 2.53 (3H, s), 2.63 (2H, t, J=8 Hz), 3.33 (3H, s), 3.65 (2H, t, J=5 Hz), 3.81 (2H, s), 4.49 (2H, t, J=5 Hz), 5.00 (1H, s), 6.70 (2H, s), 7.29 (3H, br), 7.44 (2H, br).

### Example 33

### Preparation of 2-[4-[{4-butyl-1-(2-methoxyethyl)-2-methyl-6-oxo-1,6-dihydr opyrimidin-5-yl}methyl]-2,6-dipropylphenoxy]-2-phenylacetic acid

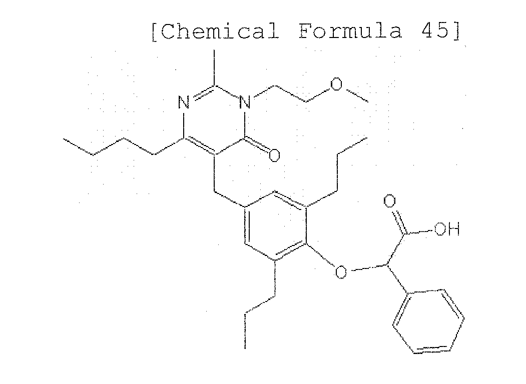

### Process 1:

With use of 6-butyl-3-(2-methoxyethoxy)-5-{4-(tert-butyldimethylsilylox y)-3,5-dipropylbenzyl}-2-methylpyrimidin-4(3H)-one prepared in Process 1 of Example 32, similar reactions and treatments were performed to those of Process 2 of Example 28, to give 6-butyl-5-(4-hydroxy-3,5-dipropylbenzyl)-3-(2-methoxyethyl) -2-methylpyrimidin-4(3H)-one as a byproduct of Example 33.

¹H-NMR(CDCl₃) δ: 0.89 (3H, t, J=7 Hz), 0.94 (6H, t, J=7 Hz), 1.30-1.42 (2H, m), 1.49-1.63 (6H, m), 2.49 (4H, t, J=8 Hz), 2.52-2.57 (5H, m), 3.29 (3H, s), 3.67 (2H, t, J=5 Hz), 3.78 (2H, s), 4.18 (2H, t, J=5 Hz), 4.76 (1H, s), 6.82 (2H, s).

### Process 2:

With use of 6-butyl-5-(4-hydroxy-3,5-dipropylbenzyl)-3-(2-methoxyethyl) -2-methylpyrimidin-4(3H)-one instead of 6-butyl-5-(4-hydroxy-3,5-dipropylbenzyl)-2-methyl-3-(pyridi n-2-yl)pyrimidin-4(3H)-one, similar reactions and treatments were performed to those of Process 6 of Example 1, to give methyl 2-[4-[{4-butyl-1-(2-methoxyethyl)-2-methyl-6-oxo-1,6-dihydr opyrimidin-5-yl}methyl]-2,6-dipropylphenoxy]-2-phenyl acetate.

¹H-HMR(CDCl₃) δ: 0.72 (6H, t, J=7 Hz), 0.87 (3H, t, J=7 Hz), 1.29-1.62 (8H, m), 2.24-2.36 (4H, m), 2.50 (2H, t, J=8 Hz), 2.58 (3H, s), 3.29 (3H, s), 3.67 (2H, t, J=5 Hz), 3.71 (3H, s), 3.79 (2H, s), 4.17-4.20 (2H, m), 5.07 (1H, s), 6.81 (2H, s), 7.34-7.38 (3H, m), 7.48-7.51 (2H, m).

With use of methyl 2-[4-[{4-butyl-1-(2-methoxyethyl)-2-methyl-6-oxo-1,6-dihydr opyrimidin-5-yl}methyl]-2,6-dipropylphenoxy]-2-phenyl acetate instead of methyl 2-[4-[{4-butyl-2-methyl-6-oxo-1-(pyridin-2-yl)-1,6-dihydrop yrimidin-5-yl}methyl]-2,6-dipropylphenoxy]-2-phenyl acetate, similar reactions and treatments were performed to those of Process 7 of Example 1, to give the titled compound (yield: 63.1%).

¹H-NMR(CDCl₃) δ: 0.72 (6H, br), 0.82 (3H, t, J=7 Hz), 1.21-1.47 (8H, m), 2.26 (4H, br), 2.49 (2H, t, J=8 Hz), 2.57 (3H, s), 3.27 (3H, s), 3.64 (2H, t, J=5 Hz), 3.76 (2H, s), 4.27 (2H, t, J=5 Hz), 5.03 (1H, s), 6.77 (2H, s), 7.31 (3H, br), 7.43 (2H, br).

Test example 1: Angiotensin II antagonistic activity in isolated rabbit blood vessels
By using a specimen of isolated rabbit blood vessels, antagonistic activity of the compounds of the invention against angiotensin II type 1 receptor was estimated from a dose-response curve of angiotensin II-induced blood vessel contraction.
Specifically, the specimen of thoracic aorta ring of a rabbit (New Zealand White: male, 2.4 to 3.0 kg) was suspended in a magnus bath filled with Krebs-Henseleite buffer (composition: 118 mM NaCl, 4.7 mM KCl, 2.55 mM CaCl₂, 1.18 mM MgSO₄, 1.18 mM KH₂PO₄, 24.88 mM NaHCO₃, and 11. mM D-gl-ucose), and angiotensin II (10 nM) -induced contraction was obtained in the presence of the compounds of each example (1 nmol to 10 µmol/L). During the measurement, the inside temperature of the magnus bath was maintained at 37°C and the bath was continuously ventilated with a sufficient amount of mixed gas (95% O₂ and 5% CO₂). The angiotensin II-induced contraction was converted into a relative value (%) that is based on the angiotensin II (10 nM) -induced contraction in the absence of the compounds of each example. From the concentration-response curve obtained therefrom, 50% inhibition concentration (IC₅₀ value) was calculated by using SAS Preclinical Package Ver5.0 (trade name, manufactured by SAS institute Japan Co., Tokyo, Japan), which is a statistical analysis program.

As a result, it was found that the compound described in each example has an angiotensin II inhibition activity at 10 µM concentration. These activity values are shown in Table 1. As shown in Table 1, it was confirmed that the compound of the present invention has potent angiotensin II antagonistic activity. Particularly, the compounds of the Examples 15, 16, 23, and 25 have 0.1 µM or less of the IC₅₀ value, which means that they have equivalent angiotensin II-antagonistic activity to that of termisartan. The angiotensin II-inhibitory activity of termisartan was 0.025 µM of the IC₅₀ value at the same conditions.

**[Table 1]**

| **Example No**. | IC₅₀ (*µ*M) |
|---|---|
| 1 | 0.53 |
| 15 | 0.092 |
| 16 | 0.058 |
| 20 | 0.115 |
| 23 | 0.067 |
| 25 | 0.076 |
| 26 | 0.353 |
| 27 | 0.468 |
| 28 | 0.724 |
| 30 | 0.836 |
| 33 | 0.25 |

### Test example 2: PPARγ activation activity

The agonistic activity of the compounds of the invention on PPARγ was measured based on the transfection assay using COS7 cells (DS Pharma Biomedical Co. Lad., Osaka, Japan), which are the cell line derived from the kidney of the African green monkey. COS7 cells were cultured under 5% CO₂ concentration, and DMEM medium containing 10% fetal bovine serum, glutamic acid, and antibiotics was used as a medium.
As an expression vector, a chimera in which DNA binding domain of Ga14, which is a yeast transcription factor, and ligand binding domain of human PPARγ2 are fused, i.e., a fused product between the amino acids 1 to 147 of Ga14 transcription factor and the amino acids 182 to 505 of human PPARγ2, was used. Furthermore, as a reporter vector, a firefly luciferase containing five copies of Ga14 recognition sequence in the promoter region was used. Plasmid transfection to the cells was performed according to a method which uses jetPEI (trade name, manufactured by Funakoshi Co., Ltd., Tokyo, Japan). Furthermore, β-galactosidase expression vector was employed as an internal standard.
After the transfection of the cells, the medium was replaced with a DMEM medium (containing 1% serum) added with the compound, and the cells were further cultured for 16 hours. After that, the luciferase activity and β-galactosidase activity in the cell lysis solution were measured.
For the present test, dimethyl sulfoxide (DMSO) was used for dissolution and dilution of the test compounds , and dulling the cell treatment, the DMSO concentration in DMEM medium (containing 1% serum) was adjusted to 0.1%. As a positive compound, rosiglitazone (trade name, manufactured by ALEXIS Corporation, Switzerland) was used. The luciferase activity (%) of the test compounds (1 to 30 µmol/L) was calculated when the luciferase activity of rosiglitazone (3 to 10 µmol/L) is 100% and the luciferase activity in the absence of the test compound is 0%. The 50% effective concentration of the test compound (EC₅₀, 50% effect concentration) was calculated by using SAS Preclinical Package Ver5.0 (trade name, manufactured by SAS institute Japan Co., Tokyo, Japan), which is a statistical analysis program.

As a result, it was found that the each compound described in Example has a PPARγ activation activity at 30 µM concentration. The results of compounds that showed preferable results, are shown in Table 2. As shown in Table 2, it was confirmed that the compound of the present invention has potent PPARγ activation activity. Particularly, the compounds of Examples 9, 12 to 14, 19, 28, 30, etc., have 1 µM or less of EC₅₀ value, which means that they have more potent PPARγ activation activity than termisartan. Maximum activity strength of some compounds relative to the maximum activity of rosiglitazone is given in Table 3. As shown in Table 3, it was confirmed that the compound of the present invention showed 20 to 63 maximum activity to that of rosiglitazone, and has agonist activity with respect to PPARγ. Particularly, the compounds of Examples 9, 12, 14, 15, 18 to 20, etc., had maximum activity equal to or more than that of termisartan. Under the same condition, the PPARγ activation activity of telmisartan, i.e., EC₅₀, was 1 to 5 µM, and the maximum activity strength relative to the maximum activity of rosiglitazone (i.e., % MAX vs rosiglitazone) was 30 to 50%.

**[Table 2]**

| Example No. | EC₅₀ (µM) | Example No. | EC₅₀ (µM) |
|---|---|---|---|
| 1 | 3.07 | 15 | 2.71 |
| 2 | 3.31 | 16 | 1.46 |
| 3 | 3.52 | 17 | 3.71 |
| 4 | 1.72 | 18 | 1.40 |
| 5 | 1.19 | 19 | 0.65 |
| 6 | 3.19 | 20 | 2.59 |
| 7 | 3.46 | 24 | 1.34 |
| 8 | 2.02 | 28 | 0.85 |
| 9 | 0.54 | 29 | 2.49 |
| 10 | 3.29 | 30 | 0.22 |
| 11 | 2.63 | 31 | 1.21 |
| 12 | 0.44 | 32 | 2.40 |
| 13 | 0.43 | 33 | 2.67 |
| 14 | 0.60 | | |

**[Table 3]**

| Example No. | % MAX vs Rosiglitazone | Example No. | % MAX vs Rosiglitazone |
|---|---|---|---|
| 1 | 32.0 | 14 | 63.0 |
| 2 | 25.2 | 15 | 47.2 |
| 5 | 35.1 | 16 | 44.4 |
| 7 | 23.3 | 18 | 49.3 |
| 8 | 41.4 | 19 | 49.9 |
| 9 | 47.0 | 20 | 58.6 |
| 10 | 25.6 | 24 | 39.8 |
| 12 | 57.5 | 28 | 21.3 |
| 13 | 42.9 | 30 | 28.5 |

From the results obtained above, it was confirmed that the compounds represented by the formula (I) have both a potent angiotensin II receptor antagonistic activity and a PPARγ activation activity. Thus, it was found that the compounds represented by the formula (I) and pharmaceutically acceptable salts thereof are useful as an effective component of a prophylactic and/or therapeutic agent for disorders involved with angiotensin II and PPARγ, for example, hypertension, heart diseases, angina pectoris, cerebrovascular disorders, cerebral circulatory disorders, ischemic peripheral circulatory disorders, renal diseases, arteriosclerosis, inflammatory diseases, type 2 diabetes, diabetic complications, insulin resistance syndrome, syndrome X, metabolic syndrome, and hyperinsulinemia.

### INDUSTRIAL APPLICABILITY

The invention provides a novel compound of α-phenoxybenzeneacetic acid derivative represented by the formula (I) of the invention, or salt or solvate thereof, which has both an angiotensin II receptor antagonistic activity and a PPARγ activation activity. They can be used as an effective component of a novel pharmaceutical product, i.e., a prophylactic and/or therapeutic agent for disorders involved with angiotensin II and PPARγ, for example, hypertersion, heart diseases, angina pectoris, cerebrovascular disorders, cerebral circulatory disorders, ischemic peripheral circulatory disorders, renal diseases, arteriosclerosis, inflammatory diseases, type 2 diabetes, diabetic complications, insulin resistance syndrome, syndrome X, metabolic syndrome, and hyperinsulinemia, and therefore have an industrial applicability.

## Claims

1. An α-phenoxybenzeneacetic acid derivative represented by the following general formula (1): 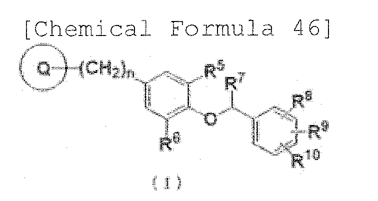 wherein Q represents the following formula (II) or (III): 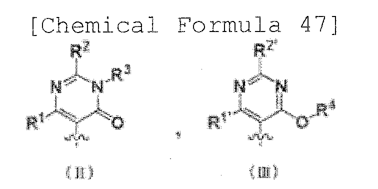
wherein R¹ and R^{1'} represent a C₁₋₆ alkyl group,
R² and R^{2'} represent a C₁₋₆ alkyl group or a C₁₋₆ alkyl-pyridinyl-C₁₋₆ alkyl group,
R³ represents a hydrogen atom, a C₁₋₆ alkyl group, a C₁₋₆ alkoxy-C₁₋₆ alkyl group, a C₃₋₈ cycloalkyl-C₁₋₆ alkyl group, a C₆₋₁₀ aryl-C₁₋₆ alkyl group or the following formula (IV) : 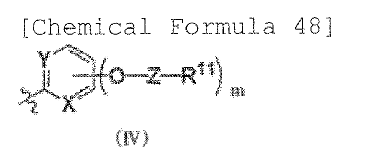 wherein X and Y represent CH or N, Z represents a single bond or a C₁₋₆ alkylene chain, R¹¹ represents a hydrogen atom, a C₆₋₁₀ aryl group, a carbamoyl group, a C₁₋₆ alkylthio group, a C₁₋₆ alkylsulfinyl group or a C₁₋₆ alkylsulfonyl group, and m represents 0 or 1], and
R⁴ represents a C₁₋₆ alkoxy-C₁₋₆ alkyl group or a C₆₋₁₀ aryl-C₁₋₆ alkyl group],
R⁵ and R⁶ represent, independently from each other, a C₁₋₆ alkyl group,
R⁷ represents a carboxyl group, a C₂₋₇ alkoxycarbonyl group, a carbamoyl group, a C₁₋₆ alkylsulfonyl-carbamoyl group, a tetrazolyl group or an oxooxadiazolyl group,
R⁸, R⁹, and R¹⁰ represent, independently from each other, a hydrogen atom, a halogen atom, or a C₁₋₆ alkoxy group, and
n represents an integer of 1 to 3.], or a salt thereof or a solvate thereof.

2. The α-phenoxybenzeneacetic acid derivative, or the salt thereof or the solvate thereof according to claim 1, wherein the compound represented by the general formula (I) is a compound selected from a group consisting of:
2-[4-[{4-butyl-2-methyl-6-oxo-1-(pyridin-2-yl)-1,6-di hydropyrimidin-5-yl}methyl]-2,6-dipropylphenoxy]-2-phenylac etic acid,
2-[4-[{2-methyl-6-oxo-4-propyl-1-(pyridin-2-yl)-1,6-d ihydropyrimidin-5-yl}methyl]-2,6-dipropylphenoxy]-2-phenyla cetic acid,
2-[4-[{4-ethyl-2-methyl-6-oxo-1-(pyridin-2-yl)-1,6-di hydropyrimidin-5-yl}methyl]-2,6-dipropylphenoxy]-2-phenylac etic acid,
6-butyl-2-methyl-5-[4-{phenyl(1H-tetrazol-5-yl)methox y}-3,5-dipropylbenzyl]-3-(pyridin-2-yl)pyrimidin-4(3H)-one,
3-[[4-[{4-butyl-2-methyl-6-oxo-1-(pyridin-2-yl)-1,6-d ihydropyrimidin-5-yl}methyl]-2,6-dipropylphenoxy] (phenyl)me thyl]-1,2,4-oxadiazol-5(4H)-one,
2-[4-[{4-butyl-2--methyl--6-oxo-1-(pyridin-2--yl)-1,6-di hydropyrimidin-5-yl}methyl]-2,6-dipropylphenoxy]-N-(methyls ulfonyl)-2-phenyl acetoamide,
2-[4-[{4-butyl--2-methyl-6-oxo-1-(pyridin-2-yl)-1,6-di hydropyrimidin-5-yl}methyl]-2,6-dipropylphenoxy]-2-(2-chlor ophenyl)acetic acid,
2-[4-[{4-butyl-2-methyl-6-oxo-1-(pyridin-2-yl)-1,6-di hydropyrimidin-5-yl}methyl]-2,6-dipropylphenoxy]-2-(3-chlor ophenyl)acetic acid,
2-[4-[{4-butyl-2-methyl-6-oxo-1-(pyridin-2-yl)-1,6-di hydropyrimidin-5-yl}methyl]-2,H-dipropylphenoxy]-2-(4-chlor ophenyl)acetic acid,
2-[4-[{4-butyl-2-methyl-6-oxo-1-(pyridin-2-yl)-1,6-di hydropyrimidin-5-yl}methyl]-2,6-dipropylphenoxy]-2-(2-metho xyphenyl)acetic acid,
6-butyl-5-[4-{ (2-chlorophenyl) (1H-tetrazol-5-yl)metho xy}-3,5-dipropylbenzyl]-2-methyl-3-(pyridin-2-yl)pyrimidin-4(3H)-one,
2-[4-[[1-{4-(benzyloxy)phenyl}-4-butyl-2-methyl-6-oxo -1, 6-dihydropyrimidin-5-yl]methyl]-2, 6-dipropylpherioxy]-2-p henylacetic acid,
2-[4-[{4-butyl-1-(4-ethoxyphenyl)-2-methyl-6-oxo-1,6-dihydropyrimidin-5-yl}methyl]-2,6-dipropylphenoxy]-2-phenyl acetic acid,
2-[4-[{4-butyl-1-(4-isopropoxyphenyl)-2-methyl-6-oxo-1,6-dihydropyrimidin-5-yl}methyl]-2,6-dipropylphenoxy]-2-ph enylacetic acid,
2-[4-[{4-but-1-(5-methoxypyrimidin-2-yl)-2-methyl-6 -oxo-1,6-dihydropyrimidin-5-yl}methyl]-2,6-dipropylphenoxy] -2-phenylacetic acid,
(-)-2-[4-[{4-butyl-1-(5-methoxypyrimidin-2-yl)-2-meth yl-6-oxo-1,6-dihydropyrimidin-5-yl}methyl]-2,6-dipropylphen oxy]-2-phenylacetic acid,
(+)-2-[4-[{4-butyl-1-(5-methoxypyrimidin-2-yl)-2-meth yl-6-oxe-1,6-dihydropyrimidin-5-yl}methyl]-2,6-dipropylphen oxy]-2-phenylacetic acid,
2-[4-[{4-butyl-1-(5-methoxypyrimidin-2-yl)-2-methyl-6 -oxo-1,6-dihydropyrimidin-5-yl}methyl]-2,6-dipropylphenoxy] -2-(4-chlorophenyl)acetic acid,
2-[4-[[1-{5-(benzyloxy)pyrimidin-2-yl}-4-butyl-2-meth yl-6-oxo-1,6-dihydropyrimidin-5-yl]methyl]-2,6-dipropylphen oxy]-2-phenylacetic acid,
2-[4-[{4-butyl-1-(5-ethoxypyrimidin-2-yl)-2-methyl-6-oxo-1,6-dihydropyrimidin-5-yl}methyl]-2,6-dipropylphenoxy]-2-phenylacetic acid,
(-)-2-[4-[{4-butyl-1-(5-ethoxypyrimidin-2-yl)-2-methy 1-6-oxo-1,6-dihydropyrimidin-5-yl}methyl]-2,6-dipropylpheno xy]-2-phenylacetic acid,
(+)-2-[4-[{4-butyl-1-(5-ethoxypyrimidin-2-yl)-2-methy 1-6-oxo-1,6-dihydropyrimidin-5-yl}methyl]-2,6-dipropylpheno xy]-2-phenylacetic acid,
2-[4-[[1-{5-(4-amino-4-oxobutoxy)pyrimindin-2-yl}-4-bu tyl-2-methyl-6-oxo-1,6-dihydropyrimidin-5-yl]methyl]-2,6-di propylphenoxy]-2-phenylacetic acid,
2-[4-[[4-butyl-2-methyl-1-[5-{2-(methylthio)ethoxy}py rimidin-2-yl]-6-oxo-1,6-dihydropyrimidin-5-yl]methyl]-2,6-d ipropylphenoxy]-2-phenylacetic acid,
2-[4-[[4-butyl-2-methyl-1-[5-{2-(methylsulfinyl)ethox y}pyrimidin-2-yl]-6-oxo-1,6-dihydropyrimidin-5-yl]methyl]-2 ,6-dipropylphenoxy]-2-phenylacetic acid,
2-[4-[[4-butyl-2-methyl-1-[5-{2-(methylsulfonyl)ethox y}pyrimidin-2-yl]-6-oxo-1,6-dihydropyrimidin-5-yl]methyl]-2 ,6-dipropylphenoxy]-2-phenylacetic acid,
2-[4-[{4-butyl-1-(5-hydroxypyrimidin-2-yl)-2-methyl-6 -oxo-1,6-dihydropyrimidin-5-yl}methyl]-2,6-dipropylphenoxy] -2-phenylacetic acid,
2-[4-{(1-benzyl-4-butyl-2-methyl-6-oxo-1,6-dihydropyr imidin-5-yl)methyl}-2,5-dipropy]phenoxy]-2-phenylacetic acid,
2-[4-[{4-(benzyloxy)-6-butyl-2-methylpyrimidin-5-yl}m ethyl]-2,6-dipropylphenoxy]-2-phenylace-tic acid,
2-[4-[{4-butyl-1-(cyclohexylmethyl)-2-methyl-6-oxo-1, 6-dihydropyrimidin-5-yl}methyl]-2,6-dipropylphenoxy]-2-phen ylacetic acid,
2-[4-[[4-butyl-2-{2-(5-ethylpyridin-2-yl)ethyl}-6-oxo -1,6-dihydropyrimidin-5-yl]methyl]-2,6-dipropylpheoxy]-2-p henylacetic acid,
2-[4-[{4-butyl-6-(2-methoxyethoxy)-2-methylpyrimidin-5-yl}methyl]-2,6-dipropylphenoxy]-2-phenylacetic acid, and
2-[4-[{4-butyl-1-(2-methoxyethyl)-2-methyl-6-oxo-1,6-dihydropyrimidin-5-yl}methyl]-2,6-dipropylphenoxy]-2-phenyl acetic acid.

3. A pharmaceutical composition comprising the α-phenoxybenzeneacetic acid derivative, or the salt thereof or the solvate thereof according to any one of claims 1 and 2, and a pharmaceutically accepted carries.

4. A pharmaceutical composition comprising the α-phenoxybenzeneacetic acid derivative, or the salt thereof, or the solvate thereof according to any one of claims 1 and 2 as an active ingredient, which has angiotensin II receptor-antagonistic activity and PPARγ activation activity in combination.

5. The preventive and/or therapeutic agent in a cardiovascular disease comprising the α-phenoxybenzeneacetic acid derivative, or the salt thereof, or the solvate thereof according to any one of claims 1 and 2 as an active ingredient.

6. The preventive and/or therapeutic agent according to claim 5, wherein the cardiovascular disease is hypertension, a cardiac disease, angina pectoris, a cerebrovascular disorder, a cerebral circulatory disorder, an ischemic peripheral circulatory disorder, a renal disease, or arteriosclerosis.

7. A preventive and/or therapeutic agent in a metabolic disease comprising the α-phenoxybenzeneacetic acid derivative, or the salt thereof, or the solvate thereof according to any one of claims 1 and 2 as an active ingredient.

8. The preventive and/or therapeutic agent according to claim 7, wherein the metabolic disease is Type II diabetes mellitus, diabetic retinopathy, a diabetic nerve disorder, diabetic nephropathy, insulin resistance syndrome, metabolic syndrome, or hyperinsulinemia.

9. A. method of preventing and/or treating a cardiovascular disease, which is **characterized by** administering an effective amount of the α-phenoxybenzeneacetic acid derivative, or the salt thereof, or the solvate thereof according to any one of claims 1 and 2 to a patient in need of the treatment.

10. The method of preventing and/or treating a cardiovascular disease according to claim 9, wherein the cardiovascular disease is hypertension, a cardiac disease, angina pectoris, a cerebrovascular disorder, a cerebral circulatory disorder, an ischemic peripheral circulatory disorder, a renal disease, or arteriosclerosis.

11. A method of preventing and/or treating a metabolic disease, which is **characterized by** administering an effective amount of the α-phenoxybenzeneacetic acid derivative, or a salt thereof, or a solvate thereof according to any one of claims 1 and 2 to a patient in need of the treatment.

12. The method of preventing and/or treating a metabolic disease according to claim 11, wherein the metabolic disease is Type II diabetes mellitus, diabetic retinopathy, a diabetic nerve disorder, diabetic nephropathy, insulin resistance syndrome, metabolic syndrome, or hyperinsulinemia.

13. Use of the α-phenoxybenzeneacetic acid derivative, or the salt thereof, or the solvate thereof according to any one of claims 1 and 2 configured to manufacture a preparation for prevention and/or treatment of a cardiovascular disease.

14. The use according to claim 13, wherein the cardiovascular disease is hypertension, a cardiac disease, angina pectoris, a cerebrovascular disorder, a cerebral circulatory disorder, an ischemic peripheral circulatory disorder, a renal disease, or arteriosclerosis.

15. Use of the α-phenoxybenzeneacetic acid derivative, or the salt thereof, or the solvate thereof according to any one of claims 1 and 2 configured to manufacture a preparation for prevention and/or treatment of a metabolic disease.

16. The use according to claim 15, wherein the metabolic disease is Type II diabetes mellitus, diabetic retinopathy, a diabetic nerve disorder, diabetic nephropathy, insulin resistance syndrome, metabolic syndrome, or hyperinsulinemia.
